# FASCICULE DE BREVET EUROPEEN

(11) **EP 4 153 769 B1**
(45) Date de publication et mention de la délivrance du brevet: **26.03.2025**
(21) Numéro de dépôt: 21733502.5
(22) Date de dépôt: 20.05.2021
(51) Int. Cl.: G01N 33/543, G01N 33/52, G01N 33/569, C12Q 1/14

(54) **DISPOSITIF DE DETECTION D'UNE BACTERIE D'INTERET**
VORRICHTUNG ZUM NACHWEIS EINES BESTIMMTEN BAKTERIUMS
DEVICE FOR DETECTING A BACTERIUM OF INTEREST

(30) Priorité: 20.05.2020 FR 2005352
(43) Date de publication de la demande: 29.03.2023
(73) Titulaire: Vetophage, 69007 Lyon (FR)
(72) Inventeur: CHATAIN-LY, Mai Huong, 69150 DECINES (FR)
(74) Mandataire: Plasseraud IP
(86) Numéro de dépôt international: PCT/FR2021/050925
(87) Numéro de publication internationale: WO 2021/234321

(56) Documents cités:
- EP-A1- 2 141 176
- EP-B1- 2 141 176
- WO-A1-2006/105504
- WO-A1-2010/092333
- US-A1- 2016 024 478
- US-A1- 2019 276 868
- US-B2- 8 124 355

## Description

### DOMAINE TECHNIQUE

La présente divulgation concerne un dispositif de détection de la présence d'une bactérie d'intérêt dans un échantillon grâce à l'utilisation d'un phage spécifique de la bactérie d'intérêt ou d'une protéine d'un phage spécifique de la bactérie d'intérêt.

### CONTEXTE DE L'INVENTION

*Staphylococcus aureus* est une des espèces bactériennes les plus rencontrées en pathologie humaine et vétérinaire (Reinoso et al., 2004).

En élevage bovin, cette bactérie est la responsable majeure de la mammite, pathologie contagieuse dans les troupeaux laitiers. En effet, après la traite, les trayons restent ouverts et des germes pathogènes, dont *S. aureus,* peuvent pénétrer. La bactérie peut coloniser et infecter les mamelles de la vache, ce qui induit une forte diminution de la production de lait. La baisse de production laitière due à une mammite clinique est en moyenne de 5 % sur une lactation, soit 400 litres de lait pour une lactation de 8.000 litres (www.web-agri.fr/conduite-elevage/sante-animale/article/l-impact-economique-atteint-230-vache-an-1184-97330.html).

*Staphylococcus aureus* étant souvent résistant aux antibiotiques, il est très difficile de l'éradiquer (Foster Timothy J., 2017).

Pour lutter contre l'apparition de mammites dues à *Staphylococcus aureus,* un moyen serait de mettre en place des traitements préventifs pour réduire le nombre de vaches infectées, en limitant tout contact entre les vaches malades et les vaches saines ou en évitant la lésion des trayons.

Il est donc essentiel de développer des solutions rapides pour pouvoir détecter la présence de cette bactérie dans le milieu d'élevage, notamment dans le lait cru issu de vaches, afin de prévenir et/ou traiter les infections bactériennes, en particulier par l'utilisation d'antibiotiques appropriés à la bactérie pathogène détectée. Cependant, à ce jour, aucune méthode de détection n'est pleinement satisfaisante.

Les dispositifs de diagnostic de la mammite présents sur le marché sont principalement basés sur le dépistage des cellules somatiques qu'on retrouve dans le lait. Or, cette méthode ne permet pas d'identifier la ou les bactérie(s) responsable(s) de l'infection. D'autre part ces dernières peuvent constituer une trace d'une infection plus ou moins ancienne et donc constituer un indicateur non-spécifique d'une infection en cours. Cela conduit donc les éleveurs à utiliser des antibiothérapies de façon massive et non ciblée.

D'autres méthodes de détection des bactéries peuvent être utilisées telles que la réaction en chaîne par polymérase (PCR) ou la méthode de culture. Toutefois, celles-ci nécessitent d'envoyer les échantillons au laboratoire et donc l'intervention de personnes qualifiées pour mettre en œuvre la méthode de détection et pour analyser les résultats.

De plus, l'utilisation de la méthode de culture nécessite un temps d'incubation de 24h à 48h, ce qui représente une attente trop longue des résultats, compte tenu des enjeux du secteur. L'approche par la biologie moléculaire a permis de développer des méthodes spécifiques telles que la PCR et la PCR quantitative. Cependant, bien que plus rapide et plus spécifique, les méthodes par PCR sont bien plus onéreuses. De plus, la technique de PCR détecte seulement la présence d'ADN des bactéries et non la présence de bactéries viables, ce qui peut entraîner la détection de faux positifs (bactéries mortes et déjà prises en charge par le système immunitaire de la vache).

La demande US2019/0276868A1 décrit des méthodes et des systèmes de détection pour capturer et détecter un microorganisme d'intérêt, dans un échantillon, à l'aide d'un composant de liaison cellulaire (CBD) spécifique du microorganisme d'intérêt.

Ainsi, les méthodes existantes ne répondent pas aux besoins des éleveurs car elles ne peuvent pas être mises en œuvre par les éleveurs eux-mêmes. De plus, elles sont assez contraignantes car elles nécessitent le prélèvement de l'échantillon dans des conditions aseptiques et un envoi rapide au laboratoire en conditions réfrigérées. Les méthodes d'une spécificité moindre sont donc relativement longues et inadaptées à la nécessité d'une mise en place rapide de mesures de traitement/prévention face au caractère contagieux des bactéries responsables de la mammite bovine dont *S. aureus* est une des premières causes. Les méthodes par PCR représentant, quant-à-elle, un coût d'analyse élevé, ne sont donc pas adaptées à la nécessité de diagnostic d'un nombre de vaches important.

L'enjeu majeur est d'avoir une méthode simple, rapide, pratique (sans gros appareillage), et peu couteuse afin que les éleveurs puissent l'utiliser eux-mêmes sur le terrain pour dépister la présence de *S. aureus* dans leurs élevages.

En outre, une telle méthode de détection de bactéries *S. aureus* pourra également être appliquée dans différents domaines comme par exemple dans la sécurité alimentaire, le contrôle des eaux ou en santé humaine.

### RESUME DE L'INVENTION

L'invention est définie dans les revendications.

Un premier aspect de la description porte sur une bandelette de détection d'une bactérie d'intérêt par chromatographie à flux latéral comprenant une partie qui comprend au moins un bactériophage spécifique de la bactérie d'intérêt et/ou au moins une protéine d'au moins un bactériophage spécifique de la bactérie d'intérêt.

Un second aspect de la description porte sur un procédé de détection d'une bactérie d'intérêt dans un échantillon.

Un troisième aspect de la description porte sur un kit de détection d'une bactérie d'intérêt dans un échantillon.

Un quatrième aspect de la description porte sur l'utilisation d'un bactériophage spécifique d'une bactérie d'intérêt ou d'une protéine de celui-ci dans un dispositif de chromatographie à flux latéral pour détecter ladite bactérie d'intérêt dans un échantillon.

Un cinquième aspect porte sur des protéines de bactériophages en tant que telles et leur utilisation dans des systèmes de détection de bactéries.

### DEFINITIONS

Ci-après sont données des définitions qui s'appliquent à l'ensemble de la présente divulgation.

Au sens de la présente description, par « chromatographie à flux latéral » (lateral flow assay, LFA), on entend un dispositif de chromatographie dans lequel un échantillon liquide migre par capillarité dans un flux dirigé au travers d'une bandelette comprenant plusieurs parties. La LFA est connue de l'art antérieure et a notamment été décrite pour la détection immunologique d'un analyte d'intérêt dans un échantillon liquide, grâce à un anticorps primaire et un anticorps secondaire (EP1086372B1 ; US 8,399.261 B2 ; WO 2017/072078 ; Nanerjee and Jaiswal, 2018).

Au sens de la présente description, par « au moins un », on entend un, deux, trois, quatre, cinq, six et suivants.

Au sens de la présente description, un « bactériophage spécifique d'une bactérie d'intérêt » est un bactériophage capable de se lier spécifiquement à cette bactérie. Cette spécificité peut être déterminée par la méthode des spots décrite dans la présente description. Un bactériophage est spécifique d'une bactérie si une plage de lyse est observée autour d'un spot contenant ledit bactériophage, déposé sur une boite de Pétri recouverte d'une culture de ladite bactérie en phase exponentielle, après 18h d'incubation à une température adaptée à la bactérie.

Par « au moins un bactériophage spécifique », on entend qu'il peut s'agir d'un, deux, trois, quatre, cinq ou six bactériophages spécifiques d'une bactérie d'intérêt, ou plus. Au sens de la présente description, ledit au moins un bactériophage spécifique est en particulier au moins un bactériophage spécifique de *S. aureus.* Plus particulièrement, ledit au moins un bactériophage spécifique de *S. aureus* est choisi parmi les bactériophages spécifiques de *S. aureus* déposés à la Collection Nationale de Cultures de Microorganismes (CNCM) sous les numéros CNCM I-5408, CNCM I-5409, CNCM I-5410, CNCM I-5491, CNCM I-5492 et CNCM I-5680. En particulier, il peut s'agir de deux bactériophages spécifiques de *S. aureus* choisis parmi les bactériophages déposés sous les numéros CNCM I-5408, CNCM I-5409, CNCM I-5410, CNCM I-5491, CNCM I-5492 et CNCM I-5680.

Par exemple il s'agit des couples suivants : le bactériophage CNCM I-5408 et le bactériophage CNCM I-5409, le bactériophage CNCM I-5409 et le bactériophage CNCM I-5410, le bactériophage CNCM I-5408 et le bactériophage CNCM I-5410, le bactériophage CNCM I-5408 et le bactériophage CNCM I-5491, le bactériophage CNCM I-5409 et le bactériophage CNCM I-5491, le bactériophage CNCM I-5410 et le bactériophage CNCM I-5491, le bactériophage CNCM I-5408 et le bactériophage CNCM I-5492, le bactériophage CNCM I-5409 et le bactériophage CNCM I-5492, le bactériophage CNCM I-5410 et le bactériophage CNCM I-5492, le bactériophage CNCM I-5491 et le bactériophage CNCM I-5492, le bactériophage CNCM I-5680 et le bactériophage CNCM I-5408, le bactériophage CNCM I-5680 et le bactériophage CNCM I-5409, le bactériophage CNCM I-5680 et le bactériophage CNCM I-5492, le bactériophage CNCM I-5680 et le bactériophage CNCM I-5410, le bactériophage CNCM I-5680 et le bactériophage CNCM I-5491, le bactériophage CNCM I-5680 et le bactériophage CNCM I-5492.

En particulier, il peut s'agir de trois bactériophages spécifiques de S. *aureus* choisis parmi les bactériophages déposés sous les numéros CNCM I-5408, CNCM I-5409, CNCM I-5410, CNCM I-5491, CNCM I-5492 et CNCM I-5680.

Par exemple, il peut s'agir de trois bactériophages spécifiques de *S. aureus* tels que les bactériophages déposés sous les numéros CNCM I-5408, CNCM I-5409 et CNCM I-5410, les bactériophages déposés sous les numéros CNCM I-5408, CNCM I-5491 et CNCM I-5492, les bactériophages déposés sous les numéros CNCM I-5409, CNCM I-5491 et CNCM I-5492, les bactériophages déposés sous les numéros CNCM I-5410, CNCM I-5491 et CNCM I-5492, les bactériophages déposés sous les numéros CNCM I-5408, CNCM I-5409 et CNCM I-5491, les bactériophages déposés sous les numéros CNCM I-5408, CNCM I-5410 et CNCM I-5491, les bactériophages déposés sous les numéros CNCM I-5408, CNCM I-5409 et CNCM I-5492, les bactériophages déposés sous les numéros CNCM I-5408, CNCM I-5410 et CNCM I-5492, les bactériophages déposés sous les numéros CNCM I-5409, CNCM I-5410 et CNCM I-5491, les bactériophages déposés sous les numéros CNCM I-5409, CNCM I-5410 et CNCM I-5492, les bactériophages déposés sous les numéros CNCM I-5409, CNCM I-5491 et CNCM I-5680, les bactériophages déposés sous les numéros CNCM I-5410, CNCM I-5491 et CNCM I-5680, les bactériophages déposés sous les numéros CNCM I-5408, CNCM I-5409 et CNCM I-5680, les bactériophages déposés sous les numéros CNCM I-5408, CNCM I-5410 et CNCM I-5680, les bactériophages déposés sous les numéros CNCM I-5408, CNCM I-5680 et CNCM I-5492, les bactériophages déposés sous les numéros CNCM I-5680, CNCM I-5410 et CNCM I-5492, les bactériophages déposés sous les numéros CNCM I-5409, CNCM I-5410 et CNCM I-5680, les bactériophages déposés sous les numéros CNCM I-5409, CNCM I-5680 et CNCM I-5492, les bactériophages déposés sous les numéros CNCM I-5491, CNCM I-5492 et CNCM I-5680, les bactériophages déposés sous les numéros CNCM I-5408, CNCM I-5491 et CNCM I-5680.

En particulier, il peut s'agir de quatre bactériophages spécifiques de *S. aureus* choisis parmi les bactériophages déposés sous les numéros CNCM I-5408, CNCM I-5409, CNCM I-5410, CNCM I-5491, CNCM I-5492 et CNCM I-5680.

Par exemple, il peut s'agir de quatre bactériophages spécifiques de *S. aureus* tels que les bactériophages déposés sous les numéros CNCM I-5408, CNCM I-5409, CNCM I-5410 et CNCM I-5491, les bactériophages déposés sous les numéros CNCM I-5408, CNCM I-5409, CNCM I-5410 et CNCM I-5492, les bactériophages déposés sous les numéros CNCM I-5408, CNCM I-5409, CNCM I-5491 et CNCM I-5492, les bactériophages déposés sous les numéros CNCM I-5408, CNCM I-5410, CNCM I-5491 et CNCM I-5492, les bactériophages déposés sous les numéros CNCM I-5410, CNCM I-5409, CNCM I-5491 et CNCM I-5492, les bactériophages déposés sous les numéros CNCM I-5680, CNCM I-5409, CNCM I-5410 et CNCM I-5491, les bactériophages déposés sous les numéros CNCM I-5680, CNCM I-5409, CNCM I-5410 et CNCM I-5492, les bactériophages déposés sous les numéros CNCM I-5680, CNCM I-5409, CNCM I-5491 et CNCM I-5492, les bactériophages déposés sous les numéros CNCM I-5680, CNCM I-5410, CNCM I-5491 et CNCM I-5492, les bactériophages déposés sous les numéros CNCM I-5408, CNCM I-5409, CNCM I-5410 et CNCM I-5680, les bactériophages déposés sous les numéros CNCM I-5408, CNCM I-5409, CNCM I-5491 et CNCM I-5680, les bactériophages déposés sous les numéros CNCM I-5408, CNCM I-5409, CNCM I-5492 et CNCM I-5680.

En particulier, il peut s'agir de cinq bactériophages spécifiques de *S. aureus* choisis parmi les bactériophages déposés sous les numéros CNCM I-5408, CNCM I-5409, CNCM I-5410, CNCM I-5491, CNCM I-5492 et CNCM I-5680.

En particulier, il peut s'agir de six bactériophages spécifiques de S. *aureus* tels que les bactériophages déposés sous les numéros CNCM I-5408, CNCM I-5409, CNCM I-5410, CNCM I-5491, CNCM I-5492 et CNCM I-5680.

Par « au moins une protéine d'au moins un bactériophage», on entend qu'il peut s'agir d'une protéine d'un bactériophage, de plusieurs protéines (par exemple deux ou trois) d'un même bactériophage, d'une protéine de plusieurs bactériophages (par exemple deux ou trois), ou de plusieurs protéines de plusieurs bactériophages différents. La définition donnée ci-dessus pour « au moins un bactériophage spécifique » s'intègre dans la définition de « au moins une protéine d'au moins un bactériophage». Autrement dit, en particulier, ladite au moins une protéine d'au moins un bactériophage spécifique d'une bactérie d'intérêt peut être au moins une protéine d'au moins un bactériophage spécifique de *S. aureus.*

En particulier, ladite au moins une protéine de phage est une protéine de liaison (RBP, receptor binding protein).

Plus particulièrement, il peut s'agir d'au moins une protéine, en particulier d'une RBP, d'au moins un bactériophage spécifique de *S. aureus* choisi parmi les bactériophages spécifiques de S. *aureus* déposés à la Collection Nationale de Cultures de Microorganismes (CNCM) sous les numéros CNCM I-5408, CNCM I-5409, CNCM I-5410, CNCM I-5491, CNCM I-5492 et CNCM I-5680.

En particulier, il peut s'agir de deux protéines, en particulier des RBP, provenant d'un même bactériophage spécifique de *S. aureus,* ledit bactériophage spécifique de *S. aureus* étant choisi parmi les bactériophages déposés sous les numéros CNCM I-5408, CNCM I-5409, CNCM I-5410, CNCM I-5491, CNCM I-5492 et CNCM I-5680.

En particulier, il peut s'agir de deux protéines, en particulier des RBP, provenant de bactériophages spécifiques de *S. aureus* différents, lesdits bactériophages spécifiques de *S. aureus* étant choisis parmi les bactériophages déposés sous les numéros CNCM I-5408, CNCM I-5409, CNCM I-5410, CNCM I-5491, CNCM I-5492 et CNCM I-5680.

Par exemple, il peut s'agir d'au moins une protéine du bactériophage CNCM I-5408 et d'au moins une protéine du bactériophage CNCM I-5409, d'au moins une protéine du bactériophage CNCM I-5409 et d'au moins une protéine du bactériophage CNCM I-5410, d'au moins une protéine du bactériophage CNCM I-5408 et d'au moins une protéine du bactériophage CNCM I-5410, d'au moins une protéine du bactériophage CNCM I-5408 et d'au moins une protéine du bactériophage CNCM I-5491, d'au moins une protéine du bactériophage CNCM I-5409 et d'au moins une protéine du bactériophage CNCM I-5491, d'au moins une protéine du bactériophage CNCM I-5410 et d'au moins une protéine du bactériophage CNCM I-5491, d'au moins une protéine du bactériophage CNCM I-5408 et d'au moins une protéine du bactériophage CNCM I-5492, d'au moins une protéine du bactériophage CNCM I-5409 et d'au moins une protéine du bactériophage CNCM I-5492, d'au moins une protéine du bactériophage CNCM I-5410 et d'au moins une protéine du bactériophage CNCM I-5492, d'au moins une protéine du bactériophage CNCM I-5491 et d'au moins une protéine du bactériophage CNCM I-5492, d'au moins une protéine du bactériophage CNCM I-5680 et d'au moins une protéine du bactériophage CNCM I-5408, d'au moins une protéine du bactériophage CNCM I-5680 et d'au moins une protéine du bactériophage CNCM I-5409, d'au moins une protéine du bactériophage CNCM I-5680 et d'au moins une protéine du bactériophage CNCM I-5410, d'au moins une protéine du bactériophage CNCM I-5680 et d'au moins une protéine du bactériophage CNCM I-5491, d'au moins une protéine du bactériophage CNCM I-5680 et d'au moins une protéine du bactériophage CNCM I-5492.

En particulier, il peut s'agir de trois protéines, en particulier des RBP, provenant d'au moins un bactériophage spécifique de *S. aureus,* lesdits bactériophages spécifiques de *S. aureus* étant choisis parmi les bactériophages déposés sous les numéros CNCM I-5408, CNCM I-5409, CNCM I-5410, CNCM I-5491, CNCM I-5492 et CNCM I-5680.

En particulier, il peut s'agir de trois protéines, en particulier des RBP, provenant de bactériophages spécifiques de *S. aureus* différents, lesdits bactériophages spécifiques de *S. aureus* étant choisis parmi les bactériophages déposés sous les numéros CNCM I-5408, CNCM I-5409, CNCM I-5410, CNCM I-5491, CNCM I-5492 et CNCM I-5680.

Par exemple, il peut s'agir d'au moins une protéine du bactériophage CNCM I-5408, d'au moins une protéine du bactériophage CNCM I-5409 et d'au moins une protéine du bactériophage CNCM I-5410 ; d'au moins une protéine du bactériophage CNCM I-5408, d'au moins une protéine du bactériophage CNCM I-5491 et d'au moins une protéine du bactériophage CNCM I-5492 ; d'au moins une protéine du bactériophage CNCM I-5409, d'au moins une protéine du bactériophage CNCM I-5491 et d'au moins une protéine du bactériophage CNCM I-5492 ; d'au moins une protéine du bactériophage CNCM I-5410, d'au moins une protéine du bactériophage CNCM I-5491 et d'au moins une protéine du bactériophage CNCM I-5492 ; d'au moins une protéine du bactériophage CNCM I-5408, d'au moins une protéine du bactériophage CNCM I-5409 et d'au moins une protéine du bactériophage CNCM I-5491 ; d'au moins une protéine du bactériophage CNCM I-5408, d'au moins une protéine du bactériophage CNCM I-5410 et d'au moins une protéine du bactériophage CNCM I-5491 ; d'au moins une protéine du bactériophage CNCM I-5408, d'au moins une protéine du bactériophage CNCM I-5409 et d'au moins une protéine du bactériophage CNCM I-5492 ; d'au moins une protéine du bactériophage CNCM I-5408, d'au moins une protéine du bactériophage CNCM I-5410 et d'au moins une protéine du bactériophage CNCM I-5492 ; d'au moins une protéine du bactériophage CNCM I-5409, d'au moins une protéine du bactériophage CNCM I-5410 et d'au moins une protéine du bactériophage CNCM I-5491 ; d'au moins une protéine du bactériophage CNCM I-5409, d'au moins une protéine du bactériophage CNCM I-5410 et d'au moins une protéine du bactériophage CNCM I-5492 ; d'au moins une protéine du bactériophage CNCM I-5409, d'au moins une protéine du bactériophage CNCM I-5491 et d'au moins une protéine du bactériophage CNCM I-5680 ; d'au moins une protéine du bactériophage CNCM I-5410, d'au moins une protéine du bactériophage CNCM I-5491 et d'au moins une protéine du bactériophage CNCM I-5680 ; d'au moins une protéine du bactériophage CNCM I-5408, d'au moins une protéine du bactériophage CNCM I-5409 et d'au moins une protéine du bactériophage CNCM I-5680 ; d'au moins une protéine du bactériophage CNCM I-5408, d'au moins une protéine du bactériophage CNCM I-5410 et d'au moins une protéine du bactériophage CNCM I-5680 ; d'au moins une protéine du bactériophage CNCM I-5408, d'au moins une protéine du bactériophage CNCM I-5680 et d'au moins une protéine du bactériophage CNCM I-5492 ; d'au moins une protéine du bactériophage CNCM I-5680, d'au moins une protéine du bactériophage CNCM I-5410 et d'au moins une protéine du bactériophage CNCM I-5492 ; d'au moins une protéine du bactériophage CNCM I-5409, d'au moins une protéine du bactériophage CNCM I-5410 et d'au moins une protéine du bactériophage CNCM I-5680 ; d'au moins une protéine du bactériophage CNCM I-5409, d'au moins une protéine du bactériophage CNCM I-5680 et d'au moins une protéine du bactériophage CNCM I-5492; d'au moins une protéine du bactériophage CNCM I-5491, d'au moins une protéine du bactériophage CNCM I-5492 et d'au moins une protéine du bactériophage CNCM I-5680 ; d'au moins une protéine du bactériophage CNCM I-5408, d'au moins une protéine du bactériophage CNCM I-5491 et d'au moins une protéine du bactériophage CNCM I-5680.

En particulier, il peut s'agir de quatre protéines, en particulier des RBP, provenant d'au moins un bactériophage spécifique de *S. aureus,* lesdits bactériophages spécifiques de *S. aureus* étant choisis parmi les bactériophages déposés sous les numéros CNCM I-5408, CNCM I-5409, CNCM I-5410, CNCM I-5491, CNCM I-5492 et CNCM I-5680.

En particulier, il peut s'agir de quatre protéines, en particulier des RBP, provenant de bactériophages spécifiques de *S. aureus* différents, lesdits bactériophages spécifiques de *S. aureus* étant choisis parmi les bactériophages déposés sous les numéros CNCM I-5408, CNCM I-5409, CNCM I-5410, CNCM I-5491, CNCM I-5492 et CNCM I-5680.

Par exemple, il s'agit d'au moins une protéine du bactériophage CNCM I-5408, d'au moins une protéine du bactériophage CNCM I-5409, d'au moins une protéine du bactériophage CNCM I-5410 et d'au moins une protéine du bactériophage CNCM I-5491 ; d'au moins une protéine du bactériophage CNCM I-5408, d'au moins une protéine du bactériophage CNCM I-5409, d'au moins une protéine du bactériophage CNCM I-5410 et d'au moins une protéine du bactériophage CNCM I-5492 ; d'au moins une protéine du bactériophage CNCM I-5408, d'au moins une protéine du bactériophage CNCM I-5409, d'au moins une protéine du bactériophage CNCM I-5491 et d'au moins une protéine du bactériophage CNCM I-5492 ; d'au moins une protéine du bactériophage CNCM I-5408, d'au moins une protéine du bactériophage CNCM I-5410, d'au moins une protéine du bactériophage CNCM I-5491 et d'au moins une protéine du bactériophage CNCM I-5492 ; d'au moins une protéine du bactériophage CNCM I-5410, d'au moins une protéine du bactériophage CNCM I-5409, d'au moins une protéine du bactériophage CNCM I-5491 et d'au moins une protéine du bactériophage CNCM I-5492 ; d'au moins une protéine du bactériophage CNCM I-5680, d'au moins une protéine du bactériophage CNCM I-5409, d'au moins une protéine du bactériophage CNCM I-5410 et d'au moins une protéine du bactériophage CNCM I-5491 ; d'au moins une protéine du bactériophage CNCM I-5680, d'au moins une protéine du bactériophage CNCM I-5409, d'au moins une protéine du bactériophage CNCM I-5410 et d'au moins une protéine du bactériophage CNCM I-5492 ; d'au moins une protéine du bactériophage CNCM I-5680, d'au moins une protéine du bactériophage CNCM I-5409, d'au moins une protéine du bactériophage CNCM I-5491 et d'au moins une protéine du bactériophage CNCM I-5492 ; d'au moins une protéine du bactériophage CNCM I-5680, d'au moins une protéine du bactériophage CNCM I-5410, d'au moins une protéine du bactériophage CNCM I-5491 et d'au moins une protéine du bactériophage CNCM I-5492 ; d'au moins une protéine du bactériophage CNCM I-5408, d'au moins une protéine du bactériophage CNCM I-5409, d'au moins une protéine du bactériophage CNCM I-5410 et d'au moins une protéine du bactériophage CNCM I-5680 ; d'au moins une protéine du bactériophage CNCM I-5408, d'au moins une protéine du bactériophage CNCM I-5409, d'au moins une protéine du bactériophage CNCM I-5491 et d'au moins une protéine du bactériophage CNCM I-5680 ; d'au moins une protéine du bactériophage CNCM I-5408, d'au moins une protéine du bactériophage CNCM I-5409, d'au moins une protéine du bactériophage CNCM I-5492 et d'au moins une protéine du bactériophage CNCM I-5680.

En particulier, il peut s'agir de cinq protéines, en particulier des RBP, provenant d'au moins un bactériophage spécifique de *S. aureus,* lesdits bactériophages spécifiques de *S. aureus* étant choisis parmi les bactériophages déposés sous les numéros CNCM I-5408, CNCM I-5409, CNCM I-5410, CNCM I-5491, CNCM I-5492 et CNCM I-5680.

En particulier, il peut s'agir de cinq protéines, en particulier des RBP, provenant de bactériophages spécifiques de *S. aureus* différents, lesdits bactériophages spécifiques de *S. aureus* étant choisis parmi les bactériophages déposés sous les numéros CNCM I-5408, CNCM I-5409, CNCM I-5410, CNCM I-5491, CNCM I-5492 et CNCM I-5680.

Par exemple, il s'agit d'au moins une protéine du bactériophage CNCM I-5408, d'au moins une protéine du bactériophage CNCM I-5409, d'au moins une protéine du bactériophage CNCM I-5410, d'au moins une protéine du bactériophage CNCM I-5491 et d'au moins une protéine du bactériophage CNCM I-5492.

En particulier, il peut s'agir de six protéines, en particulier des RBP, provenant d'au moins un bactériophage spécifique de *S. aureus,* lesdits bactériophages spécifiques de *S. aureus* étant choisis parmi les bactériophages déposés sous les numéros CNCM I-5408, CNCM I-5409, CNCM I-5410, CNCM I-5491, CNCM I-5492 et CNCM I-5680.

En particulier, il peut s'agir de six protéines, en particulier des RBP, provenant de bactériophages spécifiques de *S. aureus* différents, lesdits bactériophages spécifiques de *S. aureus* étant les bactériophages déposés sous les numéros CNCM I-5408, CNCM I-5409, CNCM I-5410, CNCM I-5491, CNCM I-5492 et CNCM I-5680.

Dans un mode particulier, ladite au moins une protéine d'au moins un bactériophage spécifique d'une bactérie d'intérêt ou ladite au moins une protéine d'au moins un bactériophage spécifique de *S. aureus* est donc une protéine de liaison (RBP, receptor binding protein). Plus particulièrement, il s'agit d'au moins une protéine RBP d'au moins un bactériophage spécifique de *S. aureus* choisi parmi les bactériophages spécifiques de *S. aureus* déposés à la Collection Nationale de Cultures de Microorganismes (CNCM) sous les numéros CNCM I-5408, CNCM I-5409, CNCM I-5410, CNCM I-5491, CNCM I-5492 et CNCM I-5680.

Encore plus particulièrement, la protéine RBP du bactériophage CNCM I-5408 est la protéine SEQ ID NO :1 ; la protéine RBP du bactériophage CNCM I-5409 est la protéine SEQ ID NO :2 ; la protéine RBP du bactériophage CNCM I-5410 est la protéine SEQ ID NO :3 ; la protéine RBP du bactériophage CNCM I-5680 est la protéine SEQ ID NO :4, la protéine RBP du bactériophage CNCM I-5491 est la protéine SEQ ID NO :5, la protéine RBP du bactériophage CNCM I-5492 est la protéine SEQ ID NO :6.

Ainsi, toutes les combinaisons décrites précédemment de 2, 3, 4, 5 ou 6 protéines de phages CNCM I-5408, CNCM I-5409, CNCM I-5410, CNCM I-5491, CNCM I-5492 et/ou CNCM I-5680, se lisent en particulier avec ces séquences spécifiques de protéines.

Ainsi, en particulier ladite au moins une protéine d'au moins un bactériophage spécifique est une protéine spécifique de *S. aureus* choisi parmi les protéines de séquences : SEQ ID NO :1, SEQ ID NO :2, SEQ ID NO :3, SEQ ID NO :4, SEQ ID NO :5 et SEQ ID NO :6.

En particulier, ladite au moins une protéine d'au moins un bactériophage spécifique est une protéine spécifique de *S. aureus* ayant au moins 70% d'identité avec la protéine de séquence SEQ ID NO :1, SEQ ID NO :2, SEQ ID NO :3, SEQ ID NO :4, SEQ ID NO :5 ou SEQ ID NO :6, en particulier au moins 80%, plus particulièrement au moins 90%, et encore plus particulièrement au moins 95% d'identité.

Les protéines de liaison au récepteur de phage (RBP) déterminent de la spécificité d'un phage pour une bactérie hôte. Chez les phages, ces protéines se trouvent au niveau de la queue et sont impliquées dans la première étape du cycle d'infection des phages (lytique ou lysogéniques). Elles assurent le contact initial avec le récepteur sur l'enveloppe de la cellule hôte. En effet, les RBP reconnaissent des motifs cibles à la surface des bactéries et permettent ainsi la fixation spécifique d'un phage sur une bactérie hôte. Les RBP de chaque souche de phage sont différentes et déterminent ainsi le spectre d'activité de chaque phage (capacité de reconnaitre plusieurs souches bactérienne).

Les protéines RBP sont différentes des domaines de liaison à la paroi cellulaire (CBD, Cell Binding Domain). Les RBP sont impliquées dans la première étape du cycle d'infection des phages, alors que les CBD sont impliquées en fin du cycle d'infection. Les CDB sont des domaines protéiques retrouvées dans enzymes lytiques produites par les phages, qui permettent la reconnaissance des peptidoglycanes dans les parois cellulaires. Par exemple, l'endolysine est une enzyme produite au stade final du cycle lytique pour cliver la paroi cellulaire bactérienne et libérer le virion descendant. Les endolysines ciblant les bactéries Gram-positives ont généralement une structure modulaire comprenant un domaine enzymatiquement actif (EAD) pour l'hydrolyse et un domaine de liaison à la paroi cellulaire (CBD) pour la reconnaissance des peptidoglycanes. Les endolysines reconnaissent spécifiquement la structure du peptidoglycane (PG) de la bactérie hôte et hydrolysent ensuite la paroi cellulaire en fragments, conduisant à la lyse osmotique des cellules.

La spécificité de reconnaissance d'une protéine de phage pour une bactérie peut être établie par la mise en présence de la bactérie et de billes magnétiques recouvertes d'une protéine de phage, suivie de la récupération des billes magnétiques grâce à un support aimanté, puis après resuspension dans un tampon, étalement sur un milieu de culture (en particulier une gélose spécifique). Après 24 à 48h à une température appropriée pour la bactérie, une différence significative entre le dénombrement de colonies bactériennes sur le milieu de culture où les billes magnétiques recouvertes de la protéine ont été étalées et le dénombrement des colonies bactériennes sur un milieu de culture où des billes magnétiques incubées avec la bactérie mais non recouvertes de la protéine, signifie que la protéine de phage est capable de se lier à la bactérie.

La spécificité peut également être évaluée par le test ELISA tel décrit dans la description. Les protéines RBP du phage sont fixées au fond des puits d'une plaque. Les solutions ou échantillons contenant les bactéries sont ensuite incubés et une étape de lavage est réalisée pour enlever les bactéries non fixées. Une étape d'incubation avec un anticorps anti*-S.aureus* couplé à HRP ou un autre substrat est alors réalisé pour révéler la présence de la bactérie.

Dans la présente description, les termes « bactériophage » et phage » désignent la même entité, elles sont utilisées de manière indifférente et sont interchangeables.

Dans la présente description, les expressions « anticorps dirigé contre X » et « anticorps anti-X » sont équivalentes et interchangeables.

Dans la présente description, les expressions « X conjugué à Y » et Y conjugué à X » sont équivalentes. Le terme « marqué » signifie « conjugué à un marqueur ».

### EXPOSE DE L'INVENTION

Un premier aspect de la présente divulgation porte sur une bandelette de chromatographie à flux latéral pour détecter une bactérie d'intérêt dans un échantillon.

Au sens de la présente divulgation, un échantillon désigne toute solution susceptible de contenir la bactérie d'intérêt que l'on cherche à détecter.

Les modes de réalisation de bandelette de chromatographie à flux latéral décrits ci-après sont décrits avant utilisation de ladite bandelette, c'est-à-dire avant mise en contact avec l'échantillon à analyser.

Ainsi, la présente divulgation concerne une bandelette de chromatographie à flux latéral pour détecter une bactérie d'intérêt, en particulier *S. aureus,* dans un échantillon, comprenant au moins une partie qui comprend au moins un bactériophage spécifique de la bactérie d'intérêt et/ou au moins une protéine d'un bactériophage spécifique de la bactérie d'intérêt.

La présente invention concerne une bandelette de chromatographie à flux latéral pour détecter *S. aureus* dans un échantillon caractérisée en qu'elle comprend au moins une partie qui comprend au moins une protéine d'un bactériophage spécifique de *S. aureus,* ladite au moins une protéine étant choisie parmi une protéine de séquence SEQ ID NO :1, une protéine de séquence SEQ ID NO :2, une protéine de séquence SEQ ID NO :3, une protéine de séquence SEQ ID NO :4, une protéine de séquence SEQ ID NO :5, une protéine de séquence SEQ ID NO :6, et une protéine ayant au moins 70% d'identité avec l'une des protéines de séquence SEQ ID NOs :1- 6.

Ladite partie comprenant au moins un bactériophage spécifique de la bactérie d'intérêt et/ou au moins une protéine d'un bactériophage spécifique de la bactérie d'intérêt pouvant être une partie de marquage et/ou une partie de détection.

Dans un mode de réalisation particulier, ladite bandelette de chromatographie à flux latéral comprend dans l'ordre au moins :
- une partie de contact comprenant une zone de contact avec l'échantillon ;
- une partie de détection comprenant une zone de détection de la bactérie d'intérêt qui comprend au moins un bactériophage spécifique de ladite bactérie d'intérêt et/ou au moins une protéine d'au moins un bactériophage spécifique de ladite bactérie d'intérêt,
- une partie absorbante.

Ainsi, la présente invention porte sur une bandelette de chromatographie à flux latéral comprend dans l'ordre au moins :
- une partie de contact comprenant une zone de contact avec l'échantillon,
- une partie de détection comprenant une zone de détection de *S. aureus* qui comprend au moins une protéine d'un bactériophage spécifique de *S. aureus,*
- une partie absorbante,
ladite au moins une protéine étant choisie parmi une protéine de séquence SEQ ID NO :1, une protéine de séquence SEQ ID NO :2, une protéine de séquence SEQ ID NO :3, une protéine de séquence SEQ ID NO :4, une protéine de séquence SEQ ID NO :5, une protéine de séquence SEQ ID NO :6, et une protéine ayant au moins 70% d'identité avec l'une des protéines de séquence SEQ ID NOs :1- 6.

La partie de contact (sample pad) comprend une zone de contact destinée à être mise en contact avec l'échantillon. Sa fonction est de répartir uniformément l'échantillon pour que la migration de l'échantillon par capillarité vers les parties suivantes soit lisse, continue et homogène. La partie de contact et la zone de contact sont une matrice capillaire. Par exemple, la partie de contact et la zone de contact sont en cellulose ou en fibres de verre.

La partie de détection (detection pad) comprend une zone de détection où la bactérie d'intérêt, si elle est présente dans l'échantillon analysé, est captée sur la bandelette grâce à un ligand spécifique de celle-ci qui y est immobilisé. Le rôle de la partie de détection est donc de fournir un bon support et une bonne liaison pour l'immobilisation des ligands spécifiques, tout en limitant les adsorptions non spécifiques. Par exemple, la partie de détection et la zone de détection sont une membrane de nitrocellulose.

En particulier, ladite au moins une protéine de bactériophage spécifique de la bactérie d'intérêt est une protéine RBP.

La partie absorbante (absorbent pad) a un rôle d'absorption. Elle contribue ainsi à maintenir le débit du flux au travers de la bandelette et permet de récupérer les excédents d'échantillon et de réactifs, évitant ainsi le reflux d'échantillon. Par exemple, la partie absorbante est en coton, en coton mélangé avec des fibres de verre, en cellulose ou en nitrocellulose.

Au sens de la présente description « dans l'ordre » signifie l'ordre dans lequel les différentes parties qui composent la bandelette vont être traversées par le flux contenant l'échantillon, mais ce terme ne signifie pas que ces parties sont forcément contiguës.

Dans un mode de réalisation particulier, l'une des extrémités de la partie de contact est superposée avec l'une des extrémités de la partie en aval de celle-ci pour former une première zone de superposition, l'une des extrémités de la partie de détection est superposée avec l'une des extrémités de la partie en amont de celle-ci pour former une deuxième zone de superposition et l'autre extrémité de la partie de détection est superposée avec l'une des extrémités de la partie en aval de celle-ci pour former une troisième zone de superposition, et l'une des extrémités de la partie absorbante est superposée avec l'une des extrémités de la partie en aval de celle-ci pour former une quatrième zone de superposition. En particulier, la première, la deuxième et la troisième zone de superposition sont distinctes les unes des autres. En particulier, la zone de contact est distincte de la première zone de superposition. En particulier, la zone de détection est distincte de la deuxième et de la troisième zone de superposition.

Dans un mode de réalisation particulier, l'une des extrémités de la partie de détection est superposée avec l'une des extrémités de la partie de contact et l'autre extrémité de la partie de détection est superposée avec l'une des extrémités de la partie absorbante.

Dans un mode de réalisation plus particulier, l'une des extrémités de la partie de détection est superposée avec l'une des extrémités de la partie de contact pour former une première zone de superposition, et l'autre extrémité de la partie de détection est superposée avec l'une des extrémités de la partie absorbante pour former une deuxième zone de superposition. En particulier, la première, la deuxième et la troisième zone de superposition sont séparées les unes des autres. En particulier, la zone de contact est distincte de la première zone de superposition. En particulier, la zone de détection est distincte de la première et de la deuxième zone de superposition.

Dans un mode de réalisation particulier, la zone de détection est une ligne transversale à la bandelette, formant ainsi une ligne de détection ou ligne de test (test line).

Optionnellement, la bandelette comprend un système de contrôle de la migration de l'échantillon de la partie de contact jusqu'à la partie de détection. Ainsi, optionnellement, la partie de détection comprend une zone de contrôle, de préférence en aval de la zone de détection. Cette zone de contrôle est distincte de la zone de détection. En particulier, elle est également distincte des zones de superpositions. En particulier, la zone de contrôle est une ligne transversale à la bandelette, formant ainsi une ligne de contrôle ou ligne témoin (control line). La zone de contrôle comprend un ligand spécifique d'un analyte autre que la bactérie d'intérêt. L'analyte est conjugué à un marqueur. Optionnellement, l'analyte conjugué à un marqueur est déposé sur une partie de la bandelette en amont de la partie de détection et/ou est ajouté préalablement à l'échantillon. Par exemple, l'analyte marqué est un anticorps primaire conjugué à un marqueur et le ligand sur la zone de contrôle est un anticorps secondaire dirigé contre l'anticorps primaire conjugué à un marqueur.

Dans un mode de réalisation plus particulier, la présente divulgation porte sur une bandelette de chromatographie à flux latéral pour détecter *S. aureus* dans un échantillon, comprenant dans l'ordre au moins :
- une partie de contact comprenant une zone de contact avec l'échantillon ;
- une partie de détection comprenant une zone de détection de *S. aureus* qui comprend au moins un bactériophage spécifique de *S. aureus* et/ou au moins une protéine d'au moins un bactériophage spécifique de *S. aureus,* en particulier ladite au moins une protéine est une RBP,
- une partie absorbante.

En particulier, la zone de détection comprend un phage CNCM I-5408, un phage CNCM I-5409, un phage CNCM I-5410, un phage CNCM I-5491, un phage CNCM I-5492 et /ou un phage CNCM I-5680. En particulier, la zone de détection comprend un phage CNCM I-5408, un phage CNCM I-5409 et un phage CNCM I-5410. En particulier, la zone de détection comprend un phage CNCM I-5408 et un phage CNCM I-5680.

En particulier, la zone de détection comprend au moins une protéine du phage CNCM I-5408, au moins une protéine du phage CNCM I-5409, au moins une protéine du phage CNCM I-5410, au moins une protéine du phage CNCM I-5491, au moins une protéine du phage CNCM I-5492 et/ou au moins une protéine du phage CNCM I-5680. En particulier ladite au moins une protéine de phage est une RBP. En particulier, la zone de détection comprend au moins une protéine du phage CNCM I-5408, au moins une protéine du phage CNCM I-5409 et au moins une protéine du phage CNCM I-5410. En particulier, la zone de détection comprend au moins une protéine du phage CNCM I-5408, au moins une protéine du phage CNCM I-5410 et/ou au moins une protéine du phage CNCM I-5680.

Dans un mode de réalisation particulier, ladite bandelette comprend en outre, entre la partie de contact et la partie de détection, une partie de marquage qui comprend une zone de marquage.

La partie de marquage (conjugate pad) comprend une zone de marquage où les bactéries sont marquées avec un marqueur lorsque le flux d'échantillon à analyser la traverse. Leur rôle est de maintenir le marqueur jusqu'à utilisation de la bandelette et de libérer le marqueur lorsqu'il y a reconnaissance entre le marqueur et une bactérie, au passage du flux d'échantillon. Par exemple la partie de marquage et la zone de marquage sont en fibres de verre, cellulose ou polyesters.

Ainsi, la présente description porte sur une bandelette de chromatographie à flux latéral pour détecter une bactérie d'intérêt dans un échantillon, comprenant dans l'ordre au moins :
- une partie de contact comprenant une zone de contact avec l'échantillon ;
- une partie de marquage comprenant une zone de marquage qui comprend un marqueur ;
- une partie de détection comprenant une zone de détection de la bactérie d'intérêt qui comprend au moins un bactériophage spécifique de ladite bactérie d'intérêt et/ou au moins une protéine d'au moins un bactériophage spécifique de ladite bactérie d'intérêt, en particulier ladite au moins une protéine de phage est une RBP,
- une partie absorbante.

Le marqueur peut être tout marqueur apte à marquer une bactérie. Les marqueurs de bactéries sont bien connus de l'Homme du métier.

Le terme « marqueur » englobe les marqueurs non conjugués et les marqueurs conjugués.

Au sens de la présente description, un marqueur non conjugué est un marqueur libre, c'est-à-dire qu'il n'est pas lié à un ligand pour marquer la bactérie. Il s'agit par exemple de colorants fluorescents. Les colorants fluorescents capables de marquer des bactéries sont bien connus de l'Homme du métier, tels que le DAPI (4',6-diamidino-2-phénylindole) ou le SYT09 à titre d'exemples.

Un marqueur conjugué peut être par exemple : une nanoparticule d'or (AuNP) ou or colloïdale, une nanoparticule de carbone ou carbone colloïdal, une bille de latex colorée, une particule magnétique, une boite quantique (quantum dots), un phosphore de conversion ascendante (upconverting phosphors, UCP), une molécule luminescente incluant les molécules fluorescentes (fluorophores) et les molécules phosphorescentes, un colorant, un liposome ou une enzyme telles que la peroxydase de raifort (HRP) qui catalyse la conversion de substrats chromogènes en composés colorés, ou qui produit de la lumière à partir de substrats chimioluminescents. Ces marqueurs conjugués sont connus de l'art antérieur (Sajid et al., 2015).

En particulier, le marqueur est une nanoparticule d'or (AuNP). Par « nanoparticule d'or » on entend qu'il peut s'agit d'une nanosphère d'or ou d'un nanobâtonnet d'or. L'agrégation des nanoparticules d'or entraine l'apparition d'une couleur détectable dans le visible, qui varie en fonction de la taille des nanoparticules.

En particulier, le marqueur est l'HRP.

Au sens de la présente description, un marqueur conjugué signifie que le marqueur est lié à un ligand pour marquer la bactérie. En particulier, le marqueur conjugué est un marqueur conjugué à au moins un ligand spécifique de la bactérie d'intérêt. Par « conjugué à au moins un ligand spécifique », on entend qu'un marqueur peut être conjugué à un ligand spécifique ou à plusieurs ligands spécifiques différents.

Pour lier un marqueur à un ligand, une liaison biotine/streptavidine peut par exemple être utilisée.

Sauf mention contraire, au sens de la présente divulgation, ledit au moins un ligand spécifique de la bactérie d'intérêt peut être au moins un anticorps dirigé contre la bactérie d'intérêt, au moins un aptamère spécifique de la bactérie d'intérêt, au moins un bactériophage spécifique de la bactérie d'intérêt, au moins une protéine d'au moins un bactériophage spécifique de la bactérie d'intérêt, au moins un bactériophage spécifique de la bactérie d'intérêt couplé à un anticorps dirigé contre la bactérie d'intérêt, au moins une protéine d'un bactériophage spécifique de la bactérie d'intérêt couplée à un anticorps dirigé contre la bactérie d'intérêt. En particulier, ladite au moins une protéine d'au moins un bactériophage spécifique de la bactérie d'intérêt est une protéine RBP.

En particulier, la bactérie d'intérêt est *S. aureus* et ces ligands spécifiques se lisent comme des ligands spécifiques de *S. aureus* ou dirigés contre *S. aureus.* En particulier, lorsque le ligand est spécifique de *S. aureus,* ledit au moins un bactériophage spécifique de *S. aureus* est un bactériophage CNCM I-5408, un bactériophage CNCM I-5409, un bactériophage CNCM I-5410, un bactériophage CNCM I-5491,un bactériophage CNCM I-5492 et/ou un bactériophage CNCM I-5680; et ladite au moins une protéine d'au moins un bactériophage est au moins une protéine du bactériophage CNCM I-5408, au moins une protéine du bactériophage CNCM I-5409, au moins une protéine du bactériophage CNCM I-5410, au moins une protéine du bactériophage CNCM I-5491, au moins une protéine du bactériophage CNCM I-5492 et/ou au moins une protéine du bactériophage CNCM I-5680. En particulier, ladite au moins une protéine de ces bactériophages est une RBP. Plus particulièrement, ladite au moins une protéine du phage CNCM I-5408 est la protéine SEQ ID NO :1, ladite au moins une protéine du phage CNCM I-5409 est la protéine SEQ ID NO :2, ladite au moins une protéine du phage CNCM I-5410 est la protéine SEQ ID NO :3, ladite au moins une protéine du phage CNCM I-5680 est la protéine SEQ ID NO :4, ladite au moins une protéine du phage CNCM I-5491 est la protéine SEQ ID NO :5, ladite au moins une protéine du phage CNCM I-5492 est la protéine SEQ ID NO :6.

En particulier, ladite au moins une protéine est une protéine ayant au moins 70% d'identité avec la protéine de séquence SEQ ID NO :1, SEQ ID NO :2, SEQ ID NO :3, SEQ ID NO :4, SEQ ID NO :5 ou SEQ ID NO :6, en particulier au moins 80%, plus particulièrement au moins 90%, et encore plus particulièrement au moins 95% d'identité. En effet, l'efficacité des protéines de séquence SEQ ID NO : 1 et de séquence SEQ ID NO :3 est démontrée dans la présente description pour la détection de *S. aureus.* Ces deux séquences ayant 68% d'identité, cela supporte qu'une protéine ayant au moins 70% d'identité avec la séquence SEQ ID NO :1 ou SEQ ID NO :3, mais également avec les séquences SEQ ID NO :2, SEQ ID NO :4, SEQ ID NO :5 ou SEQ ID NO :6 serait également efficace pour la détection.

Ainsi, le marqueur conjugué à un ligand spécifique de la bactérie d'intérêt, en particulier *S. aureus,* peut être un marqueur conjugué à un anticorps dirigé contre la bactérie d'intérêt, un marqueur conjugué à un aptamère spécifique de la bactérie d'intérêt, un marqueur conjugué à un phage spécifique de la bactérie d'intérêt, un marqueur conjugué à une protéine d'un phage spécifique de la bactérie d'intérêt, un marqueur conjugué à un phage spécifique de la bactérie d'intérêt couplé à un anticorps dirigé contre la bactérie d'intérêt, un marqueur conjugué à une protéine d'un phage spécifique de la bactérie d'intérêt couplée à un anticorps dirigé contre la bactérie d'intérêt, un marqueur conjugué à un anticorps dirigé contre la bactérie d'intérêt couplé à un phage spécifique de la bactérie d'intérêt ou un marqueur conjugué à un anticorps dirigé contre la bactérie d'intérêt couplé à une protéine d'un phage spécifique de la bactérie d'intérêt.

Dans un mode de réalisation plus particulier, la présente description porte sur une bandelette de chromatographie à flux latéral pour détecter *S. aureus* dans un échantillon, comprenant dans l'ordre au moins :
- une partie de contact comprenant une zone de contact avec l'échantillon ;
- une partie de marquage comprenant une zone de marquage qui comprend un marqueur conjugué à au moins un ligand spécifique de *S. aureus,* en particulier une nanoparticule d'or conjuguée à au moins un ligand spécifique de *S*. *aureus;*
- une partie de détection comprenant une zone de détection de *S. aureus* qui comprend au moins un bactériophage spécifique de *S. aureus* et/ou une protéine d'au moins un bactériophage spécifique de *S. aureus,* en particulier une protéine RBP,
- une partie absorbante.

En particulier, la zone de marquage comprend un marqueur conjugué à un anticorps anti-*S. aureus* ou un marqueur conjugué à un aptamère spécifique de *S. aureus,* plus particulièrement une nanoparticule d'or conjugué à un anticorps anti- *S. aureus* ou une nanoparticule d'or conjugué à un aptamère spécifique de *S. aureus.*

En particulier, la zone de marquage comprend un marqueur conjugué à au moins un bactériophage spécifique de *S. aureus.* En particulier, elle comprend un marqueur conjugué au phage CNCM I-5408, un marqueur conjugué au phage CNCM I-5409, un marqueur conjugué au phage CNCM I-5410, un marqueur conjugué au phage CNCM I-5491, un marqueur conjugué au phage CNCM I-5492, et/ou un marqueur conjugué au phage CNCM I-5680. En particulier, elle comprend un marqueur conjugué au phage CNCM I-5408, un marqueur conjugué au phage CNCM I-5409 et un marqueur conjugué au phage CNCM I-5410. En particulier, elle comprend un marqueur conjugué au phage CNCM I-5408 et un marqueur conjugué au phage CNCM I-5680. Plus particulièrement, ledit marqueur conjugué à ces phages est une nanoparticule d'or ou l'HRP.

En particulier, la zone de marquage comprend un marqueur conjugué à au moins une protéine d'au moins un bactériophage spécifique de S. *aureus.* En particulier, ladite au moins une protéine est une RBP. En particulier, la zone de marquage comprend un marqueur conjugué à au moins une protéine du phage CNCM I-5408, un marqueur conjugué à au moins une protéine du phage CNCM I-5409, un marqueur conjugué à au moins une protéine du phage CNCM I-5410, un marqueur conjugué à au moins une protéine du phage CNCM I-5491, un marqueur conjugué à au moins une protéine du phage CNCM I-5492, et/ou un marqueur conjugué à au moins une protéine du phage CNCM I-5680. En particulier, la zone de marquage comprend un marqueur conjugué à au moins une protéine du phage CNCM I-5408, un marqueur conjugué à au moins une protéine du phage CNCM I-5409, et un marqueur conjugué à au moins une protéine du phage CNCM I-5410. En particulier, la zone de marquage comprend un marqueur conjugué à au moins une protéine du phage CNCM I-5408, un marqueur conjugué à au moins une protéine du phage CNCM I-5410 et/ou un marqueur conjugué à au moins une protéine du phage CNCM I-5680.

En particulier, dans ces exemples de conjugaison du marqueur, ladite au moins une protéine du phage CNCM I-5408 est la protéine SEQ ID NO :1, ladite au moins une protéine du phage CNCM I-5409 est la protéine SEQ ID NO :2, ladite au moins une protéine du phage CNCM I-5410 est la protéine SEQ ID NO :3, ladite au moins une protéine du phage CNCM I-5680 est la protéine SEQ ID NO :4, ladite au moins une protéine du phage CNCM I-5491 est la protéine SEQ ID NO :5 et ladite au moins une protéine du phage CNCM I-5492 est la protéine SEQ ID NO :6. En particulier, la protéine est une protéine ayant au moins 70% d'identité avec la protéine de séquence SEQ ID NO :1, SEQ ID NO :2, SEQ ID NO :3, SEQ ID NO :4, SEQ ID NO :5 ou SEQ ID NO :6, en particulier au moins 80%, plus particulièrement au moins 90%, et encore plus particulièrement au moins 95% d'identité. Typiquement, ledit marqueur conjugué aux protéines de phages est une nanoparticule d'or ou l'HRP.

En particulier, la zone de marquage comprend un marqueur conjugué au phage CNCM I-5408 couplé à un anticorps anti- *S. aureus,* un marqueur conjugué au phage CNCM I-5409 couplé à un anticorps anti- *S. aureus,* un marqueur conjugué au phage CNCM I-5410 couplé à un anticorps anti- *S. aureus,* un marqueur conjugué au phage CNCM I-5491 couplé à un anticorps anti- *S. aureus,* un marqueur conjugué au phage CNCM I-5492 couplé à un anticorps anti- *S. aureus,* et/ou un marqueur conjugué au phage CNCM I-5680 couplé à un anticorps anti- *S. aureus.* Encore plus particulièrement, ledit marqueur conjugué au phage est une nanoparticule d'or ou l'HRP.

En particulier, elle comprend un marqueur conjugué à une protéine du phage CNCM I-5408 couplé à un anticorps anti- *S. aureus,* un marqueur conjugué à une protéine du phage CNCM I-5409 couplé à un anticorps anti- *S. aureus,* un marqueur conjugué à une protéine du phage CNCM I-5410 couplé à un anticorps anti- *S. aureus,* un marqueur conjugué à une protéine du phage CNCM I-5491 couplé à un anticorps anti- *S. aureus,* un marqueur conjugué à une protéine du phage CNCM I-5492 couplé à un anticorps anti- *S. aureus,* et/ou un marqueur conjugué à une protéine du phage CNCM I-5680 couplé à un anticorps anti- *S. aureus.* En particulier, ladite protéine de phage est une RBP. Encore plus particulièrement, ledit marqueur conjugué à une protéine de phage est une nanoparticule d'or ou l'HRP.

En particulier, la zone de marquage comprend un marqueur conjugué à un anticorps anti-*S. aureus* couplé au phage CNCM I-5408, un marqueur conjugué à un anticorps anti- *S. aureus* couplé au phage CNCM I-5409, un marqueur conjugué à un anticorps anti- *S. aureus* couplé au phage CNCM I-5410, un marqueur conjugué à un anticorps anti- *S. aureus* couplé au phage CNCM I-5491, un marqueur conjugué à un anticorps anti- *S. aureus* couplé au phage CNCM I-5492, et/ou un marqueur conjugué à un anticorps anti- *S. aureus* couplé au phage CNCM I-5680.

En particulier, la zone de marquage comprend un marqueur conjugué à un anticorps anti-*S. aureus* couplé à une protéine du phage CNCM I-5408, un marqueur conjugué à un anticorps anti-*S. aureus* couplé à une protéine du phage CNCM I-5409, un marqueur conjugué à un anticorps anti-*S. aureus* couplé à une protéine du phage CNCM I-5410, un marqueur conjugué à un anticorps anti-*S. aureus* couplé à une protéine du phage CNCM I-5491, un marqueur conjugué à un anticorps anti-*S. aureus* couplé à une protéine du phage CNCM I-5492, et/ou un marqueur conjugué à un anticorps anti- *S. aureus* couplé à une protéine du phage CNCM I-5680. En particulier, ladite protéine de phage est une RBP.

En particulier, dans ces exemples de conjugaison du marqueur, ladite au moins une protéine du phage CNCM I-5408 est la protéine SEQ ID NO :1, ladite au moins une protéine du phage CNCM I-5409 est la protéine SEQ ID NO :2, ladite au moins une protéine du phage CNCM I-5410 est la protéine SEQ ID NO :3, ladite au moins une protéine du phage CNCM I-5680 est la protéine SEQ ID NO :4, ladite au moins une protéine du phage CNCM I-5491 est la protéine SEQ ID NO :5, et ladite au moins une protéine du phage CNCM I-5492 est la protéine SEQ ID NO :6. En particulier, la protéine est une protéine ayant au moins 70% d'identité avec la protéine de séquence SEQ ID NO :1, SEQ ID NO :2, SEQ ID NO :3, SEQ ID NO :4, SEQ ID NO :5 ou SEQ ID NO :6, en particulier au moins 80%, plus particulièrement au moins 90%, et encore plus particulièrement au moins 95% d'identité. Typiquement, ledit marqueur conjugué à un anticorps anti- *S. aureus* est une nanoparticule d'or ou l'HRP.

En particulier, la zone de détection comprend un phage CNCM I-5408, un phage CNCM I-5409, un phage CNCM I-5410, un phage CNCM I-5491, CNCM I-5492, et/ou CNCM I-5680. En particulier, la zone de détection comprend un phage CNCM I-5408, un phage CNCM I-5409 et un phage CNCM I-5410. En particulier, la zone de détection comprend un phage CNCM I-5408 et un phage CNCM I-5680.

En particulier, la zone de détection comprend au moins une protéine du phage CNCM I-5408, au moins une protéine du phage CNCM I-5409, au moins une protéine du phage CNCM I-5410, au moins une protéine du phage CNCM I-5491, au moins une protéine du phage CNCM I-5492, et/ou au moins une protéine du phage CNCM I-5680. En particulier, ladite protéine de phage est une RBP. En particulier, la zone de détection comprend au moins une protéine du phage CNCM I-5408, au moins une protéine du phage CNCM I-5409 et au moins une protéine du phage CNCM I-5410. En particulier, ladite au moins une protéine du phage CNCM I-5408 est la protéine SEQ ID NO :1, ladite au moins une protéine du phage CNCM I-5409 est la protéine SEQ ID NO :2, ladite au moins une protéine du phage CNCM I-5410 est la protéine SEQ ID NO :3, ladite au moins une protéine du phage CNCM I-5680 est la protéine SEQ ID NO :4, ladite au moins une protéine du phage CNCM I-5491 est la protéine SEQ ID NO :5, ladite au moins une protéine du phage CNCM I-5492 est la protéine SEQ ID NO :6. Ainsi, plus particulièrement, la zone de détection comprend une protéine du phage CNCM I-5408 de SEQ ID NO :1, une protéine du phage CNCM I-5409 de SEQ ID NO :2, une protéine du phage CNCM I-5410 de SEQ ID NO :3, une protéine du phage CNCM I-5680 de SEQ ID NO :4, une protéine du phage CNCM I-5491 de SEQ ID NO :5 et/ou une protéine du phage CNCM I-5492 de SEQ ID NO :6.

En particulier, la zone de détection comprend au moins une protéine ayant au moins 70% d'identité avec la protéine de séquence SEQ ID NO :1, SEQ ID NO :2, SEQ ID NO :3, SEQ ID NO :4, SEQ ID NO :5 ou SEQ ID NO :6, en particulier au moins 80%, plus particulièrement au moins 90%, et encore plus particulièrement au moins 95% d'identité.

Dans un mode de réalisation particulier, la présente invention porte sur une bandelette de chromatographie à flux latéral pour détecter *S. aureus* dans un échantillon, comprenant dans l'ordre au moins :
- une partie de contact comprenant une zone de contact avec l'échantillon ;
- une partie de marquage comprenant une zone de marquage qui comprend un marqueur,
- une partie de détection comprenant une zone de détection de *S. aureus* qui comprend au moins une protéine d'au moins un bactériophage spécifique de *S. aureus,*
- une partie absorbante,
ladite au moins une protéine étant choisie parmi une protéine de séquence SEQ ID NO :1, une protéine de séquence SEQ ID NO :2, une protéine de séquence SEQ ID NO :3, une protéine de séquence SEQ ID NO :4, une protéine de séquence SEQ ID NO :5, une protéine de séquence SEQ ID NO :6, et une protéine ayant au moins 70% d'identité avec l'une des protéines de séquence SEQ ID NOs :1- 6.

Dans un autre mode de réalisation, ladite bandelette de chromatographie à flux latéral comprend dans l'ordre au moins :
- une partie de contact comprenant une zone de contact avec l'échantillon ;
- une partie de marquage comprenant une zone de marquage qui comprend un marqueur conjugué à au moins un ligand spécifique de la bactérie d'intérêt choisi parmi au moins un bactériophage spécifique de la bactérie d'intérêt, au moins une protéine d'au moins un bactériophage spécifique de la bactérie d'intérêt, au moins un bactériophage spécifique de la bactérie d'intérêt couplé à un anticorps dirigé contre la bactérie d'intérêt, au moins une protéine d'un bactériophage spécifique de la bactérie d'intérêt couplée à un anticorps dirigé contre la bactérie d'intérêt, en particulier ladite protéine de phage est une RBP.
- une partie de détection comprenant une zone de détection de la bactérie d'intérêt qui comprend au moins un ligand spécifique de la bactérie d'intérêt,
- une partie absorbante.

En particulier, ledit au moins un ligand spécifique de la bactérie d'intérêt compris dans la zone de détection est au moins un anticorps dirigé contre la bactérie d'intérêt, au moins un aptamère spécifique de la bactérie d'intérêt, au moins un bactériophage spécifique de la bactérie d'intérêt, au moins une protéine d'au moins un bactériophage spécifique de la bactérie d'intérêt, au moins un bactériophage spécifique de la bactérie d'intérêt couplé à un anticorps dirigé contre la bactérie d'intérêt, au moins une protéine d'un bactériophage spécifique de la bactérie d'intérêt couplée à un anticorps dirigé contre la bactérie d'intérêt.

Dans un mode de réalisation plus particulier, la présente description porte sur une bandelette de chromatographie à flux latéral pour détecter *S. aureus* dans un échantillon, comprenant dans l'ordre au moins :
- une partie de contact comprenant une zone de contact avec l'échantillon ;
- une partie de marquage comprenant une zone de marquage qui comprend un marqueur conjugué à au moins un ligand spécifique de *S. aureus* choisi parmi : au moins un bactériophage spécifique de *S. aureus,* au moins une protéine d'au moins un bactériophage spécifique *S. aureus,* au moins un bactériophage spécifique de *S. aureus* couplé à un anticorps dirigé contre *S. aureus,* au moins une protéine d'un bactériophage spécifique de *S. aureus* couplée à un anticorps dirigé contre *S. aureus*; en particulier ladite protéine de phage est une RBP ;
- une partie de détection comprenant une zone de détection de *S. aureus* qui comprend au moins un ligand spécifique de *S*. *aureus*, ;
- une partie absorbante.

En particulier, la zone de marquage comprend un marqueur conjugué à au moins un bactériophage spécifique de *S. aureus.* En particulier, elle comprend un marqueur conjugué au phage CNCM I-5408, un marqueur conjugué au phage CNCM I-5409, un marqueur conjugué au phage CNCM I-5410, un marqueur conjugué au phage CNCM I-5491, et/ou un marqueur conjugué au phage CNCM I-5492, et/ou un marqueur conjugué au phage CNCM I-5680. Plus particulièrement, elle comprend un marqueur conjugué au phage CNCM I-5408, un marqueur conjugué au phage CNCM I-5409 et un marqueur conjugué au phage CNCM I-5410. Plus particulièrement, elle comprend un marqueur conjugué au phage CNCM I-5408 et un marqueur conjugué au phage CNCM I-5680. Encore plus particulièrement, ledit marqueur conjugué à ces phages est une nanoparticule d'or ou l'HRP.

En particulier, la zone de marquage comprend un marqueur conjugué à au moins une protéine d'au moins un bactériophage spécifique de S. *aureus.* En particulier, ladite au moins une protéine est une RBP. En particulier, la zone de marquage comprend un marqueur conjugué à au moins une protéine du phage CNCM I-5408, un marqueur conjugué à au moins une protéine du phage CNCM I-5409, un marqueur conjugué à au moins une protéine du phage CNCM I-5410, un marqueur conjugué à au moins une protéine du phage CNCM I-5491, un marqueur conjugué à au moins une protéine du phage CNCM I-5492, et/ou un marqueur conjugué à au moins une protéine du phage CNCM I-5680. Plus particulièrement, elle comprend un marqueur conjugué à au moins une protéine du phage CNCM I-5408, un marqueur conjugué à au moins une protéine du phage CNCM I-5409 et un marqueur conjugué à au moins une protéine du phage CNCM I-5410. Plus particulièrement, elle comprend un marqueur conjugué à au moins une protéine du phage CNCM I-5408, un marqueur conjugué à au moins une protéine du phage CNCM I-5410 et/ou un marqueur conjugué à au moins une protéine du phage CNCM I-5680.

La zone de marquage peut également comprendre un marqueur conjugué tel que décrit aux paragraphes [0098] à [0102].

En particulier, dans ces exemples de conjugaison du marqueur, ladite au moins une protéine du phage CNCM I-5408 est la protéine SEQ ID NO :1, ladite au moins une protéine du phage CNCM I-5409 est la protéine SEQ ID NO :2, ladite au moins une protéine du phage CNCM I-5410 est la protéine SEQ ID NO :3, ladite au moins une protéine du phage CNCM I-5680 est la protéine SEQ ID NO :4, ladite au moins une protéine du phage CNCM I-5491 est la protéine SEQ ID NO :5, et ladite au moins une protéine du phage CNCM I-5492 est la protéine SEQ ID NO :6. En particulier, la protéine est une protéine ayant au moins 70% d'identité avec la protéine de séquence SEQ ID NO :1, SEQ ID NO :2, SEQ ID NO :3, SEQ ID NO :4, SEQ ID NO :5 ou SEQ ID NO :6, en particulier au moins 80%, plus particulièrement au moins 90%, et encore plus particulièrement au moins 95% d'identité. Typiquement, ledit marqueur conjugué à ces protéines de phages est une nanoparticule d'or ou l'HRP.

En particulier, la zone de détection comprend un anticorps anti- *S. aureus* ou un aptamère spécifique de *S. aureus.*

En particulier, la zone de détection comprend au moins un bactériophage spécifique de la bactérie d'intérêt, plus particulièrement un bactériophage CNCM I-5408, un bactériophage CNCM I-5409, un bactériophage CNCM I-5410, un bactériophage CNCM I-5491, un bactériophage CNCM I-5492, et/ou un bactériophage CNCM I-5680. Plus particulièrement, elle comprend un bactériophage CNCM I-5408, un bactériophage CNCM I-5409 et un bactériophage CNCM I-5410. Plus particulièrement, elle comprend un bactériophage CNCM I-5408 et un bactériophage CNCM I-5680.

En particulier, la zone de détection comprend au moins une protéine d'au moins un bactériophage spécifique de *S. aureus,* en particulier une RBP. Plus particulièrement, elle comprend au moins une protéine du bactériophage CNCM I-5408, au moins une protéine du bactériophage CNCM I-5409, au moins une protéine du bactériophage CNCM I-5410, au moins une protéine du bactériophage CNCM I-5491, au moins une protéine du bactériophage CNCM I-5492, et/ou au moins une protéine CNCM I-5680. Encore plus particulièrement, elle comprend au moins une protéine du bactériophage CNCM I-5408, au moins une protéine du bactériophage CNCM I-5409 et au moins une protéine du bactériophage CNCM I-5410. En particulier, ladite au moins une protéine du phage CNCM I-5408 est la protéine SEQ ID NO :1, ladite au moins une protéine du phage CNCM I-5409 est la protéine SEQ ID NO :2, ladite au moins une protéine du phage CNCM I-5410 est la protéine SEQ ID NO :3, ladite au moins une protéine du phage CNCM I-5680 est la protéine SEQ ID NO :4, ladite au moins une protéine du phage CNCM I-5491 est la protéine SEQ ID NO :5 et ladite au moins une protéine du phage CNCM I-5492 est la protéine SEQ ID NO :6. Ainsi, plus particulièrement, la zone de détection comprend une protéine du phage CNCM I-5408 de SEQ ID NO :1, une protéine du phage CNCM I-5409 de SEQ ID NO :2, une protéine du phage CNCM I-5410 de SEQ ID NO :3, une protéine du phage CNCM I-5680 de SEQ ID NO :4, une protéine du phage CNCM I-5491 de SEQ ID NO :5 et/ou une protéine du phage CNCM I-5492 de SEQ ID NO :6.

En particulier, la zone de détection comprend au moins un bactériophage spécifique de S. *aureus* couplé à un anticorps dirigé contre *S. aureus* et/ou au moins une protéine d'un bactériophage spécifique de *S. aureus* couplé à un anticorps dirigé contre *S. aureus,* en particulier ledit bactériophage est choisi parmi les bactériophages CNCM I-5408, CNCM I-5409, CNCM I-5410, CNCM I-5491, CNCM I-5492 et CNCM I-5680, et ladite au moins une protéine est choisie parmi les protéines de ces phages et est en particulier une RBP. En particulier, ladite au moins une protéine du phage CNCM I-5408 est la protéine SEQ ID NO :1, ladite au moins une protéine du phage CNCM I-5409 est la protéine SEQ ID NO :2, ladite au moins une protéine du phage CNCM I-5410 est la protéine SEQ ID NO :3, ladite au moins une protéine du phage CNCM I-5680 est la protéine SEQ ID NO :4, ladite au moins une protéine du phage CNCM I-5491 est la protéine SEQ ID NO :5 et ladite au moins une protéine du phage CNCM I-5492 est la protéine SEQ ID NO :6. En particulier, la protéine est une protéine ayant au moins 70% d'identité avec la protéine de séquence SEQ ID NO :1, SEQ ID NO :2, SEQ ID NO :3, SEQ ID NO :4, SEQ ID NO :5 ou SEQ ID NO :6, en particulier au moins 80%, plus particulièrement au moins 90%, et encore plus particulièrement au moins 95% d'identité.

Dans un mode de réalisation particulier, lorsque la bandelette comprend au moins quatre parties, l'une des extrémités de la partie de contact est superposée avec l'une des extrémités de la partie en aval de celle-ci pour former une première zone de superposition ; l'une des extrémités de la partie de marquage est superposée avec l'une des extrémités de la partie en amont de celle-ci pour former une deuxième zone de superposition et l'autre extrémité de la partie de marquage est superposée avec l'une des extrémités de la partie en aval de celle-ci pour former une troisième zone de superposition ; l'une des extrémités de la partie de détection est superposée avec l'une des extrémités de la partie en amont de celle-ci pour former une quatrième zone de superposition et l'autre extrémité de la partie de détection est superposée avec l'une des extrémités de la partie en aval de celle-ci pour former une cinquième zone de superposition ; et l'une des extrémités de la partie absorbante est superposée avec l'une des extrémités de la partie en aval de celle-ci pour former une sixième zone de superposition. En particulier, la première, la deuxième, la troisième, la quatrième, la cinquième et la sixième zone de superposition sont séparées les unes des autres. En particulier, la zone de contact est distincte de la première zone de superposition. En particulier, la zone de marquage est distincte de la deuxième et de la troisième zone de superposition. En particulier, la zone de détection est distincte de la quatrième et de la cinquième zone de superposition.

Dans un mode de réalisation particulier, l'une des extrémités de la partie de marquage est superposée à l'une des extrémités de la partie de contact et l'autre extrémité de la partie de marquage est superposée à l'une des extrémités de la partie de détection ; l'autre extrémité de la partie de détection étant superposée avec l'une des extrémités de la partie absorbante.

Dans un mode de réalisation particulier, l'une des extrémités de la partie de marquage est superposée à l'une des extrémités de la partie de contact pour former une première zone de superposition, et l'autre extrémité de la partie de marquage est superposée à l'une des extrémités de la partie de détection pour former une deuxième zone de superposition ; l'autre extrémité de la partie de détection étant superposée avec l'une des extrémités de la partie absorbante pour former une troisième zone de superposition. En particulier, la première, la deuxième et la troisième zone de superposition sont séparées les unes des autres. En particulier, la zone de contact est distincte de la première zone de superposition. En particulier, la zone de marquage est distincte de la première et de la deuxième zone de superposition. En particulier, la zone de détection est distincte de la deuxième et de la troisième zone de superposition.

Dans un mode de réalisation particulier, la zone de détection est une ligne transversale à la bandelette, formant ainsi une ligne de détection ou ligne de test (test line).

Optionnellement, la partie de détection peut comprendre en outre une zone de contrôle comme décrit précédemment.

Dans un mode de réalisation particulier des bandelettes selon la présente divulgation, la bactérie d'intérêt à détecter est *S. aureus,* et ledit au moins un bactériophage spécifique d'une bactérie d'intérêt est au moins un bactériophage spécifique de *S. aureus* choisi parmi le bactériophage CNCM I-5408, le bactériophage CNCM I-5409, le bactériophage CNCM I-5410, le bactériophage CNCM I-5491, le bactériophage CNCM I-5492 et/ou le bactériophage CNCM I-5680, et ladite au moins une protéine d'au moins un bactériophage spécifique d'une bactérie d'intérêt est au moins une protéine d'au moins un bactériophage spécifique de *S. aureus* choisie parmi au moins une protéine du bactériophage CNCM I-5408, en particulier la protéine SEQ ID NO :1, au moins une protéine du bactériophage CNCM I-5409, en particulier la protéine SEQ ID NO :2, au moins une protéine du bactériophage CNCM I-5410, en particulier la protéine SEQ ID NO :3, au moins une protéine du bactériophage CNCM I-5491, en particulier la protéine SEQ ID NO :5, au moins une protéine du bactériophage CNCM I-5492, en particulier la protéine SEQ ID NO :6, et/ou au moins une protéine du bactériophage CNCM I-5680, en particulier la protéine SEQ ID NO :4. En particulier, la protéine est une protéine ayant au moins 70% d'identité avec la protéine de séquence SEQ ID NO :1, SEQ ID NO :2, SEQ ID NO :3, SEQ ID NO :4, SEQ ID NO :5 ou SEQ ID NO :6, en particulier au moins 80%, plus particulièrement au moins 90%, et encore plus particulièrement au moins 95% d'identité.

Dans un mode de réalisation particulier, la présente invention porte sur une bandelette de chromatographie à flux latéral pour détecter *S. aureus* dans un échantillon, comprenant dans l'ordre au moins :
- une partie de contact comprenant une zone de contact avec l'échantillon ;
- une partie de marquage comprenant une zone de marquage qui comprend un marqueur conjugué à au moins un ligand spécifique de *S*. *aureus* choisi parmi au moins une protéine d'au moins un bactériophage spécifique de *S*. *aureus* et au moins une protéine d'un bactériophage spécifique de *S. aureus* couplée à un anticorps dirigé contre *S*. *aureus;*
   ladite au moins une protéine étant choisie parmi une protéine de séquence SEQ ID NO :1, une protéine de séquence SEQ ID NO :2, une protéine de séquence SEQ ID NO :3, une protéine de séquence SEQ ID NO :4, une protéine de séquence SEQ ID NO :5, une protéine de séquence SEQ ID NO :6, et une protéine ayant au moins 70% d'identité avec l'une des protéines de séquence SEQ ID NOs :1- 6.
- une partie de détection comprenant une zone de détection de *S*. *aureus* qui comprend au moins un ligand spécifique de *S*. *aureus ;*
- une partie absorbante.

Dans un mode de réalisation plus particulier, ladite au moins une protéine d'au moins un bactériophage spécifique de *S. aureus* est une protéine de liaison au récepteur de phage (RBP).

Un deuxième aspect de la présente divulgation porte sur un procédé de détection d'une bactérie d'intérêt dans un échantillon en utilisant les bandelettes de chromatographie à flux latéral décrites précédemment.

Un échantillon désigne toute solution susceptible de contenir la bactérie d'intérêt. L'échantillon peut être dilué dans une solution tampon préalablement à la mise en œuvre du procédé de détection.

Dans un mode de réalisation particulier, le procédé de détection d'une bactérie d'intérêt, en particulier de *S. aureus,* dans un échantillon comprend :
- une étape de marquage des bactéries ou de la bactérie d'intérêt dans l'échantillon ;
- une étape de mise en contact de l'échantillon avec la zone de contact de ladite bandelette ;
- une étape de détection du marqueur sur la zone de détection
- une étape d'analyse de la présence ou de l'absence de la bactérie d'intérêt dans l'échantillon.

L'étape de marquage est réalisée dans l'échantillon, c'est-à-dire préalablement à la mise en contact de l'échantillon avec la zone de contact de la bandelette. Ce mode de réalisation du procédé de détection est applicable que la bandelette de chromatographie à flux latéral utilisée comprenne ou ne comprenne pas une partie de marquage. Ainsi ce mode de réalisation du procédé de détection s'applique à toutes les bandelettes décrites dans la présente description. Lorsque la bandelette utilisée dans ce mode de détection contient une partie de marquage, un double marquage de la bactérie d'intérêt est alors réalisé, le marqueur utilisé dans l'étape de marquage dans l'échantillon et le marqueur compris dans la zone de marquage de la bandelette pouvant être identiques ou différents.

L'étape de marquage est réalisée avec un marqueur non conjugué ou un marqueur conjugué tels que définis précédemment. Le marqueur conjugué peut être conjugué à au moins un ligand spécifique comme défini précédemment. L'Homme du métier sait comment réaliser cette étape de marquage en fonction du marqueur choisi. En particulier, lorsqu'il s'agit d'un marqueur conjugué, une incubation de l'échantillon avec le marqueur conjugué peut être réalisée.

L'Homme du métier sait adapter l'étape de détection en fonction du marqueur utilisé. En particulier, lorsque le marqueur est une nanoparticule d'or, la détection se fait dans le visible. En particulier, lorsque le marqueur utilisé est un marqueur fluorescent, un lecteur de fluorescence est utilisé. En particulier, lorsque le marqueur utilisé est une enzyme, le substrat de la réaction qu'elle catalyse est ajouté. Plus particulièrement, quand il s'agit de la peroxydase de raifort (HRP) qui catalyse la conversion de substrats chromogènes en composés colorés, ou qui produit de la lumière à partir de substrats chimioluminescents, le substrat ajouté est par exemple l'acide 2,2'-azino-bis(3-éthylbenzothiazoline-6-sulphonique) (ABTS), le 1,2-diaminobenzène (OPD), le 3,3'-Diaminobenzidine (DAB), le 3-Amino-9-éthylcarbazole (AEC) ou encore le 3,3',5,5'-tétraméthylbenzidine (TMB) pour les réactions colorées ; l'acide homovanillique et la tyramine pour la spectroscopie de fluorescence ; le luminol pour la production de lumière.

Pour lier un marqueur à un ligand, une liaison biotine/streptavidine peut par exemple être utilisée.

En particulier, le marqueur utilisé est une nanoparticule d'or ou la peroxydase de raifort.

La présence de la bactérie d'intérêt dans l'échantillon analysé est faite conclue lorsque le marqueur est détecté sur la zone de détection. En particulier, lorsque le marqueur est une nanoparticule d'or, un signal coloré visible à l'œil nu apparait. En particulier, lorsque le marqueur utilisé est un marqueur fluorescent (conjugué ou non), un signal fluorescent apparait sur la zone de détection. Lorsque le marqueur utilisé est une enzyme telle que la peroxydase de raifort, un substrat tels que ceux listés ci-dessus, est ajouté et un signal coloré visible à l'œil nu, un signal fluorescent ou un signal luminescent est observé selon le substrat.

L'absence de la bactérie d'intérêt dans l'échantillon est conclue lorsque le marqueur n'est pas détecté sur la zone de détection.

Lorsque la bandelette de chromatographie à flux latéral utilisée pour le procédé de détection comprend une partie de marquage, l'étape de marquage des bactéries ou de la bactérie d'intérêt dans l'échantillon est optionnelle. Ainsi, lorsque la bandelette de chromatographie à flux latéral utilisé pour le procédé de détection comprend une partie de marquage, le procédé de détection d'une bactérie d'intérêt, en particulier de *S. aureus,* dans un échantillon peut comprendre :
- une étape de mise en contact de l'échantillon avec la zone de contact de ladite bandelette ;
- une étape de détection du marqueur sur la zone de détection ;
- une étape d'analyse de la présence ou de l'absence de la bactérie d'intérêt dans l'échantillon.

Dans un autre mode de réalisation particulier, le procédé de détection d'une bactérie d'intérêt, en particulier de *S. aureus,* dans un échantillon comprend :
- une étape de marquage de la bactérie d'intérêt dans l'échantillon avec un marqueur conjugué à au moins un ligand spécifique de la bactérie d'intérêt choisi parmi : au moins un bactériophage spécifique de la bactérie d'intérêt, au moins une protéine d'au moins un bactériophage spécifique de la bactérie d'intérêt, au moins un bactériophage spécifique de la bactérie d'intérêt couplé à un anticorps dirigé contre la bactérie d'intérêt et/ou au moins une protéine d'un bactériophage spécifique de la bactérie d'intérêt couplée à un anticorps dirigé contre la bactérie d'intérêt ;
- une étape de mise en contact de l'échantillon avec la zone de contact d'une bandelette comprenant au moins

une partie de contact comprenant une zone de contact avec l'échantillon ;
une partie de détection comprenant une zone de détection de la bactérie d'intérêt qui comprend au moins un anticorps dirigé contre la bactérie d'intérêt et/ou au moins un aptamère spécifique de la bactérie d'intérêt, en particulier ladite bactérie d'intérêt est *S. aureus,*
une partie absorbante ;
   - une étape de détection du marqueur sur la zone de détection ;
   - une étape d'analyse de la présence ou de l'absence de la bactérie dans l'échantillon.

En d'autres termes, dans ce mode de réalisation particulier, ladite zone de détection de la bandelette comprend au moins un ligand spécifique de la bactérie d'intérêt autre qu'un phage spécifique de la bactérie d'intérêt ou qu'une protéine d'un phage spécifique de la bactérie d'intérêt.

Dans un mode de réalisation particulier, ladite au moins une protéine d'au moins un bactériophage spécifique de la bactérie d'intérêt utilisée à l'étape de marquage, couplée ou pas à un anticorps, est une protéine RBP.

Dans un mode de réalisation particulier, la bactérie d'intérêt est *S. aureus* et lesdits ligands spécifiques conjugués au marqueur, utilisés à l'étape de marquage, se lisent comme des ligands spécifiques de *S. aureus.*

Ainsi, l'étape de marquage peut comprendre un marqueur conjugué à au moins un bactériophage spécifique de *S. aureus.* En particulier, il s'agit d'un marqueur conjugué au phage CNCM I-5408, un marqueur conjugué au phage CNCM I-5409, un marqueur conjugué au phage CNCM I-5410, un marqueur conjugué au phage CNCM I-5491, un marqueur conjugué au phage CNCM I-5492, et/ou un marqueur conjugué au phage CNCM I-5680. Plus particulièrement, il s'agit d'un marqueur conjugué au phage CNCM I-5408, un marqueur conjugué au phage CNCM I-5409 et un marqueur conjugué au phage CNCM I-5410. Plus particulièrement, il s'agit d'un marqueur conjugué au phage CNCM I-5408 et un marqueur conjugué au phage CNCM I-5680. Encore plus particulièrement, ledit marqueur conjugué aux phages est une nanoparticule d'or ou l'HRP.

L'étape de marquage dans l'échantillon peut également comprendre un marqueur conjugué à au moins une protéine d'au moins un bactériophage spécifique de *S. aureus.* En particulier, ladite au moins une protéine est une RBP. En particulier, il s'agit d'un marqueur conjugué à au moins une protéine du phage CNCM I-5408, un marqueur conjugué à au moins une protéine du phage CNCM I-5409, un marqueur conjugué à au moins une protéine du phage CNCM I-5410, un marqueur conjugué à au moins une protéine du phage CNCM I-5491, un marqueur conjugué à au moins une protéine du phage CNCM I-5492, et/ou un marqueur conjugué à au moins une protéine du phage CNCM I-5680. Plus particulièrement, il s'agit d'un marqueur conjugué à au moins une protéine du phage CNCM I-5408, un marqueur conjugué à au moins une protéine du phage CNCM I-5409 et un marqueur conjugué à au moins une protéine du phage CNCM I-5410. Plus particulièrement, il s'agit d'un marqueur conjugué à au moins une protéine du phage CNCM I-5408, un marqueur conjugué à au moins une protéine du phage CNCM I-5410 et/ou et un marqueur conjugué à au moins une protéine du phage CNCM I-5680.
En particulier, ladite au moins une protéine du phage CNCM I-5408 est la protéine SEQ ID NO :1, ladite au moins une protéine du phage CNCM I-5409 est la protéine SEQ ID NO :2, ladite au moins une protéine du phage CNCM I-5410 est la protéine SEQ ID NO :3, ladite au moins une protéine du phage CNCM I-5680 est la protéine SEQ ID NO :4, ladite au moins une protéine du phage CNCM I-5491 est la protéine SEQ ID NO :5 et ladite au moins une protéine du phage CNCM I-5492 est la protéine SEQ ID NO :6. En particulier, la protéine est une protéine ayant au moins 70% d'identité avec la protéine de séquence SEQ ID NO :1, SEQ ID NO :2, SEQ ID NO :3, SEQ ID NO :4, SEQ ID NO :5 ou SEQ ID NO :6, en particulier au moins 80%, plus particulièrement au moins 90%, et encore plus particulièrement au moins 95% d'identité. Typiquement, ledit marqueur conjugué à ces protéines de phages est une nanoparticule d'or ou l'HRP.

En particulier, l'étape de marquage dans l'échantillon peut comprendre un marqueur conjugué au phage CNCM I-5408 couplé à un anticorps anti- *S. aureus,* un marqueur conjugué au phage CNCM I-5409 couplé à un anticorps anti- *S. aureus,* un marqueur conjugué au phage CNCM I-5410 couplé à un anticorps anti- *S. aureus,* un marqueur conjugué au phage CNCM I-5491 couplé à un anticorps anti- *S. aureus,* un marqueur conjugué au phage CNCM I-5492 couplé à un anticorps anti- *S. aureus,* et/ou un marqueur conjugué au phage CNCM I-5680 couplé à un anticorps anti- *S. aureus.*

En particulier, l'étape de marquage dans l'échantillon peut comprendre un marqueur conjugué à une protéine du phage CNCM I-5408 couplé à un anticorps anti- *S. aureus,* un marqueur conjugué à une protéine du phage CNCM I-5409 couplé à un anticorps anti- *S. aureus,* un marqueur conjugué à une protéine du phage CNCM I-5410 couplé à un anticorps anti- *S. aureus,* un marqueur conjugué à une protéine du phage CNCM I-5491 couplé à un anticorps anti- *S. aureus,* un marqueur conjugué à une protéine du phage CNCM I-5492 couplé à un anticorps anti- *S. aureus,* et/ou un marqueur conjugué à une protéine du phage CNCM I-5680 couplé à un anticorps anti- *S. aureus.* En particulier, ladite protéine de phage est une RBP

En particulier, elle peut comprendre un marqueur conjugué à un anticorps anti- *S. aureus* couplé au phage CNCM I-5408, un marqueur conjugué à un anticorps anti- *S. aureus* couplé au phage CNCM I-5409, un marqueur conjugué à un anticorps anti- *S. aureus* couplé au phage CNCM I-5410, un marqueur conjugué à un anticorps anti- *S. aureus* couplé au phage CNCM I-5491, un marqueur conjugué à un anticorps anti- *S. aureus* couplé au phage CNCM I-5492, et/ou un marqueur conjugué à un anticorps anti- *S. aureus* couplé au phage CNCM I-5680.

En particulier, l'étape de marquage dans l'échantillon peut comprendre un marqueur conjugué à un anticorps anti- *S. aureus* couplé à une protéine du phage CNCM I-5408, un marqueur conjugué à un anticorps anti- *S. aureus* couplé à une protéine du phage CNCM I-5409, un marqueur conjugué à un anticorps anti- *S. aureus* couplé à une protéine du phage CNCM I-5410, un marqueur conjugué à un anticorps anti- *S. aureus* couplé à une protéine du phage CNCM I-5491, un marqueur conjugué à un anticorps anti- *S. aureus* couplé à une protéine du phage CNCM I-5492, et/ou un marqueur conjugué à un anticorps anti- *S. aureus* couplé à une protéine du phage CNCM I-5680. En particulier, ladite protéine de phage est une RBP.

En particulier, dans ces exemples de conjugaison du marqueur, ladite au moins une protéine du phage CNCM I-5408 est la protéine SEQ ID NO :1, ladite au moins une protéine du phage CNCM I-5409 est la protéine SEQ ID NO :2, ladite au moins une protéine du phage CNCM I-5410 est la protéine SEQ ID NO :3, ladite au moins une protéine du phage CNCM I-5680 est la protéine SEQ ID NO :4, ladite au moins une protéine du phage CNCM I-5491 est la protéine SEQ ID NO :5 et ladite au moins une protéine du phage CNCM I-5492 est la protéine SEQ ID NO :6. En particulier, la protéine est une protéine ayant au moins 70% d'identité avec la protéine de séquence SEQ ID NO :1, SEQ ID NO :2, SEQ ID NO :3, SEQ ID NO :4, SEQ ID NO :5 ou SEQ ID NO :6, en particulier au moins 80%, plus particulièrement au moins 90%, et encore plus particulièrement au moins 95% d'identité. Typiquement, ledit marqueur conjugué aux protéines de phages est une nanoparticule d'or ou l'HRP.

Optionnellement, les procédés de détection d'une bactérie d'intérêt décrits dans la présente description, comprennent préalablement une étape de mélange d'un surfactant avec l'échantillon. Au sens de la présente divulgation, un surfactant est une molécule amphiphile permettant d'abaisser la tension superficielle d'une solution aqueuse. En particulier, le surfactant est ajouté à l'échantillon à raison de 0,5% à 12% (w/v). Par exemple, le surfactant est le Tween 20.

Un troisième aspect de la présente divulgation porte sur un kit de détection d'une bactérie d'intérêt dans un échantillon en utilisant les bandelettes de chromatographie à flux latéral décrites précédemment.

Dans un mode de réalisation particulier, le kit de détection d'une bactérie d'intérêt, en particulier de *S. aureus,* dans un échantillon comprend :
- une bandelette telle que décrite dans la présente description ;
- un contenant destiné à recevoir l'échantillon ;
- un contenant comprenant un marqueur.

Le marqueur peut être un marqueur non conjugué ou un marqueur conjugué tels que définis précédemment. Le marqueur conjugué peut être conjugué à au moins un ligand spécifique comme défini précédemment. Dans un mode de réalisation particulier, le marqueur peut être compris dans le contenant destiné à recevoir l'échantillon.

Ce mode de réalisation du kit peut comprendre une bandelette de chromatographie à flux latéral avec ou sans partie de marquage. Ainsi ce mode de réalisation du kit de détection s'applique à toutes les bandelettes décrites dans la présente description.

Lorsque la bandelette de chromatographie à flux latéral comprend une partie de marquage, le contenant du kit comprend optionnellement un marqueur. Ainsi, lorsque la bandelette de chromatographie à flux latéral comprend une partie de marquage, le kit de détection d'une bactérie d'intérêt, en particulier de *S. aureus,* dans un échantillon comprend :
- ladite bandelette ;
- un contenant destiné à recevoir l'échantillon.

Dans un autre mode de réalisation particulier, le kit de détection d'une bactérie d'intérêt, en particulier de *S. aureus,* dans un échantillon comprend :
- une bandelette comprenant au moins :
   une partie de contact comprenant une zone de contact avec l'échantillon ;
   une partie de détection comprenant une zone de détection de la bactérie d'intérêt qui comprend au moins un anticorps dirigé contre la bactérie d'intérêt et/ou au moins un aptamère spécifique de la bactérie d'intérêt, en particulier ladite bactérie d'intérêt est *S. aureus,*
   une partie absorbante ;
- un contenant destiné à recevoir l'échantillon
- un contenant comprenant un marqueur conjugué à un ligand spécifique de la bactérie d'intérêt choisi parmi : au moins un bactériophage spécifique de la bactérie d'intérêt, au moins une protéine d'au moins un bactériophage spécifique de la bactérie d'intérêt, au moins un bactériophage spécifique de la bactérie d'intérêt couplé à un anticorps dirigé contre la bactérie d'intérêt, au moins une protéine d'un bactériophage spécifique de la bactérie d'intérêt couplée à un anticorps dirigé contre la bactérie d'intérêt.

En d'autres termes, dans ce mode de réalisation particulier, ladite zone de détection de la bandelette comprend au moins un ligand spécifique de la bactérie d'intérêt autre qu'un phage spécifique de la bactérie d'intérêt ou d'une protéine d'un phage spécifique de la bactérie d'intérêt.

Dans un mode de réalisation particulier, ladite au moins une protéine d'au moins un bactériophage spécifique de la bactérie d'intérêt conjuguée au marqueur, couplée ou pas à un anticorps, est une protéine RBP.

Dans un mode de réalisation particulier, le marqueur conjugué à un ligand spécifique de la bactérie d'intérêt peut être compris dans le contenant destiné à recevoir l'échantillon.

Dans un mode de réalisation particulier, il s'agit d'un kit de détection de la bactérie d'intérêt *S. aureus* et lesdits ligands spécifiques conjugués au marqueur se lisent comme des ligands spécifiques de *S. aureus.*

Dans un mode de réalisation particulier, la bactérie d'intérêt est *S. aureus* et ces ligands spécifiques utilisés à l'étape de marquage, en conjugaison avec un marqueur, se lisent comme des ligands spécifiques de *S. aureus.*

En particulier, ce mode de réalisation du kit est prévu pour détecter *S. aureus* dans un échantillon. Ainsi, le contenant peut comprendre un marqueur conjugué à au moins un bactériophage spécifique de *S. aureus.* En particulier, il s'agit d'un marqueur conjugué au phage CNCM I-5408, un marqueur conjugué au phage CNCM I-5409, un marqueur conjugué au phage CNCM I-5410, un marqueur conjugué au phage CNCM I-5491, un marqueur conjugué au phage CNCM I-5492, et/ou un marqueur conjugué au phage CNCM I-5680. Plus particulièrement, il s'agit d'un marqueur conjugué au phage CNCM I-5408, un marqueur conjugué au phage CNCM I-5409 et un marqueur conjugué au phage CNCM I-5410. Plus particulièrement, il s'agit d'un marqueur conjugué au phage CNCM I-5408 et un marqueur conjugué au phage CNCM I-5680. Encore plus particulièrement, ledit marqueur conjugué à ces phages est une nanoparticule d'or.

Le contenant peut également comprendre un marqueur conjugué à au moins une protéine d'au moins un bactériophage spécifique de *S. aureus.* En particulier, ladite au moins une protéine est une RBP. En particulier, il s'agit d'un marqueur conjugué à au moins une protéine du phage CNCM I-5408, un marqueur conjugué à au moins une protéine du phage CNCM I-5409, un marqueur conjugué à au moins une protéine du phage CNCM I-5410, un marqueur conjugué à au moins une protéine du phage CNCM I-5491, un marqueur conjugué à au moins une protéine du phage CNCM I-5492, et/ou un marqueur conjugué à au moins une protéine du phage CNCM I-5680. Plus particulièrement, il s'agit d'un marqueur conjugué à au moins une protéine du phage CNCM I-5408, un marqueur conjugué à au moins une protéine du phage CNCM I-5409 et un marqueur conjugué à au moins une protéine du phage CNCM I-5410. Plus particulièrement, il s'agit d'un marqueur conjugué à au moins une protéine du phage CNCM I-5408, un marqueur conjugué à au moins une protéine du phage CNCM I-5410 et/ou un marqueur conjugué à au moins une protéine du phage CNCM I-5680.
En particulier, ladite au moins une protéine du phage CNCM I-5408 est la protéine SEQ ID NO :1, ladite au moins une protéine du phage CNCM I-5409 est la protéine SEQ ID NO :2, ladite au moins une protéine du phage CNCM I-5410 est la protéine SEQ ID NO :3, ladite au moins une protéine du phage CNCM I-5680 est la protéine SEQ ID NO :4, ladite au moins une protéine du phage CNCM I-5491 est la protéine SEQ ID NO :5 et ladite au moins une protéine du phage CNCM I-5492 est la protéine SEQ ID NO :6. En particulier, la protéine est une protéine ayant au moins 70% d'identité avec la protéine de séquence SEQ ID NO :1, SEQ ID NO :2, SEQ ID NO :3, SEQ ID NO :4, SEQ ID NO :5 ou SEQ ID NO :6, en particulier au moins 80%, plus particulièrement au moins 90%, et encore plus particulièrement au moins 95% d'identité. Typiquement, ledit marqueur conjugué à ces protéines de phages est une nanoparticule d'or ou l'HRP.

En particulier, le contenant peut comprendre un marqueur conjugué au phage CNCM I-5408 couplé à un anticorps anti- *S. aureus,* un marqueur conjugué au phage CNCM I-5409 couplé à un anticorps anti- *S. aureus,* un marqueur conjugué au phage CNCM I-5410 couplé à un anticorps anti- *S. aureus,* un marqueur conjugué au phage CNCM I-5491 couplé à un anticorps anti- *S. aureus,* un marqueur conjugué au phage CNCM I-5492 couplé à un anticorps anti- *S. aureus,* et/ou un marqueur conjugué au phage CNCM I-5680 couplé à un anticorps anti- *S. aureus.*

En particulier, le contenant peut comprendre un marqueur conjugué à une protéine du phage CNCM I-5408 couplé à un anticorps anti- *S. aureus,* un marqueur conjugué à une protéine du phage CNCM I-5409 couplé à un anticorps anti- *S. aureus,* un marqueur conjugué à une protéine du phage CNCM I-5410 couplé à un anticorps anti- *S. aureus,* un marqueur conjugué à une protéine du phage CNCM I-5491 couplé à un anticorps anti- *S. aureus,* un marqueur conjugué à une protéine du phage CNCM I-5492 couplé à un anticorps anti- *S. aureus,* et/ou un marqueur conjugué à une protéine du phage CNCM I-5680 couplé à un anticorps anti- *S. aureus.*

En particulier, le contenant peut comprendre un marqueur conjugué à un anticorps anti-*S. aureus* couplé au phage CNCM I-5408, un marqueur conjugué à un anticorps anti- *S. aureus* couplé au phage CNCM I-5409, un marqueur conjugué à un anticorps anti- *S. aureus* couplé au phage CNCM I-5410, un marqueur conjugué à un anticorps anti- *S. aureus* couplé au phage CNCM I-5491, un marqueur conjugué à un anticorps anti- *S. aureus* couplé au phage CNCM I-5492, et/ou un marqueur conjugué à un anticorps anti- *S. aureus* couplé au phage CNCM I-5680.

En particulier, le contenant peut comprendre un marqueur conjugué à un anticorps anti- *S. aureus* couplé à une protéine du phage CNCM I-5408, un marqueur conjugué à un anticorps anti- *S. aureus* couplé à une protéine du phage CNCM I-5409, un marqueur conjugué à un anticorps anti- *S. aureus* couplé à une protéine du phage CNCM I-5410, un marqueur conjugué à un anticorps anti- *S. aureus* couplé à une protéine du phage CNCM I-5491, un marqueur conjugué à un anticorps anti- *S. aureus* couplé à une protéine du phage CNCM I-5492, et/ou un marqueur conjugué à un anticorps anti- *S. aureus* couplé à une protéine du phage CNCM I-5680. En particulier, ladite protéine de phage est une RBP.

En particulier, dans ces exemples de conjugaison du marqueur, ladite au moins une protéine du phage CNCM I-5408 est la protéine SEQ ID NO :1, ladite au moins une protéine du phage CNCM I-5409 est la protéine SEQ ID NO :2, ladite au moins une protéine du phage CNCM I-5410 est la protéine SEQ ID NO :3, ladite au moins une protéine du phage CNCM I-5680 est la protéine SEQ ID NO :4, ladite au moins une protéine du phage CNCM I-5491 est la protéine SEQ ID NO :5 et ladite au moins une protéine du phage CNCM I-5492 est la protéine SEQ ID NO :6. En particulier, la protéine est une protéine ayant au moins 70% d'identité avec la protéine de séquence SEQ ID NO :1, SEQ ID NO :2, SEQ ID NO :3, SEQ ID NO :4, SEQ ID NO :5 ou SEQ ID NO :6, en particulier au moins 80%, plus particulièrement au moins 90%, et encore plus particulièrement au moins 95% d'identité. Encore plus particulièrement, ledit marqueur conjugué aux protéines de phages est une nanoparticule d'or ou l'HRP.

Pour les kits décrits dans la présente description, l'Homme du métier sait sélectionner un contenant aux proportions adéquates pour recevoir l'échantillon et/ou pour comprendre le volume nécessaire de marqueur. En particulier, si le contenant est destiné à contenir la bandelette afin de mettre œuvre le procédé de détection, l'Homme du métier sait choisir un contenant avec les proportions adéquates pour contenir la bandelette, l'échantillon et optionnellement le marqueur, et il sait adapter le volume d'échantillon et le volume de marqueur (si nécessaire), de façon à ce qu'uniquement l'échantillon ou le mélange échantillon et marqueur, soit en contact avec la zone de contact de la bandelette lors de la mise en œuvre du procédé de détection. Par exemple, le contenant comprendra une graduation indiquant le volume maximal d'échantillon à ajouter.

Optionnellement, les kits décrits dans la présente description comprennent un contenant comprenant un surfactant. En particulier, il comprend un volume de surfactant représentant de 0,5 à 12% (w/v) du volume d'échantillon avec lequel le surfactant sera mélangé. Dans un mode de réalisation particulier, le surfactant peut être compris dans le contenant destiné à recevoir l'échantillon, ou dans un contenant destiné à recevoir l'échantillon et comprenant un marqueur.

Selon un quatrième aspect, la présente divulgation concerne l'utilisation d'au moins un bactériophage spécifique d'une bactérie d'intérêt et/ou d'au moins une protéine d'au moins un bactériophage spécifique d'une bactérie d'intérêt pour détecter ladite bactérie d'intérêt dans un échantillon, caractérisée en ce que ledit au moins un bactériophage spécifique de la bactérie d'intérêt et/ou ladite au moins une protéine d'au moins un bactériophage spécifique de la bactérie d'intérêt sont utilisés dans un dispositif de chromatographie à flux latéral. En particulier, un dispositif de chromatographie à flux latéral est une bandelette de chromatographie à flux latéral. Plus particulièrement, il s'agit d'une bandelette telle que décrite dans la présente description.

Dans un mode d'utilisation particulier, la bactérie d'intérêt est *Staphylococcus aureus.* Ainsi, ledit au moins un bactériophage est spécifique de *S. aureus.* Dans un mode plus particulier, ledit au moins un bactériophage spécifique de *S. aureus* est choisi parmi les bactériophages spécifiques de *S. aureus* déposés à la Collection Nationale de Cultures de Microorganismes (CNCM) sous les numéros CNCM I-5408, CNCM I-5409, CNCM I-5410, CNCM I-5491, CNCM I-5492 et CNCM I-5680.En particulier, il peut s'agir de l'utilisation de deux bactériophages spécifiques de *S. aureus* choisi parmi les bactériophages déposés sous les numéros CNCM I-5408, CNCM I-5409, CNCM I-5410, CNCM I-5491, CNCM I-5492 et CNCM I-5680.

Par exemple il s'agit de l'utilisation des couples suivants : le bactériophage CNCM I-5408 et le bactériophage CNCM I-5409, le bactériophage CNCM I-5409 et le bactériophage CNCM I-5410, le bactériophage CNCM I-5408 et le bactériophage CNCM I-5410, le bactériophage CNCM I-5408 et le bactériophage CNCM I-5491, le bactériophage CNCM I-5409 et le bactériophage CNCM I-5491, le bactériophage CNCM I-5410 et le bactériophage CNCM I-5491, le bactériophage CNCM I-5408 et le bactériophage CNCM I-5492, le bactériophage CNCM I-5409 et le bactériophage CNCM I-5492, le bactériophage CNCM I-5410 et le bactériophage CNCM I-5492, le bactériophage CNCM I-5491 et le bactériophage CNCM I-5492, le bactériophage CNCM I-5680 et le bactériophage CNCM I-5408, le bactériophage CNCM I-5680 et le bactériophage CNCM I-5409, le bactériophage CNCM I-5680 et le bactériophage CNCM I-5492, le bactériophage CNCM I-5680 et le bactériophage CNCM I-5410, le bactériophage CNCM I-5680 et le bactériophage CNCM I-5491, le bactériophage CNCM I-5680 et le bactériophage CNCM I-5492.

En particulier, il peut s'agir de l'utilisation trois bactériophages spécifiques de *S. aureus* choisis parmi les bactériophages déposés sous les numéros CNCM I-5408, CNCM I-5409, CNCM I-5410, CNCM I-5491, CNCM I-5492 et CNCM I-5680.

Par exemple, il peut s'agir de l'utilisation de trois bactériophages spécifiques de *S. aureus* tels que les bactériophages déposés sous les numéros CNCM I-5408, CNCM I-5409 et CNCM I-5410, les bactériophages déposés sous les numéros CNCM I-5408, CNCM I-5491 et CNCM I-5492, les bactériophages déposés sous les numéros CNCM I-5409, CNCM I-5491 et CNCM I-5492, les bactériophages déposés sous les numéros CNCM I-5410, CNCM I-5491 et CNCM I-5492, les bactériophages déposés sous les numéros CNCM I-5408, CNCM I-5409 et CNCM I-5491, les bactériophages déposés sous les numéros CNCM I-5408, CNCM I-5410 et CNCM I-5491, les bactériophages déposés sous les numéros CNCM I-5408, CNCM I-5409 et CNCM I-5492, les bactériophages déposés sous les numéros CNCM I-5408, CNCM I-5410 et CNCM I-5492, les bactériophages déposés sous les numéros CNCM I-5409, CNCM I-5410 et CNCM I-5491, les bactériophages déposés sous les numéros CNCM I-5409, CNCM I-5410 et CNCM I-5492, les bactériophages déposés sous les numéros CNCM I-5409, CNCM I-5491 et CNCM I-5680, les bactériophages déposés sous les numéros CNCM I-5410, CNCM I-5491 et CNCM I-5680, les bactériophages déposés sous les numéros CNCM I-5408, CNCM I-5409 et CNCM I-5680, les bactériophages déposés sous les numéros CNCM I-5408, CNCM I-5410 et CNCM I-5680, les bactériophages déposés sous les numéros CNCM I-5408, CNCM I-5680 et CNCM I-5492, les bactériophages déposés sous les numéros CNCM I-5680, CNCM I-5410 et CNCM I-5492, les bactériophages déposés sous les numéros CNCM I-5409, CNCM I-5410 et CNCM I-5680, les bactériophages déposés sous les numéros CNCM I-5409, CNCM I-5680 et CNCM I-5492, les bactériophages déposés sous les numéros CNCM I-5491, CNCM I-5492 et CNCM I-5680, les bactériophages déposés sous les numéros CNCM I-5408, CNCM I-5491 et CNCM I-5680.

En particulier, il peut s'agir de l'utilisation de quatre bactériophages spécifiques de *S. aureus* choisis parmi les bactériophages déposés sous les numéros CNCM I-5408, CNCM I-5409, CNCM I-5410, CNCM I-5491, CNCM I-5492 et CNCM I-5680.

Par exemple, il peut s'agir de l'utilisation de quatre bactériophages spécifiques de *S. aureus* tels que les bactériophages déposés sous les numéros CNCM I-5408, CNCM I-5409, CNCM I-5410 et CNCM I-5491, les bactériophages déposés sous les numéros CNCM I-5408, CNCM I-5409, CNCM I-5410 et CNCM I-5492, les bactériophages déposés sous les numéros CNCM I-5408, CNCM I-5409, CNCM I-5491 et CNCM I-5492, les bactériophages déposés sous les numéros CNCM I-5408, CNCM I-5410, CNCM I-5491 et CNCM I-5492, les bactériophages déposés sous les numéros CNCM I-5410, CNCM I-5409, CNCM I-5491 et CNCM I-5492, les bactériophages déposés sous les numéros CNCM I-5680, CNCM I-5409, CNCM I-5410 et CNCM I-5491, les bactériophages déposés sous les numéros CNCM I-5680, CNCM I-5409, CNCM I-5410 et CNCM I-5492, les bactériophages déposés sous les numéros CNCM I-5680, CNCM I-5409, CNCM I-5491 et CNCM I-5492, les bactériophages déposés sous les numéros CNCM I-5680, CNCM I-5410, CNCM I-5491 et CNCM I-5492, les bactériophages déposés sous les numéros CNCM I-5408, CNCM I-5409, CNCM I-5410 et CNCM I-5680, les bactériophages déposés sous les numéros CNCM I-5408, CNCM I-5409, CNCM I-5491 et CNCM I-5680, les bactériophages déposés sous les numéros CNCM I-5408, CNCM I-5409, CNCM I-5492 et CNCM I-5680.

En particulier, il peut s'agir de l'utilisation de cinq bactériophages spécifiques de S. aureus choisis parmi les bactériophages déposés sous les numéros CNCM I-5408, CNCM I-5409, CNCM I-5410, CNCM I-5491, CNCM I-5492 et CNCM I-5680.

En particulier, il peut s'agir de l'utilisation des six bactériophages spécifiques de *S. aureus* tels que déposés sous les numéros CNCM I-5408, CNCM I-5409, CNCM I-5410, CNCM I-5491, CNCM I-5492 et CNCM I-5680.

Dans le cas de l'utilisation d'au moins une protéine d'au moins un bactériophage spécifique d'une bactérie d'intérêt, ledit au moins un bactériophage spécifique d'une bactérie d'intérêt est tel que défini ci-dessus. Ainsi, dans un mode d'utilisation particulier, ladite au moins une protéine d'au moins un bactériophage spécifique d'une bactérie d'intérêt est au moins une protéine d'au moins un bactériophage spécifique de *S. aureus.*

En particulier, il s'agit d'une protéine de liaison (RBP).

En particulier, ladite au moins une protéine d'au moins un bactériophage spécifique de *S. aureus* est au moins une protéine du bactériophage CNCM I-5408, au moins une protéine du phage CNCM I-5409, au moins une protéine du bactériophage CNCM I-5410, au moins une protéine du phage CNCM I-5491, au moins une protéine du bactériophage CNCM I-5492 et /ou au moins une protéine du phage CNCM I-5680.

En particulier, il peut s'agir de l'utilisation de deux protéines, en particulier des RBP, provenant d'un même bactériophage spécifique de *S. aureus,* ledit bactériophage spécifique de *S. aureus* étant choisi parmi les bactériophages déposés sous les numéros CNCM I-5408, CNCM I-5409, CNCM I-5410, CNCM I-5491, CNCM I-5492 et CNCM I-5680.

En particulier, il peut s'agir de l'utilisation de deux protéines, en particulier des RBP, provenant de bactériophages spécifiques de *S. aureus* différents, lesdits bactériophages spécifiques de *S. aureus* étant choisis parmi les bactériophages déposés sous les numéros CNCM I-5408, CNCM I-5409, CNCM I-5410, CNCM I-5491, CNCM I-5492 et CNCM I-5680.

Par exemple, il peut s'agir de l'utilisation d'au moins une protéine du bactériophage CNCM I-5408 et d'au moins une protéine du bactériophage CNCM I-5409, d'au moins une protéine du bactériophage CNCM I-5409 et d'au moins une protéine du bactériophage CNCM I-5410, d'au moins une protéine du bactériophage CNCM I-5408 et d'au moins une protéine du bactériophage CNCM I-5410, d'au moins une protéine du bactériophage CNCM I-5408 et d'au moins une protéine du bactériophage CNCM I-5491, d'au moins une protéine du bactériophage CNCM I-5409 et d'au moins une protéine du bactériophage CNCM I-5491, d'au moins une protéine du bactériophage CNCM I-5410 et d'au moins une protéine du bactériophage CNCM I-5491, d'au moins une protéine du bactériophage CNCM I-5408 et d'au moins une protéine du bactériophage CNCM I-5492, d'au moins une protéine du bactériophage CNCM I-5409 et d'au moins une protéine du bactériophage CNCM I-5492, d'au moins une protéine du bactériophage CNCM I-5410 et d'au moins une protéine du bactériophage CNCM I-5492, d'au moins une protéine du bactériophage CNCM I-5491 et d'au moins une protéine du bactériophage CNCM I-5492, d'au moins une protéine du bactériophage CNCM I-5680 et d'au moins une protéine du bactériophage CNCM I-5408, d'au moins une protéine du bactériophage CNCM I-5680 et d'au moins une protéine du bactériophage CNCM I-5409, d'au moins une protéine du bactériophage CNCM I-5680 et d'au moins une protéine du bactériophage CNCM I-5410, d'au moins une protéine du bactériophage CNCM I-5680 et d'au moins une protéine du bactériophage CNCM I-5491, d'au moins une protéine du bactériophage CNCM I-5680 et d'au moins une protéine du bactériophage CNCM I-5492.

En particulier, il peut s'agir de l'utilisation de trois protéines, en particulier des RBP, provenant d'au moins un bactériophage spécifique de *S. aureus,* lesdits bactériophages spécifiques de *S. aureus* étant choisis parmi les bactériophages déposés sous les numéros CNCM I-5408, CNCM I-5409, CNCM I-5410, CNCM I-5491, CNCM I-5492 et CNCM I-5680.

En particulier, il peut s'agir de l'utilisation de trois protéines, en particulier des RBP, provenant de bactériophages spécifiques de *S. aureus* différents, lesdits bactériophages spécifiques de S. aureus étant choisis parmi les bactériophages déposés sous les numéros CNCM I-5408, CNCM I-5409, CNCM I-5410, CNCM I-5491, CNCM I-5492 et CNCM I-5680.

Par exemple, il peut s'agir de l'utilisation d'au moins une protéine du bactériophage CNCM I-5408, d'au moins une protéine du bactériophage CNCM I-5409 et d'au moins une protéine du bactériophage CNCM I-5410 ; d'au moins une protéine du bactériophage CNCM I-5408, d'au moins une protéine du bactériophage CNCM I-5491 et d'au moins une protéine du bactériophage CNCM I-5492 ; d'au moins une protéine du bactériophage CNCM I-5409, d'au moins une protéine du bactériophage CNCM I-5491 et d'au moins une protéine du bactériophage CNCM I-5492 ; d'au moins une protéine du bactériophage CNCM I-5410, d'au moins une protéine du bactériophage CNCM I-5491 et d'au moins une protéine du bactériophage CNCM I-5492 ; d'au moins une protéine du bactériophage CNCM I-5408, d'au moins une protéine du bactériophage CNCM I-5409 et d'au moins une protéine du bactériophage CNCM I-5491 ; d'au moins une protéine du bactériophage CNCM I-5408, d'au moins une protéine du bactériophage CNCM I-5410 et d'au moins une protéine du bactériophage CNCM I-5491 ; d'au moins une protéine du bactériophage CNCM I-5408, d'au moins une protéine du bactériophage CNCM I-5409 et d'au moins une protéine du bactériophage CNCM I-5492 ; d'au moins une protéine du bactériophage CNCM I-5408, d'au moins une protéine du bactériophage CNCM I-5410 et d'au moins une protéine du bactériophage CNCM I-5492 ; d'au moins une protéine du bactériophage CNCM I-5409, d'au moins une protéine du bactériophage CNCM I-5410 et d'au moins une protéine du bactériophage CNCM I-5491 ; d'au moins une protéine du bactériophage CNCM I-5409, d'au moins une protéine du bactériophage CNCM I-5410 et d'au moins une protéine du bactériophage CNCM I-5492, d'au moins une protéine du bactériophage CNCM I-5409, d'au moins une protéine du bactériophage CNCM I-5491 et d'au moins une protéine du bactériophage CNCM I-5680 ; d'au moins une protéine du bactériophage CNCM I-5410, d'au moins une protéine du bactériophage CNCM I-5491 et d'au moins une protéine du bactériophage CNCM I-5680 ; d'au moins une protéine du bactériophage CNCM I-5408, d'au moins une protéine du bactériophage CNCM I-5409 et d'au moins une protéine du bactériophage CNCM I-5680 ; d'au moins une protéine du bactériophage CNCM I-5408, d'au moins une protéine du bactériophage CNCM I-5410 et d'au moins une protéine du bactériophage CNCM I-5680 ; d'au moins une protéine du bactériophage CNCM I-5408, d'au moins une protéine du bactériophage CNCM I-5680 et d'au moins une protéine du bactériophage CNCM I-5492 ; d'au moins une protéine du bactériophage CNCM I-5680, d'au moins une protéine du bactériophage CNCM I-5410 et d'au moins une protéine du bactériophage CNCM I-5492 ; d'au moins une protéine du bactériophage CNCM I-5409, d'au moins une protéine du bactériophage CNCM I-5410 et d'au moins une protéine du bactériophage CNCM I-5680 ; d'au moins une protéine du bactériophage CNCM I-5409, d'au moins une protéine du bactériophage CNCM I-5680 et d'au moins une protéine du bactériophage CNCM I-5492 ; d'au moins une protéine du bactériophage CNCM I-5491, d'au moins une protéine du bactériophage CNCM I-5492 et d'au moins une protéine du bactériophage CNCM I-5680 ; d'au moins une protéine du bactériophage CNCM I-5408, d'au moins une protéine du bactériophage CNCM I-5491 et d'au moins une protéine du bactériophage CNCM I-5680.

En particulier, il peut s'agir de l'utilisation de quatre protéines, en particulier des RBP, provenant d'au moins un bactériophage spécifique de *S*. *aureus,* lesdits bactériophages spécifiques de *S*. *aureus* étant choisis parmi les bactériophages déposés sous les numéros CNCM I-5408, CNCM I-5409, CNCM I-5410, CNCM I-5491, CNCM I-5492 et CNCM I-5680.

En particulier, il peut s'agir de l'utilisation de quatre protéines, en particulier des RBP, provenant de bactériophages spécifiques de *S*. *aureus* différents, lesdits bactériophages spécifiques de *S*. *aureus* étant choisis parmi les bactériophages déposés sous les numéros CNCM I-5408, CNCM I-5409, CNCM I-5410, CNCM I-5491, CNCM I-5492 et CNCM I-5680.

Par exemple, il s'agit de l'utilisation d'au moins une protéine du bactériophage CNCM I-5408, d'au moins une protéine du bactériophage CNCM I-5409, d'au moins une protéine du bactériophage CNCM I-5410 et d'au moins une protéine du bactériophage CNCM I-5491 ; d'au moins une protéine du bactériophage CNCM I-5408, d'au moins une protéine du bactériophage CNCM I-5409, d'au moins une protéine du bactériophage CNCM I-5410 et d'au moins une protéine du bactériophage CNCM I-5492 ; d'au moins une protéine du bactériophage CNCM I-5408, d'au moins une protéine du bactériophage CNCM I-5409, d'au moins une protéine du bactériophage CNCM I-5491 et d'au moins une protéine du bactériophage CNCM I-5492 ; d'au moins une protéine du bactériophage CNCM I-5408, d'au moins une protéine du bactériophage CNCM I-5410, d'au moins une protéine du bactériophage CNCM I-5491 et d'au moins une protéine du bactériophage CNCM I-5492 ; d'au moins une protéine du bactériophage CNCM I-5410, d'au moins une protéine du bactériophage CNCM I-5409, d'au moins une protéine du bactériophage CNCM I-5491 et d'au moins une protéine du bactériophage CNCM I-5492, d'au moins une protéine du bactériophage CNCM I-5680, d'au moins une protéine du bactériophage CNCM I-5409, d'au moins une protéine du bactériophage CNCM I-5410 et d'au moins une protéine du bactériophage CNCM I-5491, d'au moins une protéine du bactériophage CNCM I-5680, d'au moins une protéine du bactériophage CNCM I-5409, d'au moins une protéine du bactériophage CNCM I-5410 et d'au moins une protéine du bactériophage CNCM I-5492, d'au moins une protéine du bactériophage CNCM I-5680, d'au moins une protéine du bactériophage CNCM I-5409, d'au moins une protéine du bactériophage CNCM I-5491 et d'au moins une protéine du bactériophage CNCM I-5492, d'au moins une protéine du bactériophage CNCM I-5680, d'au moins une protéine du bactériophage CNCM I-5410, d'au moins une protéine du bactériophage CNCM I-5491 et d'au moins une protéine du bactériophage CNCM I-5492, d'au moins une protéine du bactériophage CNCM I-5408, d'au moins une protéine du bactériophage CNCM I-5409, d'au moins une protéine du bactériophage CNCM I-5410 et d'au moins une protéine du bactériophage CNCM I-5680, d'au moins une protéine du bactériophage CNCM I-5408, d'au moins une protéine du bactériophage CNCM I-5409, d'au moins une protéine du bactériophage CNCM I-5491 et d'au moins une protéine du bactériophage CNCM I-5680, d'au moins une protéine du bactériophage CNCM I-5408, d'au moins une protéine du bactériophage CNCM I-5409, d'au moins une protéine du bactériophage CNCM I-5492 et d'au moins une protéine du bactériophage CNCM I-5680.

En particulier, il peut s'agir de l'utilisation de cinq protéines, en particulier des RBP, provenant d'au moins un bactériophage spécifique de S. *aureus,* lesdits bactériophages spécifiques de S. *aureus* étant choisis parmi les bactériophages déposés sous les numéros CNCM I-5408, CNCM I-5409, CNCM I-5410, CNCM I-5491, CNCM I-5492 et CNCM I-5680.

En particulier, il peut s'agir de l'utilisation de cinq protéines, en particulier des RBP, provenant de bactériophages spécifiques de *S*. *aureus* différents, lesdits bactériophages spécifiques de *S*. *aureus* étant choisis parmi les bactériophages déposés sous les numéros CNCM I-5408, CNCM I-5409, CNCM I-5410, CNCM I-5491, CNCM I-5492 et CNCM I-5680.

Par exemple, il s'agit de l'utilisation d'au moins une protéine du bactériophage CNCM I-5408, d'au moins une protéine du bactériophage CNCM I-5409, d'au moins une protéine du bactériophage CNCM I-5410, d'au moins une protéine du bactériophage CNCM I-5491 et d'au moins une protéine du bactériophage CNCM I-5492.

En particulier, il peut s'agir de l'utilisation de six protéines provenant d'au moins un bactériophage spécifique de *S*. *aureus,* lesdits bactériophages spécifiques de *S*. *aureus* étant choisis parmi les bactériophages déposés sous les numéros CNCM I-5408, CNCM I-5409, CNCM I-5410, CNCM I-5491, CNCM I-5492 et CNCM I-5680.

En particulier, il peut s'agir de six protéines, en particulier des RBP, provenant de bactériophages spécifiques de *S*. *aureus* différents, lesdits bactériophages spécifiques de *S*. *aureus* étant les bactériophages déposés sous les numéros CNCM I-5408, CNCM I-5409, CNCM I-5410, CNCM I-5491, CNCM I-5492 et CNCM I-5680.

Dans un mode d'utilisation particulier, ladite au moins une protéine d'au moins un bactériophage spécifique d'une bactérie d'intérêt ou ladite au moins une protéine d'au moins un bactériophage spécifique de *S*. *aureus* est donc une protéine de liaison (RBP, receptor binding protein). Dans un mode d'utilisation plus particulier, il s'agit de l'utilisation d'au moins une protéine RBP du bactériophage CNCM I-5408, d'au moins une RBP du bactériophage CNCM I-5409, d'au moins une RBP du bactériophage CNCM I-5410, d'au moins une RBP du bactériophage CNCM I-5491, d'au moins une RBP du bactériophage CNCM I-5492, et/ou d'au moins une RBP du bactériophage CNCM I-5680.

Encore plus particulièrement, ladite au moins une protéine RBP du bactériophage CNCM I-5408 est la protéine SEQ ID NO :1 ; ladite au moins une protéine RBP du bactériophage CNCM I-5409 est la protéine SEQ ID NO :2 ; ladite au moins une protéine RBP du bactériophage CNCM I-5410 est la protéine SEQ ID NO :3, ladite au moins une protéine RBP du bactériophage CNCM I-5680 est la protéine SEQ ID NO :4, ladite au moins une protéine RBP du bactériophage CNCM I-5491 est la protéine SEQ ID NO :5, ladite au moins une protéine RBP du bactériophage CNCM I-5492 est la protéine SEQ ID NO :6.

En particulier, il s'agit de l'utilisation d'une protéine ayant au moins 70% d'identité avec la protéine de séquence SEQ ID NO :1, SEQ ID NO :2, SEQ ID NO :3, SEQ ID NO :4 SEQ ID NO :5 ou SEQ ID NO :6, en particulier au moins 80%, plus particulièrement au moins 90%, et encore plus particulièrement au moins 95% d'identité.

Ainsi, la présente invention porte sur l'utilisation d'au moins une protéine d'au moins un bactériophage spécifique de *S*. *aureus,* ladite protéine étant une protéine de séquence SEQ ID NO :1, une protéine de séquence SEQ ID NO :2, une protéine de séquence SEQ ID NO :3, une protéine de séquence SEQ ID NO :4, une protéine de séquence SEQ ID NO :5, une protéine de séquence SEQ ID NO :6, ou une protéine ayant au moins 70% d'identité avec l'une des protéines de séquence SEQ ID NOs :1- 6, pour détecter *S*. *aureus* dans un échantillon, caractérisée en ce que ladite au moins une protéine d'un bactériophage spécifique de *S*. *aureus* est utilisée dans une bandelette de chromatographie à flux latéral.

Dans un mode de réalisation particulier de l'invention, ladite au moins une protéine d'au moins un bactériophage spécifique de S. *aureus* est une protéine de liaison au récepteur de phage (RBP).

Selon un cinquième aspect, la présente divulgation concerne la protéine de séquence SEQ ID NO :1, ainsi que les protéines ayant au moins 70% d'identité avec la protéine de séquence SEQ ID NO :1, en particulier au moins 80%, plus particulièrement au moins 90%, et encore plus particulièrement au moins 95% d'identité. La protéine de séquence SEQ ID NO :1 est une protéine RBP isolée du bactériophage CNCM I-5408.

La présente divulgation concerne également la protéine de séquence SEQ ID NO :2, ainsi que les protéines ayant au moins 70% d'identité avec la protéine de séquence SEQ ID NO :2, en particulier au moins 80%, plus particulièrement au moins 90%, et encore plus particulièrement au moins 95% d'identité. La protéine de séquence SEQ ID NO :2 est une protéine RBP isolée du bactériophage CNCM I-5409.

La présente divulgation concerne également la protéine de séquence SEQ ID NO :3, ainsi que les protéines ayant au moins 70% d'identité avec la protéine de séquence SEQ ID NO :3, en particulier au moins 80%, plus particulièrement au moins 90%, et encore plus particulièrement au moins 95% d'identité. La protéine de séquence SEQ ID NO :3 est une protéine RBP isolée du bactériophage CNCM I-5410.

La présente divulgation concerne également la protéine de séquence SEQ ID NO :4, ainsi que les protéines ayant au moins 70% d'identité avec la protéine de séquence SEQ ID NO :4, en particulier au moins 80%, plus particulièrement au moins 90%, et encore plus particulièrement au moins 95% d'identité. La protéine de séquence SEQ ID NO :4 est une protéine RBP isolée du bactériophage CNCM I-5680.

La présente divulgation concerne également la protéine de séquence SEQ ID NO :5, ainsi que les protéines ayant au moins 70% d'identité avec la protéine de séquence SEQ ID NO :5, en particulier au moins 80%, plus particulièrement au moins 90%, et encore plus particulièrement au moins 95% d'identité. La protéine de séquence SEQ ID NO :5 est une protéine RBP isolée du bactériophage CNCM I-5491.

La présente divulgation concerne également la protéine de séquence SEQ ID NO :6, ainsi que les protéines ayant au moins 70% d'identité avec la protéine de séquence SEQ ID NO :6, en particulier au moins 80%, plus particulièrement au moins 90%, et encore plus particulièrement au moins 95% d'identité. La protéine de séquence SEQ ID NO :6 est une protéine RBP isolée du bactériophage CNCM I-5492.

La présente divulgation concerne également l'utilisation dans un dispositif de détection de *S. aureus* du bactériophage CNCM I-5680, de la protéine de séquence SEQ ID NO :1, la protéine de séquence SEQ ID NO :2, de la protéine de séquence SEQ ID NO :3, de la protéine de séquence SEQ ID NO :4, de la protéine de séquence SEQ ID NO :5 et/ou de la protéine de séquence SEQ ID NO :6, ainsi qu'une protéine ayant au moins 70% d'identité avec la protéine de séquence SEQ ID NO :1, SEQ ID NO :2, SEQ ID NO :3, SEQ ID NO :4, SEQ ID NO :5 ou SEQ ID NO :6, en particulier au moins 80%, plus particulièrement au moins 90%, et encore plus particulièrement au moins 95% d'identité.

Un tel dispositif de détection peut être un dispositif LFA, un dispositif ELISA (enzyme-linked immunosorbent assay), une plaque multi-puits, un filtre, des billes, une bandelette, un film conçu pour la cuture bactérienne.

Le dispositif LFA peut être un dispositif LFA tel que décrit dans la présente description.

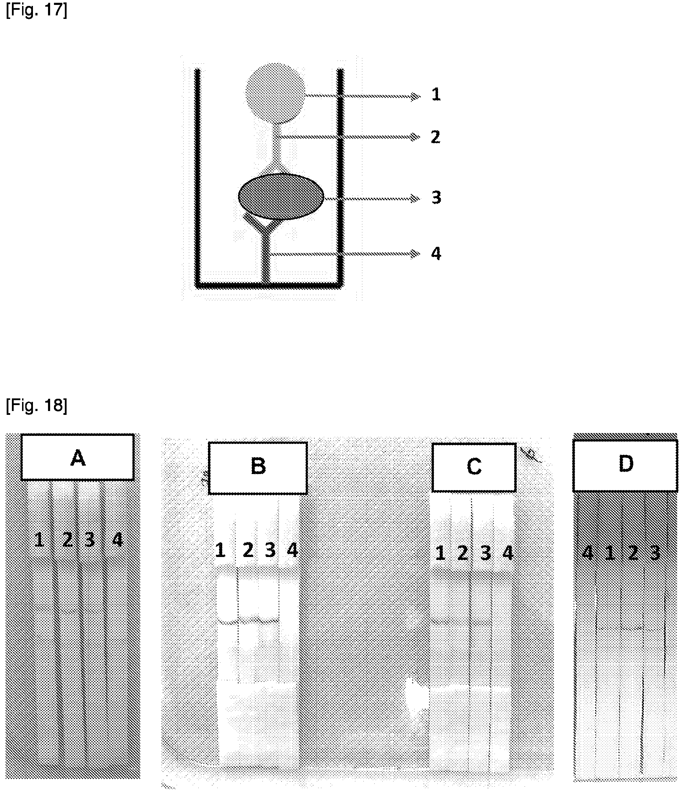

L'ELISA (enzyme-linked immunosorbent assay,) est un dosage immuno-enzymatique sur support solide, basé sur la réaction spécifique antigène-anticorps. Le principe classique d'un ELISA, dit de type sandwich, est d'utiliser un support, tel qu'une micro-plaque, sur lequel l'analyte va être immobilisé, par exemple via un anticorps de capture, immobilisé sur le support et spécifique de l'analyte. Après mise en présence du support avec l'échantillon susceptible de contenir l'analyte et rinçage, un anticorps primaire spécifique de l'analyte est ajouté. Cet anticorps primaire est couplé à un marqueur, permettant ainsi la détection de l'analyte en cas de signal. La Figure 17 illustre ce mode de réalisation d'un test ELISA.

Existe également l'ELISA indirect, dans lequel l'anticorps primaire n'est pas couplé à un marqueur, et l'on vient ajouter un anticorps secondaire dirigé contre l'anticorps primaire et couplé à un marqueur.

Lorsque le marqueur est une enzyme, l'ajout du substrat de l'enzyme permet d'obtenir une réaction colorée dosable par absorbance.

Les phages et/ou les protéines de phages décrits dans la description peuvent donc être utilisés dans un ELISA pour la détection de S. *aureus,* en remplacement de l'anticorps de capture et/ou de l'anticorps primaire couplé au marqueur.

Selon un sixième aspect, la présente divulgation concerne le bactériophage déposé à la CNCM sous le numéro CNCM I-5680 en tant que tel.

### DESCRIPTION DES FIGURES

**Fig. 1**

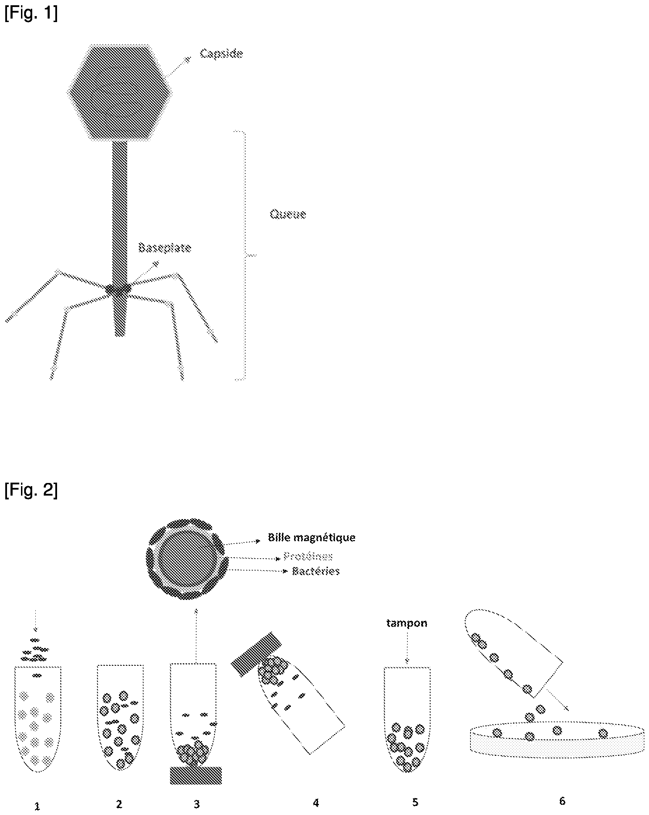

   [Fig. 1] Structure d'un phage ;
**Fig. 2**
   [Fig. 2] Schéma de la méthode des billes magnétiques pour évaluer la spécificité d'interaction entre les protéines de phages et les bactéries : 1. Ajout des bactéries dans un tube contenant des billes magnétiques recouvertes par des protéines de phages ; 2. Liaison des bactéries aux protéines fixées sur les billes magnétiques (si reconnaissance spécifique) ; 3. Précipitation des billes magnétiques à l'aide d'un support aimanté ; 4. Elimination du surnageant (bactéries non liées spécifiquement sur les billes) ; 5. Resuspension des billes magnétiques dans une solution tampon ; 6. Etalement des billes sur une boite de Pétri avec du milieu de culture.
**Fig. 3**

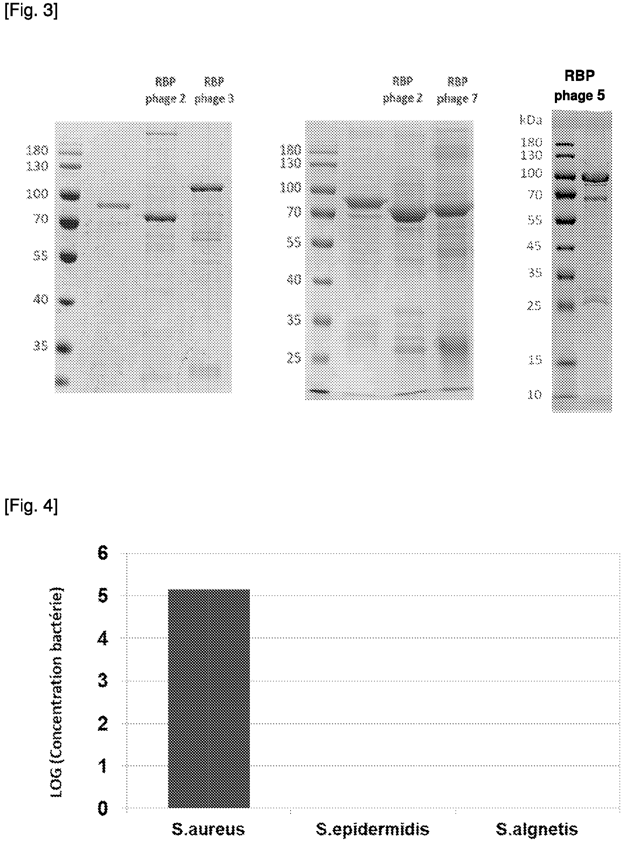

   [Fig. 3] Vérification de l'expression d'une protéine RBP (SEQ ID NO :1) du phage CNCM I-5408, d'une protéine RBP (SEQ ID NO :2) du phage CNCM I-5409, d'une protéine RBP (SEQ ID NO :3) du phage CNCM I-5410 et d'une protéine RBP (SEQ ID NO :4) du phage CNCM I-5680 sur gel d'agarose ;
**Fig. 4**
   [Fig. 4] Test de la spécificité d'une protéine RBP (SEQ ID NO :1) du phage CNCM I-5408 vis-à-vis de S. *aureus,* de *Staphylococcus epidermidis,* de *Staphylococcus agnetis ;*
**Fig. 5**

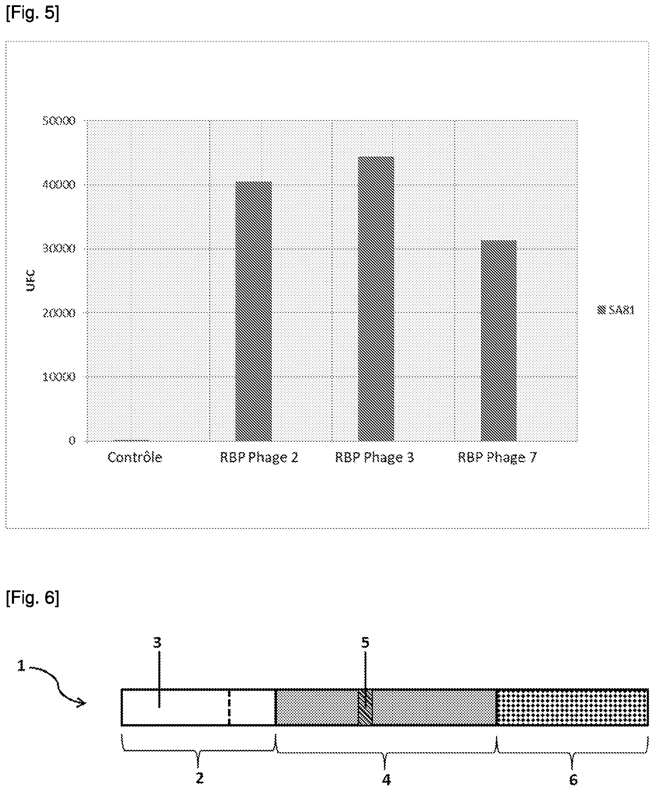

   [Fig. 5] Test d'affinité d'une protéine RBP (SEQ ID NO :1) du phage CNCM I-5408, d'une protéine RBP (SEQ ID NO :2) du phage CNCM I-5409 et d'une protéine RBP (SEQ ID NO :3) du phage CNCM I-5410 vis-à-vis d'une souche de S. *aureus* (SA 81) ;
**Fig. 6**
   [Fig. 6] Bandelette 1 de LFA comprenant une partie de contact 2 qui comprend une zone de contact 3, une partie de détection 4 qui comprend une zone de détection 5, et une partie absorbante 6 ;
**Fig. 7**

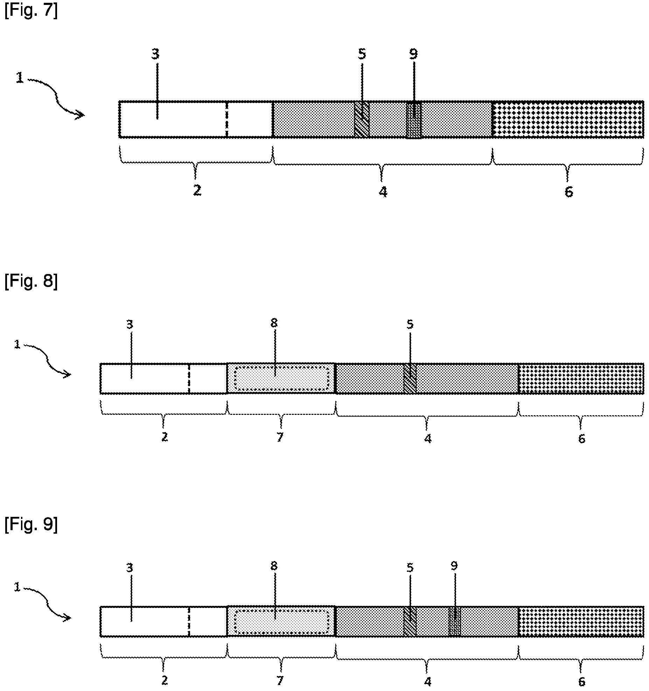

   [Fig. 7] Bandelette 1 de LFA comprenant une partie de contact 2 qui comprend une zone de contact 3, une partie de détection 4 qui comprend une zone de détection 5 et une zone de contrôle 9, et une partie absorbante 6 ;
**Fig. 8**
   [Fig. 8] Bandelette 1 de LFA comprenant une partie de contact 2 qui comprend une zone de contact 3, une partie de marquage 7 qui comprend une zone de marquage 8, une partie de détection 4 qui comprend une zone de détection 5 , et une partie absorbante 6 ;
**Fig. 9**
   [Fig. 9] Bandelette 1 de LFA comprenant une partie de contact 2 qui comprend une zone de contact 3, une partie de marquage 7 qui comprend une zone de marquage 8, une partie de détection 4 qui comprend une zone de détection 5 et une zone de contrôle 9, et une partie absorbante 6;
**Fig. 10**

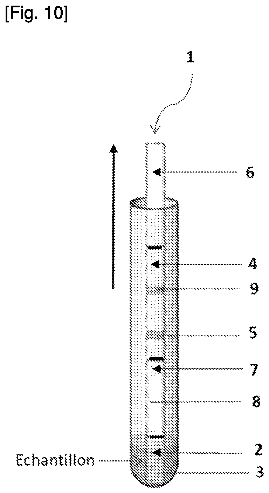

   [Fig. 10] Bandelette 1 de LFA comprenant une partie de contact 2 qui comprend une zone de contact 3, une partie de marquage 7 qui comprend une zone de marquage 8, une partie de détection 4 qui comprend une zone de détection 5 et une zone de contrôle 9, et une partie absorbante 6, plongée dans un échantillon à analyser ;
**Fig. 11**

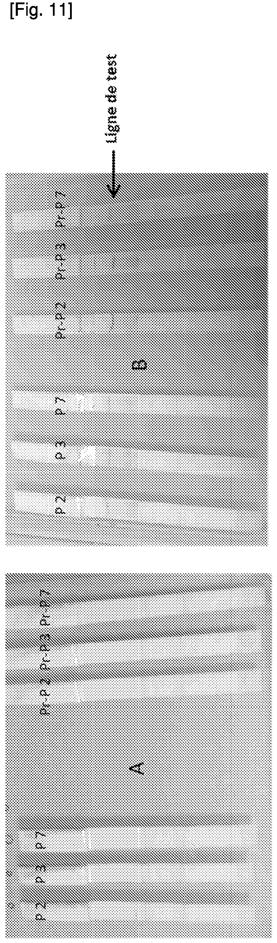

   [Fig. 11] Détection de S. *aureus* avec différentes bandelettes comprenant une partie de marquage où sont déposés des anticorps anti- S. *aureus* marqués avec des AuNP et une partie de détection où est immobilisé un phage 2 (CNCM I-5408, noté P2), un phage 3 (CNCM I-5409, noté P3), un phage 7 (CNCM I-5410, noté P7), une protéine de séquence SEQ ID NO :1 (RBP du phage 2 notée Pr-P2), une protéine de séquence SEQ ID NO :2 (RBP du phage 3, notée Pr-P3) ou une protéine de séquence SEQ ID NO :3 (RBP du phage 7, notée Pr-P7). Partie A = témoins, absence bactéries dans l'échantillon testé ; Partie B= échantillon (PBS) artificiellement contaminé avec la souche 79 de *S*. *aureus* (10⁷UFC/ml). L'ensemble des bandelettes sont réalisées sans lignes de contrôle.
**Fig. 12**

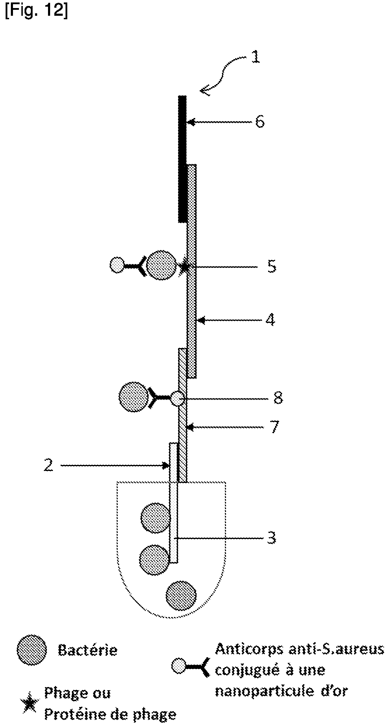

   [Fig. 12] Schéma d'un mode de réalisation de la détection d'une bactérie, en particulier S. *aureus,* dans un échantillon avec une bandelette 1 comprenant une partie de contact 2 comprenant une zone de contact 3, une partie de marquage 7 comprenant une zone de marquage 8 où est déposé un anticorps anti- S. *aureus* conjugué à une nanoparticule d'or, une partie de détection 4 comprenant une zone de détection 5 où est immobilisé un phage ou une protéine de phage spécifique de la bactérie, et une partie absorbante 6 ;
**Fig. 13**

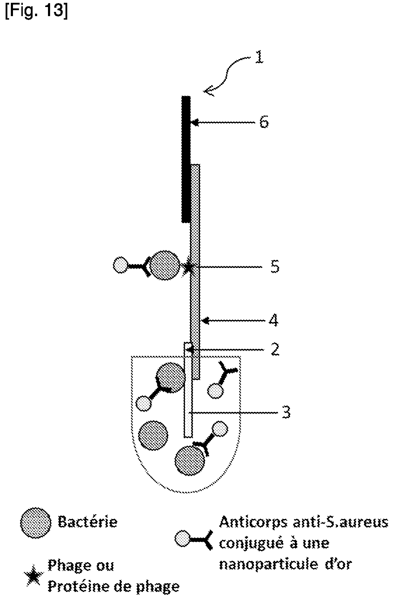

   [Fig. 13] Schéma d'un mode de réalisation de la détection d'une bactérie, en particulier S. *aureus,* dans un échantillon, auquel est directement ajouté un anticorps anti- *S. aureus* conjugué à une nanoparticule d'or, avec une bandelette 1 comprenant une partie de contact 2 comprenant une zone de contact 3, une partie de détection 4 comprenant une zone de détection 5 où est immobilisé un phage ou une protéine de phage spécifique de la bactérie, et une partie absorbante 6 ;
**Fig. 14**

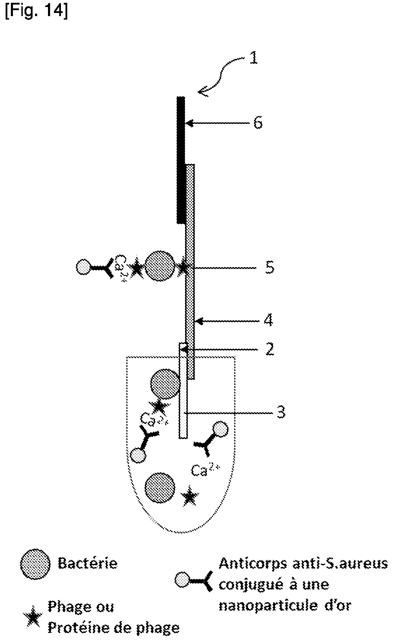

   [Fig. 14] Schéma d'un mode de réalisation de la détection d'une bactérie, en particulier S. *aureus,* dans un échantillon, auquel sont directement ajoutés un anticorps anti- *S*. *aureus* conjugué à une nanoparticule d'or et un phage ou une protéine de phage spécifique de la bactérie ainsi que du Ca2+ pour réaliser le couplage entre les deux, avec une bandelette 1 comprenant une partie de contact 2 comprenant une zone de contact 3, une partie de détection 4 comprenant une zone de détection 5 où est immobilisé un phage ou une protéine de phage spécifique de la bactérie, et une partie absorbante 6.
**Fig. 15**

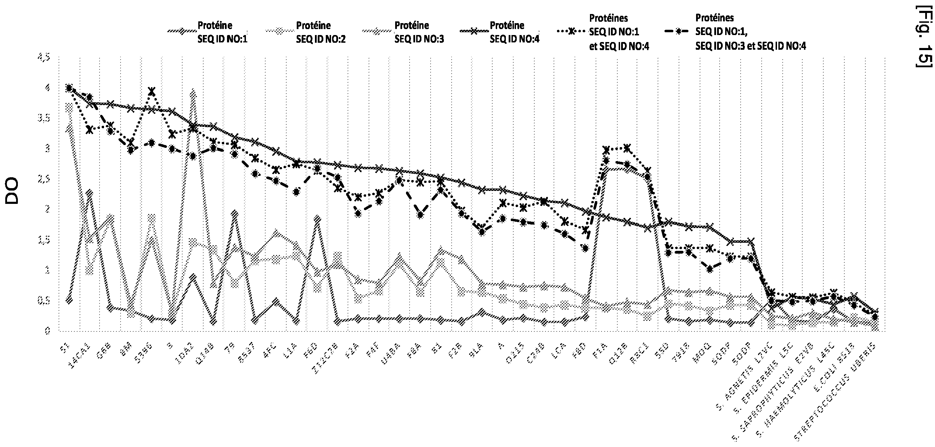

   [Fig. 15] Comparaison de la spécificité des protéines de séquences SEQ ID NO : 1, SEQ ID NO : 2, SEQ ID NO : 3, SEQ ID NO : 4, seules ou en mélange, par rapport à 24 souches différentes de S. aureus, une souche de *Staphylococcus saprophyticus,* une souche de *Staphylococcus epidermidis,* une souche de *Staphylococcus agnetis,* une souche d'Escherichia coli et une souche de *Streptococcus uberis.* Ces résultats ont été obtenus par un test ELISA dans lequel l'anticorps primaire a été remplacé par l'une des protéines biotinylées ou un mélange de celles-ci. Après migration, la révélation est faite par ajout de la peroxydase (HRP) couplée à la streptavidine, puis ajout du 3,3',5,5'-tétraméthylbenzidine (TMB) (substrat de l'enzyme HRP). L'apparition de la coloration bleue est mesurée dans chaque cas par densité optique (DO) à 540 nm.
**Fig. 16**

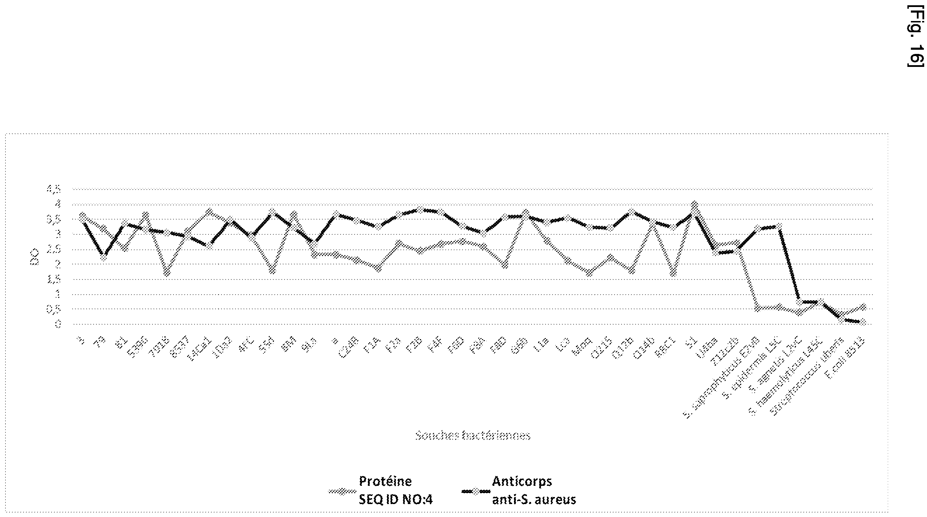

   [Fig. 16] Comparaison de la spécificité d'un anticorps anti-S. *aureus à* celle d'une protéine de phage, la protéine de séquence SEQ ID NO :4. Ces résultats sont obtenus de la même façon que pour la Figure 15 en utilisant pour la détection de la bactérie :
   - la protéine de séquence SEQ ID NO :4 biotinylée pour un marquage avec l'HRP, ou
   - un anticorps primaire Goat anti Rabbit IgG (H+L) couplé à HRP (Thermofischer, ref A16096) à 1 mg/mL.
**Fig. 17**
   [Fig. 17] Schéma d'un test ELISA de type sandwich : 1. Marqueur couplé à un anticorps. 2. Anticorps dirigé contre l'analyte. 3. Analyte (bactérie). 4. Anticorps de capture dirigé contre l'analyte et immobilisé sur le support.
**Fig. 18**
   [Fig. 18] Détection de trois souches différentes de *S. aureus* (bandelette 1, 2 et 3) avec des bandelettes comprenant une partie de marquage où sont déposés des anticorps anti- S. aureus marqués avec des AuNP et une partie de détection où est immobilisée une protéine de séquence SEQ ID NO :1 (RBP du phage 2) **(A),** une protéine de séquence SEQ ID NO :2 (RBP du phage 3) **(B),** une protéine de séquence SEQ ID NO :3 (RBP du phage 7) **(C)** ou une protéine de séquence SEQ ID NO :4 (RBP du phage 5) **(D).** Les bandelettes notées « 4 » sont les bandelettes témoins testées avec un échantillon ne contenant pas de bactéries. L'ensemble des bandelettes sont réalisées sans lignes de contrôle.
**Fig. 19**

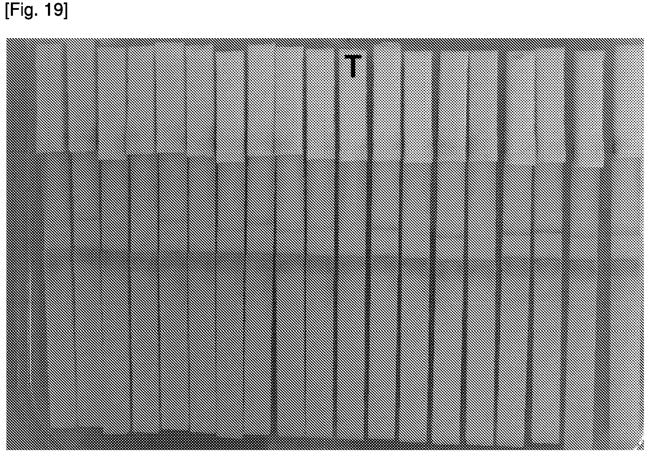

   [Fig. 19] Détection de 18 souches différentes de S. *aureus* dans un échantillon de lait cru, avec des bandelettes comprenant une partie de marquage où sont déposés des anticorps anti- S. aureus marqués avec des AuNP et une partie de détection où est immobilisée une protéine de séquence SEQ ID NO :4 (RBP du phage 5). La bandelette notée « T » est une bandelette témoin testée avec un échantillon de lait cru ne contenant pas de bactéries. L'ensemble des bandelettes sont réalisées sans lignes de contrôle.
**Fig. 20**

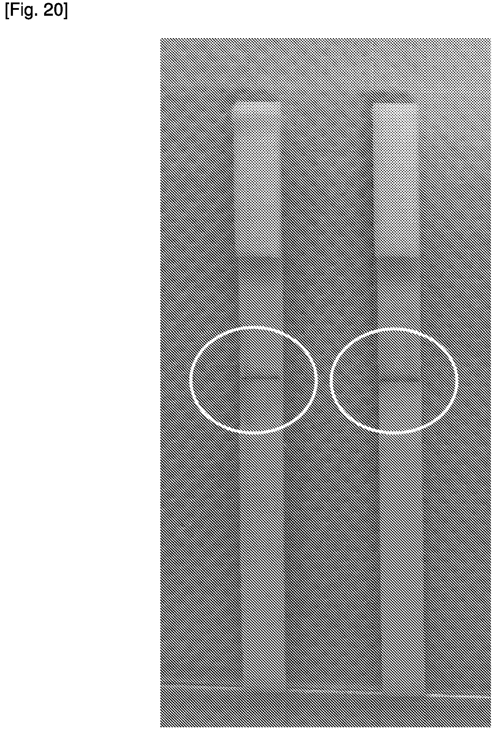

   [Fig. 20] Détection de d'une souche de S. *aureus* à deux concentrations différentes (10⁸ UFC/mL à gauche et 10⁷ UFC/mL à droite) avec des bandelettes comprenant une partie de marquage où est déposée une protéine de séquence SEQ ID NO :4 (RBP du phage 5) biotinylée, et une partie de détection où est immobilisé un anticorps anti- *S. aureus* au niveau de la ligne de test. Après migration, la révélation est faite par ajout de la peroxydase (HRP) couplée à la streptavidine, puis ajout du 3,3',5,5'-tétraméthylbenzidine (TMB) (substrat de l'enzyme HRP). L'apparition de la coloration bleue visible sur la ligne de test témoigne de la détection de la bactérie. L'ensemble des bandelettes sont réalisées sans lignes de contrôle.
**Fig. 21**

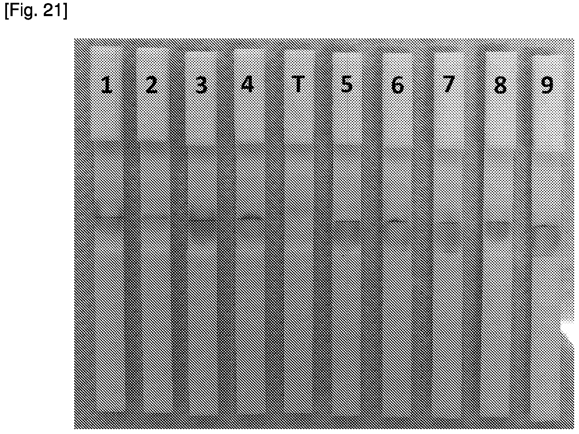

   [Fig. 21] Détection de 9 souches différentes de S. *aureus* (bandelettes 1 à 9) avec des bandelettes comprenant une partie de marquage où est déposée une protéine de séquence SEQ ID NO :4 (RBP du phage 5) biotinylée, et une partie de détection où est immobilisée une protéine de séquence SEQ ID NO :4 (RBP du phage 5) au niveau de la ligne de test. Après migration, la révélation est faite par ajout de la peroxydase (HRP) couplée à la streptavidine, puis ajout du 3,3',5,5'-tétraméthylbenzidine (TMB) (substrat de l'enzyme HRP). L'apparition de la coloration bleue visible sur la ligne de test témoigne de la détection de la bactérie. La bandelette notée « T » est une bandelette témoin testée avec un échantillon ne contenant pas de bactéries. L'ensemble des bandelettes sont réalisées sans lignes de contrôle.

### EXEMPLES

### Exemple 1 : Activité des bactériophages

### 1.1. Détermination de l'activité d'un bactériophage sur une souche bactérienne

Pour identifier si un bactériophage est spécifique d'une bactérie, on utilise la méthode des spots. Sur une boite de Pétri où est étalée une culture de bactérie en phase exponentielle, au moins un spot comprenant le bactériophage est déposé. Après 18h d'incubation à une température adaptée à la bactérie, si une plage de lyse est observée autour du spot, cela signifie que le bactériophage est actif pour cette bactérie, autrement dit que le bactériophage est spécifique de cette bactérie.

### 1.2. Bactériophages utilisés

Six phages ont été testés dans la présente demande:
- la souche de bactériophage déposée à la CNCM sous le numéro CNCM I-5408 (ci-après nommée phage 2) ;
- la souche de bactériophage déposée à la CNCM sous le numéro CNCM I-5409 (ci-après nommée phage 3);
- la souche de bactériophage déposée à la CNCM sous le numéro CNCM I-5410 (ci-après nommée phage 7) ;
- la souche de bactériophage déposée à la CNCM sous le numéro CNCM I-5680 (ci-après nommée phage 5) ;
- la souche de bactériophage déposée à la CNCM sous le numéro CNCM I-5491 (ci-après nommée phage 9) ;
- la souche de bactériophage déposée à la CNCM sous le numéro CNCM I-5492 (ci-après nommée phage 10).

### 1.3. Détermination du spectre d'activité des phages isolés

L'activité des phages 2, 3, 7, 9 et 10 a été testée sur différentes souches bactériennes issues de la collection de bactéries de Vetophage. Ces souches bactériennes ont été isolées à partir des échantillons collectés dans des fermes laitières en France. Au total 19 souches de S. *aureus* ont été utilisés pour les tests d'activité, ainsi que 5 souches d'autres espèces de staphylocoques, à savoir S. *haemolyticus, S. saprolyticus ; S. chromogenes, S. agnetis, S. epidermidis* et deux souches d'espèces non staphylocoques, *Streptococcus uberis* et *Escherichia coli.*

Ces souches bactériennes ont été cultivées sur la gélose BHI et incubées à 37 °C. Les culots bactériens ont été remis en suspension dans une solution saline tamponnée au phosphate (PBS) et ajustés à la concentration bactérienne requise.

### Résultats

L'activité des phages, c'est-à-dire leur capacité à infecter une souche bactérienne, a été testée par la méthode de spot précédemment décrite sur 19 souches de S. *aureus,* 5 souches d'autres espèces de Staphylocoques *(S. saprophyticus, S. chromogenes, S. haemolyticus, S. agnetis, S. epidermidis),* et d'une souche de bactéries non Staphylocoques *(Streptococcus uberis).* La capacité à infecter une souche bactérienne signifie que le bactériophage est capable de reconnaître ladite souche bactérienne et de s'y fixer dans le but de l'infecter pour la détruire.

Le tableau 1 représente le spectre d'activité des phages vis-à-vis de différentes souches de *S*. *aureus.* Un phage est considéré comme actif (noté « x »), lorsqu'une plage de lyse est obtenue par la méthode de spot. En revanche, lorsqu'aucune lyse est observée, l'absence d'activité est noté «/».

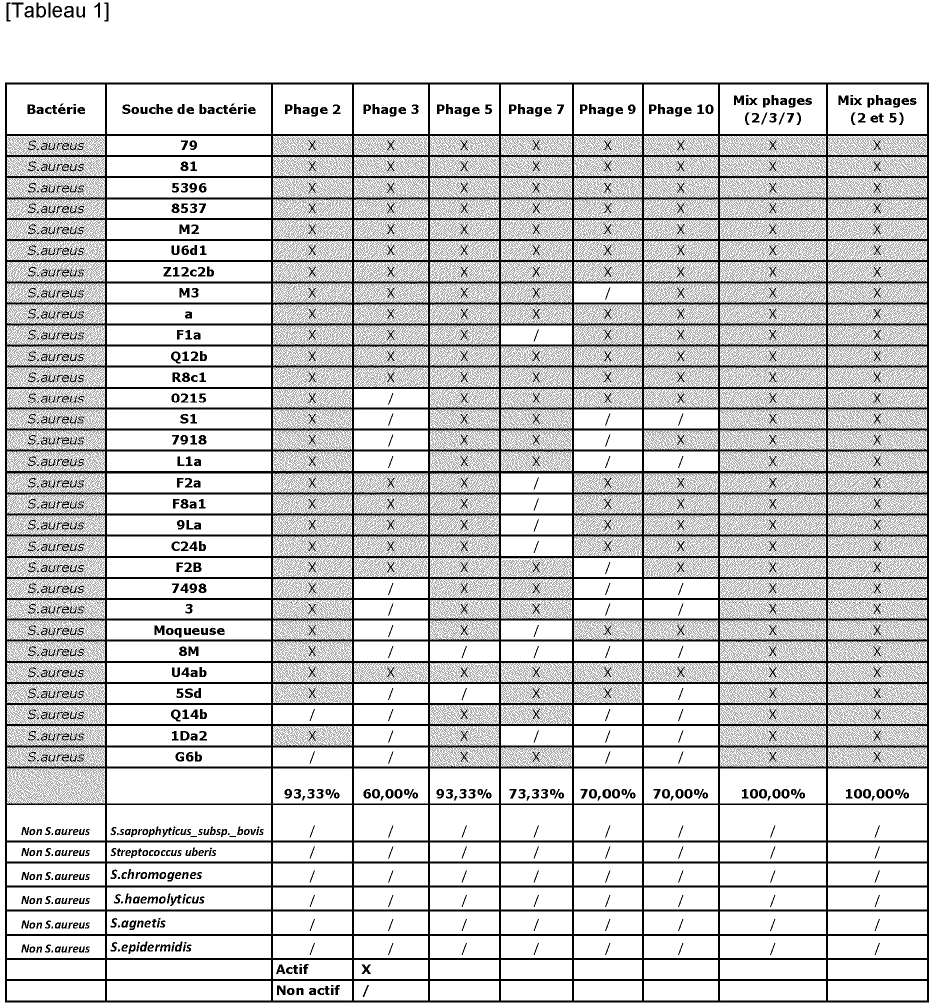

Ces résultats montrent que :
- Le bactériophage 2 déposé à la CNCM sous le numéro CNCM I-5408 (phage 2) infecte 93 % des souches ;
- Le bactériophage 3 déposé à la CNCM sous le numéro CNCM I-5409 (phage 3) infecte 60 % des souches;
- Le bactériophage 5 déposé à la CNCM sous le numéro CNCM 1-5680 (phage 5) infecte 93%
- Le bactériophage 7 déposé à la CNCM sous le numéro CNCM 1-5410 (phage 7) infecte 73 % ;
- Le bactériophage 9 déposé à la CNCM sous le numéro CNCM 1-5491 infecte 70 % des souches ;
- Le bactériophage 10 déposé à la CNCM sous le numéro CNCM I-5492 infecte 70 % des souches ;
- Le cocktail des trois phages (2, 3 et 7) ou de deux phages (2 et 5) infecte 100 % des souches.

En revanche, aucune activité des phages seuls ou en mélange n'a été observée vis-à-vis des souches bactériennes d'une autre espèce que S. *aureus* ou de souches bactériennes d'espèces non Staphylocoques. Cela démontre la spécificité des phages pour S. *aureus.*

### Exemple 2 : Sélection des protéines de bactériophages

### 2.1. Choix des protéines de bactériophages

L'adsorption des bactériophages dans la bactérie hôte s'effectue grâce à la reconnaissance spécifique à la surface bactérienne, des protéines de liaison des phages (Receptor Binding Protein ou RBP), localisées à la base (« baseplate ») de la queue des phages (Figure 1).

Afin de déterminer la spécificité de liaison à S. *aureus* de protéines de phages, les protéines suivantes ont été sélectionnées et synthétisées :
- une RBP du phage 2 (SEQ ID NO :1),
- une RBP du phage 3 (SEQ ID NO :2),
- une RBP du phage 7 (SEQ ID NO :3),
- une RBP du phage 5 (SEQ ID NO :4).

Pour cela, les phages 2, 3, 5 et 7 ont été séquencés. La recherche des RBPs a été réalisée à partir des annotations complètes des génomes des phages 2, 3, 5 et 7 en ciblant sur les séquences de la région « base plate ». L'alignement des séquences « base plate » de chaque phage a été réalisé un utilisant la technique BlastX des ORFs (open reading frame) des phages.

Les protéines RBPs ont été retrouvées au niveau du cluster des gènes de la queue aux alentours de 55000 pb à 84000 pb et plus précisément au niveau de la partie « base plate ».

Les protéines RBPs SEQ ID NO :1, SEQ ID NO :2, SEQ ID NO :3 et SEQ ID NO :4, respectivement des phages 2, 3, 7 et 5 ont ensuite été produites dans la bactérie BL21 par la technique de clonage. Pour cela, les gènes correspondants aux protéines de phages ont été clonés dans un vecteur d'expression bactérien permettant la synthèse des protéines en fusion avec l'enzyme Glutathione-S-Transférase (GST) par la bactérie hôte BL21.

### 2.2. Évaluation de la spécificité de liaison des protéines de phages à S. aureus par la méthode des billes magnétiques

Après purification, les protéines de séquences SEQ ID NO :1, SEQ ID NO :2 et SEQ ID NO :3 ont été séparément conjuguées avec des billes magnétiques de manière à ce que la surface de ces billes soit recouverte par l'une des protéines RBP.

Pour évaluer la spécificité de liaison de chacune de ces protéines à l'espèce bactérienne *S. aureus,* les billes magnétiques conjuguées aux protéines à tester ont été séparément ajoutées à un mélange de bactéries comprenant différentes espèces de Staphylocoques *(Staphylococcus aureus, Staphylococcus epidermidis et Staphylococcus agnetis).*

S'il y a une interaction spécifique (une affinité) entre la protéine testée et une ou plusieurs espèces bactériennes du mélange, la protéine va capturer la ou les bactéries (Figure 2).

Les billes sont ensuite séparées de la phase liquide grâce à un support aimanté, et lavées plusieurs fois avec une solution tampon afin d'éliminer les interactions non spécifiques.

Les billes sont ensuite récupérées et étalées en boite de Pétri sur une gélose spécifique (Baird Parker). Après 24 à 48h d'incubation, les espèces bactériennes fixées par les protéines sont identifiées visuellement selon la morphologie et la couleur des colonies se développant sur gélose. Le nombre de bactéries retenues est également déterminé par dénombrement des colonies sur la gélose. L'affinité ou l'interaction de la protéine testée avec la bactérie cible *(S. aureus)* est évaluée par le nombre de bactéries retenues. Plus le nombre de bactéries cibles sur la gélose est élevé, plus l'affinité de la protéine pour la bactérie cible est grande.

### Résultats

L'expression des protéines RBP SEQ ID NO :1, SEQ ID NO :2, SEQ ID NO :3 et SEQ ID NO :4, respectivement des phages 2, 3, 7 et 5 dans les bactéries BL21 a été vérifiée sur gel (Figure 3). Les tailles respectives de ces RBPs des phages 2, 3, 7 et 5 sont 78,1 kDa, 100,8 kDa, 79,5 kDa et 100 kDa.

La spécificité de liaison de ces protéines à l'espèce bactérienne *S*. *aureus* a ensuite été étudiée. Les résultats ont montré que la protéine RBP du phage 2 (SEQ ID NO :2) a une affinité pour la bactérie *Staphylococcus aureus* et uniquement pour cette espèce de Staphylocoque (Figure 4). De plus, les résultats en Figure 5 montrent que les protéines RBPs SEQ ID NO :1, SEQ ID NO :2 et SEQ ID NO :3, respectivement des phages 2, 3 et 7 ont une affinité pour l'espèce *S*. *aureus.*

### 2.3. Evaluation de la spécificité des protéines de phages par test ELISA

Le principe du test ELISA en sandwich a été adapté pour déterminer la spécificité des protéines de séquences SEQ ID NO :1, SEQ ID NO :2, SEQ ID NO :3 et SEQ ID NO :4, comme suit :
- Les protéines sont biotinylées préalablement (greffage de biotine) à l'aide d'un kit commercial (EZ-Link Micro NHS-PEG4-Biotinylation, Thermo, Réf : 21955),
- Une micro-plaque dans laquelle un anticorps de capture anti-S. *aureus* (Thermofischer, ref,PA1-7246) est immobilisé dans les puits, est utilisée.
- Incubation de la micro-plaque avec une solution bactérienne à 10⁷ UFC/mL pendant 1h30 à 37°C,
- retrait de la suspension bactérienne puis saturation des avec une solution contenant de la BSA à 3% et du Tween20 à 0,05 %,
- Elimination de la solution de saturation,
- Ajout d'une solution contenant les protéines biotinylées à 10µg/ml dans tampon PBS 1%BSA, 0,05% Tween20,
- Incubation 1h à 37°C,
- Laver 5 fois avec du tampon PBS -Tween de 0,05%,
- Ajout de la HRP (peroxydase de raifort- Horseradish peroxidase) couplée à la streptavidine (Invitrogene, ref, 434323) à 0,25 µg/mL, solution dans tampon PBS 1% BSA, 0,05% Tween20 pendant 1h30 à 37°C,
- Laver 5 fois avec du tampon PBS -Tween de 0,05%,
- Ajout de 100µl de solution de 3,3',5,5'-tétraméthylbenzidine (TMB) (substrat de l'enzyme HRP),
- Incubation 15 à 30 min à température ambiante,
- Ajout de 50µl d'acide sulfurique à 2 M pour stopper la réaction,
- Lecture de la plaque à 540nm.

La HRP va permettre l'oxydation du substrat TMB, entrainant une coloration détectable par spectrophotométrie. Ainsi, plus il y a de reconnaissance de la bactérie, plus le signal coloré sera fort.

En parallèle, pour comparer la spécificité d'un anticorps anti-S. *aureus à* celle d'une protéine de phage, le test décrit ci-dessus a été réalisé en utilisant pour la détection de la bactérie :
- la protéine de séquence SEQ ID NO :4 biotinylée pour un marquage avec l'HRP ou
- un anticorps primaire Goat anti Rabbit IgG (H+L) couplé à HRP (Thermofischer, ref A16096) à 1 mg/mL.
Dans les deux cas, l'anticorps de capture est resté un anti-S. *aureus* (Thermofischer, ref,PA1-7246).

### Résultats

Les résultats du test de spécificité des protéines de phages de séquences SEQ ID NO :1, SEQ ID NO :2, SEQ ID NO :3 et SEQ ID NO :4 par ELISA sont donnés en Figure 15.

L'affinité de la protéine à la bactérie est exprimée par la quantité de protéines retenue par chaque souche bactérienne. Cette quantité est évaluée par l'absorbance liée à la streptavidine couplé à HRP pour les protéines.

24 souches de *S*. aureus ont été testées, ainsi que trois souches de Staphylocoques non aureus (une souche de *Staphylococcus saprophyticus,* une souche de *Staphylococcus epidermidis* et une souche de *Staphylococcus agnetis),* et deux souches de bactéries non Staphylocoques (une souche *d'Escherichia coli* et une souche de *Streptococcus uberis).*

La Figure 15 montre que :
- La protéine de séquence SEQ ID NO :1 du phage 2reconnait 28 % des souches testées.
- La protéine de séquence SEQ ID NO :2 du phage 3 reconnait 75 % des souches testées.
- La protéine de séquence SEQ ID NO :3 du phage 4 reconnait 78 % des souches testées.
- La protéine de séquence SEQ ID NO :4 du phage 5 reconnait 100 % des souches testées.

Les protéines sont plus spécifiques que l'anticorps anti-S. *aureus.* Elles ne reconnaissent pas les souches de Staphylocoques non aureus et les autres espèces bactériennes alors que l'anticorps anti-*S*. *aureus* reconnait *Staphylococcus saprophyticus* et *Staphyoloccus epidermidis* (Figure 16).

Cela témoigne de la meilleure spécificité de ces protéines par rapport à un anticorps anti-*S. aureus.* Elles sont donc aptes à être utilisées pour la détection de *S*. *aureus.*

A noter que la reconnaissance de certaines souches de *S. aureus* (F1a, Q12b, R8C1) peut être améliorée par le mélange des protéines.

### Exemple 3 : Dispositif LFA avec des phages ou protéines de phages

### 3.1. Dispositif LFA (4 parties) avec des phages ou protéines de phages dans la partie de détection

Les Inventeurs ont mis au point une bandelette 1 de chromatographie à flux latéral (LFA) permettant de détecter la présence d'une bactérie d'intérêt dans un échantillon, en particulier la présence de *S. aureus,* grâce à un phage ou une protéine d'un phage spécifique de ladite bactérie d'intérêt.

Un premier mode de réalisation est une bandelette 1 de LFA pour détecter *S*. *aureus* dans un échantillon, qui comprend quatre parties:
- une partie de contact 2 (sample pad) comprenant une zone de contact 3;
- une partie de marquage 7 (conjugate pad) comprenant une zone de marquage 8;
- une partie de détection 4 (test pad) comprenant une zone de détection 5 ;
- une partie absorbante 6 (absorption pad) ; tel que l'une des extrémités de la partie de marquage 7 est superposée à l'une des extrémités de la partie de contact 2, l'autre extrémité de la partie de marquage 7 est superposée avec l'une des extrémités de la partie de détection 4, et l'autre extrémité de la partie de détection 4 est superposée avec l'une des extrémités de la partie absorbante 6, pour former respectivement une première, une seconde et une troisième zone de superposition, lesdites zones de superposition étant séparées les unes des autres.

La partie de contact 2 (sample pad) comprend une zone de contact 3 destinée à être mise en contact avec l'échantillon, et qui est distincte de la première zone de superposition. Lors de la mise en contact de la zone de contact 3 avec l'échantillon, un flux de migration de l'échantillon par capillarité, de la partie de contact 2 jusqu'à la partie absorbante 6, s'instaure.

La partie de marquage 7 (Conjugate pad) comprend une zone de marquage 8, distincte de la première et la deuxième zone de superposition, où est déposée un marqueur conjugué à un ligand spécifique de *S*. *aureus.* En particulier, il s'agit d'une nanoparticule d'or (AuNP) conjuguée à un anticorps dirigé contre *S*. *aureus.*

Lors de la migration de l'échantillon au travers de la zone de marquage 8, le ligand marqué, e. g. l'anticorps anti- *S*. *aureus* conjugué à la nanoparticule d'or, se lie à la bactérie S. *aureus,* formant ainsi un complexe bactérie/ligand marqué. Ledit complexe est ensuite entrainé par le flux de migration au travers de la partie de marquage 7, puis au travers de la partie de détection 4.

La partie de détection 4 comprend une zone de détection 5 distincte de la deuxième et de la troisième zone de superposition, où est immobilisé au moins un phage spécifique de *S*. *aureus* ou au moins une protéine d'au moins un phage spécifique de S. *aureus.* La zone de détection 5 est une ligne transversale à la bandelette 1, qui peut alors être appelée « ligne de détection » ou « ligne de test » (test line).

Au passage de la ligne de test, les complexes bactérie/ligand marqué sont immobilisés par la reconnaissance spécifique de *S*. *aureus* par le phage ou la protéine de phage spécifique de *S*. *aureus* immobilisé sur la ligne de test.

La présence de *S*. *aureus* est alors révélée grâce au marqueur. L'accumulation des AuNP sur la ligne de test entraine l'apparition d'un signal visuel coloré, en particulier de couleur rose.

La Figure 8 illustre un mode de réalisation d'une bandelette 1 de détection de *S*. *aureus* dans un échantillon, comprenant les quatre parties suivantes :
- une partie de contact 2 comprenant une zone de contact 3 ;
- une partie de marquage 7 comprenant une zone de marquage 8 où peut être déposé un anticorps dirigé contre *S*. *aureus* marqué avec une nanoparticule d'or (AuNP) en tant que marqueur conjugué à un ligand spécifique de *S*. *aureus ;*
- une partie de détection 4 comprenant une zone de détection 5 de *S*. *aureus,* où peut être immobilisé au moins un bactériophage spécifique de *S*. *aureus* ou au moins une protéine d'au moins un bactériophage spécifique de *S*. *aureus,*
- une partie absorbante 6.

La figure 12 illustre un mode de réalisation de la détection avec une bandelette 1 comprenant quatre parties.

Optionnellement, la bandelette 1 comprend un système de contrôle de la migration de l'échantillon de la partie de contact 2 jusqu'à la partie de détection 4. Pour cela, un premier ligand marqué non spécifique de la bactérie d'intérêt, c'est-à-dire qui n'a pas d'affinité pour la bactérie d'intérêt, tel qu'un anticorps primaire marqué, est déposé sur la partie de marquage 7 et/ou est préalablement ajouté à l'échantillon, et la partie de détection 4 comprend une zone de contrôle 9, distincte de la zone de détection 5, où est immobilisé un deuxième ligand qui est spécifique du premier ligand marqué, tel qu'un anticorps secondaire.

En particulier, cette zone de contrôle 9 est une ligne transversale à la bandelette 1, formant ainsi une ligne de contrôle ou ligne témoin (control line). En particulier, la zone de contrôle 9 est sur la partie de détection 4, entre la deuxième et de la troisième zone de superposition.

Le premier ligand marqué de la partie de marquage 7 va être entrainé par capillarité avec l'échantillon jusqu'à la ligne de contrôle, où il est systématiquement immobilisé par la reconnaissance spécifique du second ligand immobilisé sur la ligne de contrôle.

La présence du premier ligand sur la ligne de test est alors révélée grâce au marqueur. L'apparition du signal le long de la ligne de contrôle permet de s'assurer que la migration à travers la membrane s'est effectuée correctement. L'apparition de ce signal sur la ligne de contrôle est indépendante de la présence de la bactérie d'intérêt dans l'échantillon testé. En particulier, le marqueur est une AuNP.

Ainsi, dans une bandelette 1 comprenant un tel système de contrôle, une analyse positive de la présence de l'analyte d'intérêt dans l'échantillon est caractérisée par l'apparition de deux signaux visuels, l'un sur la ligne de test et l'autre sur la ligne de contrôle. A l'inverse, l'absence d'analyte d'intérêt dans l'échantillon est caractérisée par l'apparition d'un seul signal visuel sur la ligne de contrôle.

Dans un mode de réalisation du système de détection, le premier ligand marqué déposé sur la zone de marquage 8 est un anticorps de poulet couplé à une AuNP, et le deuxième ligand immobilisé sur la ligne de contrôle est un anticorps anti-anticorps de poulet.

La figure 9 illustre un mode de réalisation de la bandelette 1 comprenant quatre parties et un système de contrôle. La figure 10 illustre un mode de réalisation de la détection avec une bandelette 1 comprenant quatre parties et une zone de contrôle 9.

### 3.2. Evaluation d'un dispositif de LFA (4 parties) avec des phages ou protéines de phages dans la partie de détection

Des bandelettes 1 de LFA pour détecter *S*. *aureus* comme décrites à la partie 3.1, ont été réalisées, sans système de contrôle, en immobilisant sur la zone de détection 5 ( ligne de test) en tant que bactériophage spécifique de *S*. *aureus :*
- le phage 2 (CNCM I-5408),
- le phage 3 (CNCM I-5409),
- le phage 7 (CNCM I-5410), ou
- les phages 2, 3 et 7,
ou en immobilisant sur la zone de détection 5 en tant que protéine de bactériophage spécifique de *S. aureus :*
- une protéine RBP du phage 2 (SEQ ID NO :1),
- une protéine RBP du phage 3 (SEQ ID NO :2) ,
- une protéine RBP du phage 7 (SEQ ID NO :3),
- une protéine RBP du phage 5 (SEQ ID NO :4) ou
- les protéine RBP SEQ ID NO :1, SEQ ID NO :2 et SEQ ID NO :3 respectivement de chacun des phages 2, 3 et 7.

Les phages ont été déposés sur la zone de détection 5 (ligne de test) à une concentration de 10¹⁰ UFC/mL. Les protéines ont été déposées sur la zone de détection 5 (ligne de test) à une concentration de 0,5 mg/mL.

Pour chaque bandelette 1, l'anticorps anti- *S*. *aureus, Staphylococcus aureus* Rabbit Polyclonal Antibody fourni par Thermofisher (Réf. 11578351), conjugué à des nanoparticules d'or, a été imprégné sur la zone de marquage 8.

Ces 8 modes de réalisation de la bandelette 1 ont été testés pour la détection de 19 souches de S. *aureus* dans des échantillons de tampon PBS infectés artificiellement avec 10⁷ UFC/mL de bactéries. Des tests avec des échantillons de PBS infectés avec 10⁶ UFC/mL ont également été effectués.

Pour évaluer la spécificité, les 8 modes de réalisation de la bandelette 1 ont également été testés des échantillons de PBS infectés séparément avec d'autres espèces de Staphylocoques *(Staphylococcus aureus, Staphylococcus epidermidis, Staphylococcus agnetis)* à des concentrations de 10⁷UFC/mL.

La Figure 11 montre les résultats obtenus pour la détection de la souche 79 de S. *aureus* à 10⁷ UFC/mL dans un échantillon de PBS, avec les bandelettes 1 de LFA où le phage 2, 3 ou 7, ou où une RBP du phage 2 (SEQ ID NO :1), une RBP du phage 3 (SEQ ID NO :2) ou une RBP du phage 7 (SEQ ID NO :3), ont été immobilisés sur la ligne de test.

Lorsque l'échantillon ne contient pas de bactérie (témoin) (Figure 11 - côté A) aucun signal n'est observé sur la ligne de test. Lorsque l'échantillon contient des bactéries, un signal de couleur rose est observé le long de la ligne de test où lesdits phages ou lesdites protéines desdits phages ont été immobilisés (Figure 11- côté B).

Le tableau 2 présente les résultats obtenus pour chaque bandelette 1 testée par rapport à chaque échantillon. Un test de détection positif de la bactérie est caractérisé par l'apparition du signal rose indiqué « S » pour « signal » dans le tableau. Un test négatif est indiqué par le signe « / ».

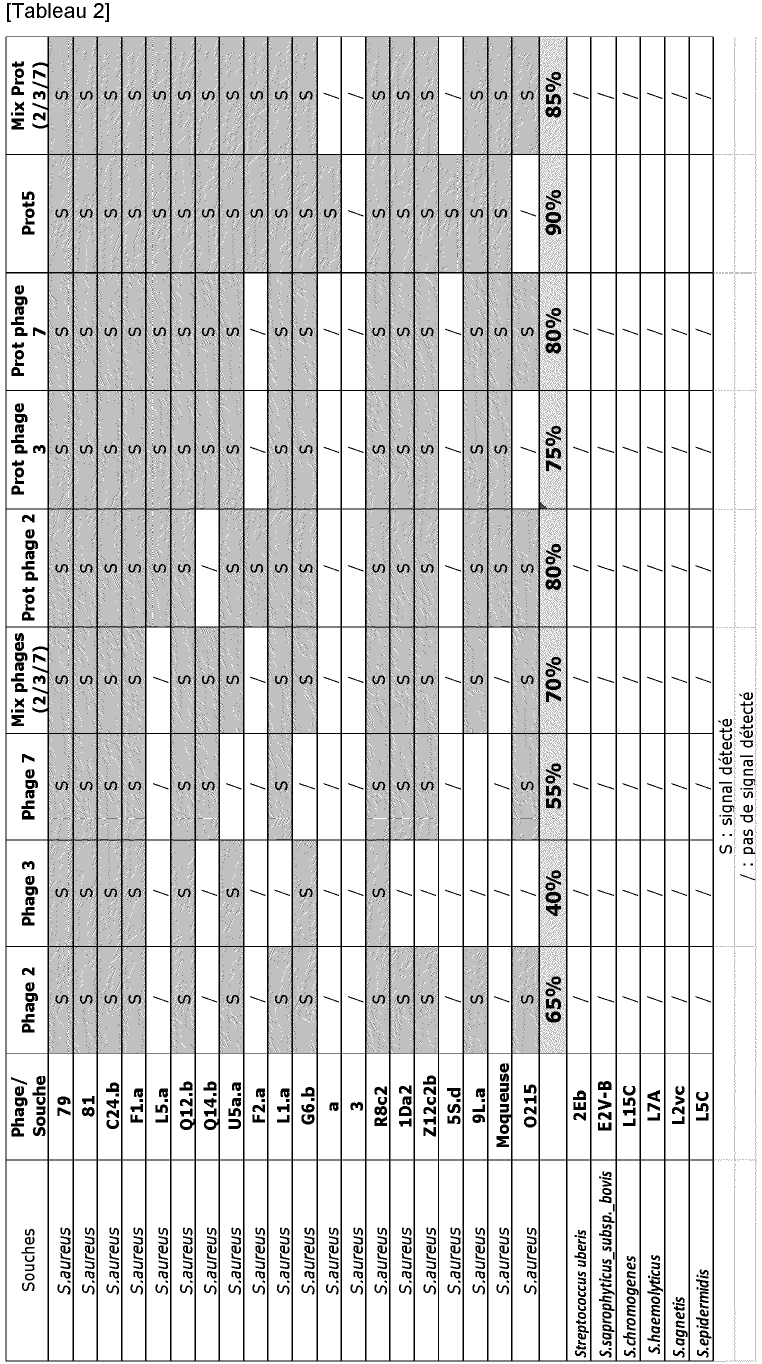

Les résultats du tableau 2 montrent qu'une bandelette 1 de LFA comprenant l'un des phages 2, 3 ou 7 ou ces trois phages, ou comprenant une protéine d'un de ces trois phages ou un mélange de ces trois protéines, est très spécifique pour la détection de la présence de la bactérie S. *aureus* dans un échantillon.

Les résultats de la Figure 11 et du Tableau 2 valident donc l'utilisation de phages entiers spécifiques de *S*. *aureus* ou de protéines de phages spécifiques de *S*. *aureus,* en particulier des RBPs, dans une bandelette 1 de LFA pour détecter la présence de *S*. *aureus* dans un échantillon.

### 3.3. Traitement préalable de l'échantillon

Un traitement préalable de l'échantillon consistant en l'ajout d'un surfactant, a été testé.

Pour cela, du tween 20 de 0,5% à 12% (w/v) a été ajouté, dans des échantillons de tampon PBS infectés avec la souche 79 de S. *aureus.* Ces échantillons ont ensuite été testés sur une bandelette 1 de LFA décrite ci-dessus comprenant le phage 2 ou la protéine RBP du phage 2 (SEQ ID NO :1) immobilisé sur la ligne de test.

Les résultats montrent qu'un échantillon comprenant de 0,5% à 12% (w/v) de surfactant permet d'obtenir un signal de détection plus intense (résultats non montrés).

### Exemple 4 : Dispositif de LFA avec des phages ou protéines de phages dans la partie de marquage

Un mode de réalisation alternatif le dispositif de LFA est une bandelette 1 de LFA pour détecter S. *aureus,* telle que décrite à l'Exemple 3, dans laquelle :
- sur la zone de marquage 8, au lieu de déposer un marqueur conjugué à un anticorps anti- S. *aureus,* il s'agit d'un marqueur conjugué à au moins un phage spécifique de S. *aureus* ou à au moins une protéine d'au moins un phage spécifique de *S*. *aureus,* et
- sur la zone de détection 5, en particulier sur la ligne de test, au lieu d'immobiliser au moins un phage spécifique de *S*. *aureus* ou au moins une protéine d'au moins un phage spécifique de S. *aureus,* il s'agit d'un anticorps dirigé contre *S*. *aureus.*

Dans ce mode de réalisation alternatif, la bandelette 1 de LFA pour détecter *S*. *aureus* dans un échantillon comprend dans l'ordre les quatre parties suivantes :
- une partie de contact 2 comprenant une zone de contact 3 avec l'échantillon;
- une partie de marquage 7 comprenant une zone de marquage 8 où est déposé un marqueur conjugué à au moins un phage spécifique de *S*. *aureus* ou un marqueur conjugué à au moins une protéine de phage spécifique de *S*. *aureus,*
- une partie de détection 4 comprenant une zone de détection 5 de *S*. *aureus,* où est immobilisé un anticorps anti- *S*. *aureus,*
- une partie absorbante 6.

La zone de marquage 8 peut contenir un marqueur conjugué au phage 2 (CNM I-5408), un marqueur conjugué au phage 3 (CNCM I-5409) ou un marqueur conjugué au phage 7 (CNCM I-5410) ou ces trois marqueurs conjugués. Elle peut également contenir un marqueur conjugué à une protéine du phage 2, un marqueur conjugué à une protéine du phage 3, un marqueur conjugué à une protéine du phage 7 ou ces trois marqueurs conjugués. En particulier, le marqueur est une nanoparticule d'or. En particulier, ladite protéine du phage 2 est la protéine SEQ ID NO :1, ladite protéine du phage 3 est la protéine SEQ ID NO :2, ladite protéine du phage 7 est la protéine SEQ ID NO :3.

### Exemple 5 : Dispositif de LFA (3 parties) avec des phages ou protéines de phages dans la partie de détection

Un mode de réalisation alternatif le dispositif de LFA est une bandelette 1 de LFA pour détecter *S*. *aureus* telle que décrite à l'Exemple 3 dans laquelle la partie de marquage 7, où est déposé un ligand marqué spécifique de la bactérie, tel qu'un anticorps anti- *S*. *aureus,* est absente. En effet, dans ce mode de réalisation, l'échantillon est préalablement mis en présence du marqueur conjugué à un ligand spécifique de la bactérie, avant la mise en contact de l'échantillon avec la bandelette 1.

Dans ce mode de réalisation alternatif, la bandelette 1 de détection de S. *aureus* dans un échantillon, comprend dans l'ordre les trois parties suivantes :
- une partie de contact 2 comprenant une zone de contact 3 avec l'échantillon ;
- une partie de détection 4 comprenant une zone de détection 5 de *S*. *aureus,* où est immobilisé au moins un bactériophage spécifique de *S*. *aureus* ou au moins une protéine d'au moins un bactériophage spécifique de *S*. *aureus,*
- une partie absorbante 6.

La zone de détection 5 peut contenir un phage 2 (CNM I-5408), un phage 3 (CNCM I-5409) ou un phage 7 (CNCM I-5410) ou ces trois phages. Elle peut également contenir une protéine du phage 2, telle que la protéine SEQ ID NO :1, une protéine du phage 3 telle que la protéine SEQ ID NO :2, une protéine du phage 7 telle que la protéine SEQ ID NO :3, ou ces trois protéines.

En utilisant ce mode de réalisation alternatif de la bandelette 1, l'échantillon est préalablement mis en présence avec un marqueur conjugué à un ligand spécifique *S. aureus,* pour former dans l'échantillon des complexes bactérie/ligand marqué.

Après marquage, lorsque l'échantillon est mis en contact avec la zone de contact 3 de la bandelette 1, les complexes vont migrer au travers de la partie de contact 2 par capillarité, puis au travers de la partie de détection 4, jusqu'à la zone de détection 5, en particulier une ligne de test, où est immobilisé au moins un phage spécifique de S. *aureus* ou au moins une protéine d'au moins un phage de S. *aureus.*

La figure 6 illustre une bandelette 1 comprenant trois parties. La figure 7 illustre une bandelette 1 comprenant trois parties dans laquelle la partie de détection 4 comprend une zone de contrôle 9.

En utilisant cette bandelette 1 alternative, un premier mode de réalisation du procédé de détection de la bactérie est un procédé dans lequel le marqueur conjugué à un ligand spécifique S. *aureus,* avec lequel l'échantillon est préalablement mis en contact, est un marqueur conjugué à un anticorps dirigé contre S. *aureus.*

La Figure 13 illustre ce mode de réalisation alternatif.

En utilisant cette bandelette 1 alternative, un deuxième mode de réalisation du procédé de détection de la bactérie est un procédé dans lequel le marqueur conjugué à un ligand spécifique S. *aureus,* avec lequel l'échantillon est préalablement mis en contact, est un marqueur conjugué à au moins un phage spécifique de *S*. *aureus* ou un marqueur conjugué à au moins une protéine d'au moins un phage spécifique de *S*. *aureus.*

En particulier, il s'agit d'un marqueur conjugué au phage 2 (CNM I-5408), d'un marqueur conjugué au phage 3 (CNCM I-5409), d'un marqueur conjugué au phage 7 (CNCM I-5410) ou d'un mélange de ces trois marqueurs conjugués à des phages. En particulier, il s'agit d'un marqueur conjugué à une protéine du phage 2, d'un marqueur conjugué à une protéine du phage 3, d'un marqueur conjugué à une protéine du phage 7 ou d'un mélange de ces trois marqueurs conjugués à des protéines. En particulier, ladite protéine du phage 2 est la protéine SEQ ID NO :1, ladite protéine du phage 3 est la protéine SEQ ID NO :2, ladite protéine du phage 7 est la protéine SEQ ID NO :3.

En utilisant cette bandelette 1 alternative, un troisième mode de réalisation du procédé de détection de la bactérie est un procédé lequel le marqueur conjugué à un ligand spécifique *S*. *aureus,* avec lequel l'échantillon est préalablement mis en contact, est un marqueur conjugué à un anticorps anti- *S*. *aureus* couplé à au moins un phage spécifique de *S*. *aureus,* ou un marqueur conjugué à un anticorps anti- S. *aureus* couplé à au moins une protéine d'un phage spécifique de *S*. *aureus.* Le couplage est réalisé par des ions Ca²⁺. La Figure 14 illustre ce troisième de mode de réalisation du procédé.

En particulier, il s'agit d'un marqueur conjugué à un anticorps anti- *S*. *aureus* couplé au phage 2 (CNCM I-5408), d'un marqueur conjugué à un anticorps anti- *S*. *aureus* couplé au phage 3 (CNCM I-5409), d'un marqueur conjugué à un anticorps anti- *S*. *aureus* couplé au phage 7 (CNCM I-5410) ou d'un mélange de ces trois marqueurs conjugués à des phages. En particulier, il s'agit d'un marqueur conjugué à un anticorps anti- *S*. *aureus* couplé à une protéine du phage 2 (CNCM I-5408), d'un marqueur conjugué à un anticorps anti- S. *aureus* couplé à une protéine du phage 3 (CNCM I-5409), d'un marqueur conjugué à un anticorps anti- S. *aureus* couplé à une protéine du phage 7 (CNCM I-5410) ou d'un mélange de ces trois marqueurs conjugués à des protéines de phages. En particulier, ladite protéine du phage 2 est la protéine SEQ ID NO :1, ladite protéine du phage 3 est la protéine SEQ ID NO :2, ladite protéine du phage 7 est la protéine SEQ ID NO :3.

Lorsque le marqueur est une AuNP, la présence de la bactérie dans l'échantillon est mise en évidence par l'apparition d'un signal visuel, en particulier de couleur rose, le long de la ligne de test.

### Exemple 6 : Dispositif de LFA (3 parties) avec des phages ou protéines de phages

Un mode de réalisation alternatif du dispositif est une bandelette 1 de LFA pour détecter *S. aureus,* telle que décrite à l'Exemple 5, dans laquelle :
- sur la zone de détection 5 au lieu d'immobiliser au moins un phage spécifique de *S*. *aureus* ou au moins une protéine d'au moins un phage spécifique de *S*. *aureus,* il s'agit d'un anticorps spécifique de *S*. *aureus* ou d'un aptamère spécifique de *S*. *aureus.*

Dans ce mode de réalisation alternatif, la bandelette 1 de LFA pour détecter *S*. *aureus* dans un échantillon, comprend dans l'ordre les trois parties suivantes :
- une partie de contact 2 comprenant une zone de contact 3 avec l'échantillon,
- une partie de détection 4 comprenant une zone de détection 5 de *S*. *aureus,* où est immobilisé un anticorps spécifique de *S*. *aureus* ou un aptamère spécifique de *S*. *aureus.*
- une partie absorbante 6.

En utilisant cette bandelette 1, un premier mode de réalisation du procédé de détection de *S. aureus* est un procédé dans lequel l'échantillon est préalablement mis en contact avec un marqueur conjugué à un phage spécifique de *S*. *aureus,* ou avec un marqueur conjugué à une protéine d'un phage spécifique de *S*. *aureus,* pour former dans l'échantillon des complexes bactérie/phage marqué ou bactérie/protéine marquée.

En particulier, il s'agit d'un marqueur conjugué au phage CNCM I-5408, un marqueur conjugué au phage CNCM I-5409, et/ou un marqueur conjugué au phage CNCM I-5410. Plus particulièrement, il s'agit d'un marqueur conjugué au phage CNCM I-5408, un marqueur conjugué au phage CNCM I-5409 et un marqueur conjugué au phage CNCM I-5410. Encore plus particulièrement, ledit marqueur conjugué à ces phages est une nanoparticule d'or.

En particulier, il s'agit d'un marqueur conjugué à au moins une protéine du phage CNCM I-5408, un marqueur conjugué à au moins une protéine du phage CNCM I-5409, et/ou un marqueur conjugué à au moins une protéine du phage CNCM I-5410. Plus particulièrement, il s'agit d'un marqueur conjugué à au moins une protéine du phage CNCM I-5408, un marqueur conjugué à au moins une protéine du phage CNCM I-5409 et un marqueur conjugué à au moins une protéine du phage CNCM I-5410. En particulier, ladite au moins une protéine est une RBP. En particulier, ladite protéine du phage 2 est la protéine SEQ ID NO :1, ladite protéine du phage 3 est la protéine SEQ ID NO :2, ladite protéine du phage 7 est la protéine SEQ ID NO :3. Plus particulièrement, ledit marqueur conjugué aux protéines de phages est une nanoparticule d'or.

En utilisant cette bandelette 1, un deuxième mode de réalisation du procédé de détection de la bactérie est un procédé dans lequel l'échantillon est préalablement mis en contact avec un marqueur conjugué à un phage spécifique de *S*. *aureus* couplé à un anticorps anti- *S*. *aureus* marqué, un marqueur conjugué à un anticorps anti- *S*. *aureus* couplé à un phage spécifique de *S*. *aureus,* un marqueur conjugué à une protéine d'un phage spécifique de S. *aureus* couplée à un anticorps anti-*S. aureus* ou un marqueur conjugué à un anticorps anti- *S*. *aureus* couplé à une protéine d'un phage spécifique de *S*. *aureus..*

En particulier, il s'agit d'un marqueur conjugué au phage CNCM I-5408 couplé à un anticorps anti- *S*. *aureus,* un marqueur conjugué au phage CNCM I-5409 couplé à un anticorps anti-*S. aureus* et/ou un marqueur conjugué au phage CNCM I-5410 couplé à un anticorps anti- *S*. *aureus.* En particulier, il s'agit d'un marqueur conjugué à une protéine du phage CNCM I-5408 couplé à un anticorps anti- *S*. *aureus,* un marqueur conjugué à une protéine du phage CNCM I-5409 couplé à un anticorps anti- *S*. *aureus* et/ou un marqueur conjugué à une protéine du phage CNCM I-5410 couplé à un anticorps anti- *S*. *aureus.* En particulier, il s'agit d'un marqueur conjugué à un anticorps anti- S. *aureus* couplé au phage CNCM I-5408, un marqueur conjugué à un anticorps anti- *S*. *aureus* couplé au phage CNCM I-5409 et/ou un marqueur conjugué à un anticorps anti- *S*. *aureus* couplé au phage CNCM I-5410. En particulier, il s'agit d'un marqueur conjugué à un anticorps anti- *S*. *aureus* couplé à une protéine du phage CNCM I-5408, un marqueur conjugué à un anticorps anti- S. *aureus* couplé à une protéine du phage CNCM I-5409 et/ou un marqueur conjugué à un anticorps anti- *S*. *aureus* couplé à une protéine du phage CNCM I-5410. En particulier, ladite protéine du phage 2 (CNCM I-5408) est la protéine SEQ ID NO :1, ladite protéine du phage 3 (CNCM I-5409) est la protéine SEQ ID NO :2, ladite protéine du phage 7 (CNCM I-5410) est la protéine SEQ ID NO :3.

Des complexes bactérie/phage-anticorps marqué, bactérie/anticorps-phage marqué, bactérie/protéine de phage-anticorps ou bactérie/anticorps- protéine de phage marqué sont alors formés dans l'échantillon.

En particulier, pour ces deux modes de réalisation du procédé de détection, des AuNP sont utilisées en tant que marqueur et la présence de la bactérie dans l'échantillon est révélée par l'apparition d'un signal coloré, en particulier de couleur rose, dans la zone de détection 5, en particulier le long de la ligne de test.

### Exemple 7 : Dispositif de LFA (4 parties) avec des phages ou protéines de phages dans la partie de marquage et dans la partie de détection

Un mode de réalisation particulier du dispositif LFA est une bandelette 1 telle que décrite à l'Exemple 3, dans lequel :
- sur la zone de marquage 8, au lieu d'un marqueur conjugué à un anticorps anti- *S*. *aureus,* le ligand spécifique de *S. aureus* marqué, est un marqueur conjugué à un phage spécifique de *S*. *aureus* ou un marqueur conjugué à une protéine d'un phage spécifique de *S*. *aureus.*

Dans ce mode de réalisation particulier, la bandelette 1 de détection d'une bactérie d'intérêt dans un échantillon, comprend dans l'ordre les quatre parties suivantes:
- une partie de contact 2 comprenant une zone de contact 3 avec l'échantillon à tester ;
- une partie de marquage 7 comprenant une zone de marquage 8 où est déposé au moins un marqueur conjugué à un phage spécifique de *S*. *aureus* ou au moins un marqueur conjugué à une protéine d'un phage spécifique de *S*. *aureus,*
- une partie de détection 4 comprenant une zone de détection 5 de *S*. *aureus,* où est immobilisé au moins un phage spécifique de *S*. *aureus* et/ou au moins une protéine d'un phage spécifique de *S*. *aureus,*
- une partie absorbante 6.

La partie de marquage 7 peut comprendre un marqueur conjugué au phage CNCM I-5408, un marqueur conjugué au phage CNCM I-5409 et/ou un marqueur conjugué au phage CNCM I-5410. En particulier, elle peut comprendre un mélange de ces trois marqueurs conjugués à des phages. La partie de marquage 7 peut comprendre un marqueur conjugué à une protéine du phage CNCM I-5408, un marqueur conjugué à une protéine du phage CNCM I-5409 et/ou un marqueur conjugué à une protéine du phage CNCM I-5410. En particulier, ladite protéine du phage 2 (CNCM I-5408) est la protéine SEQ ID NO :1, ladite protéine du phage 3 (CNCM I-5409) est la protéine SEQ ID NO :2, ladite protéine du phage 7 (CNCM I-5410) est la protéine SEQ ID NO :3. En particulier, elle peut comprendre un mélange de ces trois marqueurs conjugués à des protéines de phages.

En particulier, des AuNP sont utilisées en tant que marqueur et la présence de la bactérie dans l'échantillon est révélée par l'apparition d'un signal coloré, en particulier de couleur rose, dans la zone de détection 5, en particulier le long de la ligne de test.

### Exemple 8 : Dispositif de LFA (4 parties) avec des protéines de phages dans la partie de détection

Dans cet exemple, le dispositif LFA utilisé est une bandelette 1 de LFA pour détecter S. aureus telle que décrite précédemment à la partie 3.1, soit une bandelette comprenant 4 parties (contact, marquage, détection et absorption).

Sur la zone de détection 5 (ligne de test), ont été immobilisés en tant que protéines de bactériophages spécifiques de S. aureus :
- la protéine du phage 2 (SEQ ID NO :1),
- la protéine du phage 3 (SEQ ID NO :2),
- la protéine du phage 7 (SEQ ID NO :3), ou
- la protéine du phage 5 (SEQ ID NO :4).

Les protéines ont été fixées sur la zone de détection 5 à une concentration de 1 mg/ml, en présence de BSA (albumine de sérum bovin) à 1 mg/ml, afin d'augmenter la fixation des protéines et de minimiser les interactions non spécifiques.

La zone de marquage comprend un anticorps anti-*S*. *aureus* Rabbit Polyclonal (Thermofischer, ref, PA1-7246), couplé avec des particules d'or de diamètre 40 nm.

La Figure 18 montre l'efficacité de la détection (apparition d'un signal le long de la ligne de test) de trois souches de S. *aureus* différentes (bandelettes 1, 2 et 3) avec un tel mode de réalisation d'une bandelette LFA où les protéines de séquences SEQ ID NO :1, SEQ ID NO :2, SEQ ID NO :3 et SEQ ID NO :4 sont utilisées pour sur la ligne de test.

### Exemple 9: Dispositif de LFA avec une protéine de phage dans la partie de marquage et en utilisant l'enzyme HRP comme marqueur

Dans cet exemple, le dispositif LFA utilisé est une bandelette 1 de LFA pour détecter S. *aureus* telle que décrite précédemment à l'exemple 4, soit une bandelette comprenant 4 parties (contact, marquage, détection et absorption).

La zone de marquage 8 comprend la protéine du phage 5 (SEQ ID NO :4) biotinylée.

Sur la zone de détection 5 (ligne de test), un anticorps anti-S. aureus a été immobilisé.

Pour la détection, dans un premier temps, l'enzyme HRP liée à la streptavidine est ajoutée sur la ligne de test. Grâce à la forte affinité biotine-streptavidine, l'HRP va se localiser au niveau des protéines biotinylées.

Dans un second temps, le TMB, substrat de l'HRP, est ajouté sur la bandelette.

La HRP va permettre l'oxydation du substrat TMB, entrainant une coloration bleue.

La Figure 20 montre les résultats de la détection de deux souches différentes de S. *aureus* avec ce mode de réalisation.

Les résultats montrent d'une part que la protéine de SEQ ID NO :4 est efficace pour la détection de *S*. *aureus* lorsqu'elle est utilisée sur la partie de marquage du dispositif LFA, et d'autre part que l'utilisation de la peroxydase est aussi efficace que des nanoparticules d'or comme marqueur.

### Exemple 10 : Evaluation de la spécificité de la protéine de SEQ ID NO :4 pour la détection de S. aureus dans le lait cru

Dans cet exemple, le dispositif LFA utilisé est une bandelette 1 de LFA pour détecter S. *aureus* telle que décrite précédemment à la partie 3.1, soit une bandelette comprenant 4 parties (contact, marquage, détection et absorption).

La zone de marquage 8 comprend un anticorps anti-S. *aureus* couplé à une nanoparticule d'or.

Sur la zone de détection 5 (ligne de test), la protéine du phage 5 (SEQ ID NO :4) a été immobilisée en tant que protéine de bactériophage spécifique de *S. aureus.*

Les protéines ont été fixées sur la zone de détection 5 à une concentration de 1 mg/ml, en présence de BSA (albumine de sérum bovin) à 1 mg/ml, afin d'augmenter la fixation des protéines et de minimiser les interactions non spécifiques.

Cette bandelette a été ici utilisée pour tester la détection de 18 souches de *S*. *aureus* différentes dans un milieu lait.

Comme précédemment décrit, pour la détection dans du lait cru, l'ajout de Tween 20 à l'échantillon est nécessaire.

Les résultats en Figure 19 montre que la protéine de séquence SEQ ID NO :4 utilisée sur la ligne de test dans un dispositif LFA permet de détecter environ 87% des souches de *S*. *aureus* dans un échantillon de lait (apparition d'un signal rose sur la ligne de test) et confirme ainsi la spécificité de détection de cette protéine même en milieu lait.

### Exemple 11 : Dispositif de LFA (4 parties) avec une protéine de phage dans la partie de marquage et dans la partie de détection et en utilisant l'enzyme HRP comme marqueur

Dans cet exemple, le dispositif LFA utilisé est une bandelette 1 de LFA pour détecter S. aureus telle que décrite précédemment à l'exemple 7, soit une bandelette comprenant 4 parties (contact, marquage, détection et absorption).

La zone de marquage 8 comprend la protéine du phage 5 (SEQ ID NO :4) biotinylée.

Sur la zone de détection 5 (ligne de test), la protéine du phage 5 (SEQ ID NO :4) a été immobilisée.

Pour la détection, dans un premier temps, l'enzyme HRP liée à la streptavidine est ajoutée sur la ligne de test. Grâce à la forte affinité biotine-streptavidine, l'HRP va se localiser au niveau des protéines biotinylées.

Dans un second temps, le TMB, substrat de l'HRP, est ajouté sur la bandelette.

La HRP va permettre l'oxydation du substrat TMB, entrainant une coloration bleue.

La Figure 21 montre les résultats de la détection de 4 souches différentes de S. *aureus* avec ce mode de réalisation.

Les résultats montrent d'une part que la protéine de SEQ ID NO :4 est efficace pour la détection de *S. aureus* lorsqu'elle est utilisée sur la partie de marquage et la partie de détection du dispositif LFA, et d'autre part que l'utilisation de la peroxydase est aussi efficace que des nanoparticules d'or comme marqueur.

### Référence à du matériel biologique déposé

Dans la présente demande, il est fait référence aux souches de bactériophages suivantes, toutes déposées à la CNCM le 21 Mars 2019 par Vetophage SAS:
- phage 2, référence d'identification auprès de la CNCM « Vetophage - phi 2 », dont le numéro d'enregistrement est « I-5408 »;
- phage 3, référence d'identification auprès de la CNCM « Vetophage - phi 3 », dont le numéro d'enregistrement est « I-5409 »;
- phage 7, référence d'identification auprès de la CNCM « Vetophage - phi 7 », dont le numéro d'enregistrement est « I-5410 ».

Dans la présente demande, il est fait référence aux souches de bactériophages suivantes, toutes déposées à la CNCM le 12 Février 2020 par Vetophage SAS:
- Phage, référence d'identification auprès de la CNCM « phi 9 », dont le numéro d'enregistrement est « I-5491 » ;
- Phage, référence d'identification auprès de la CNCM « phi 10 », dont le numéro d'enregistrement est « I-5492 ».

Dans la présente demande, il est fait référence à la souche de bactériophage suivante déposée à la CNCM le 07 mai 2021 par Vetophage SAS :
- phage 5, référence d'identification auprès de la CNCM « phi 5 », dont le numéro d'enregistrement est « I-5680 ».

### Liste des documents cités

A toute fin utile, les publications suivantes sont citées dans la présente demande :
- Reinoso et al., RAPD-PCR analysis of Staphylococcus aureus strains isolated from bovine and human hosts. Microbiological Research, Volume 159, Issue 3, Septembre 2004, pages 245-255;
- Foster Timothy J., Antibiotic resistance in Staphylococcus aureus. Current status and future prospects. FEMS Microbiology Review, Volume 41, Issue 3, Mai 2017, pages 430-449.
- Nanerjee and Jaiswal., Recent Advances in Nanoparticle-Based Lateral Flow Immunoassay as a Point-Of-Care Diagnostic Tool for Infectious Agents and Diseases (Ruptanu Banerjee et Amit Jaiswal), 2018 143(9):1970-1996.
- Sajid et al.Designs, formats and applications of lateral flow assay : A literature review. Journal of Saudi Chemical Society, 2015, 19, 689-705.
- Brevet EP1086372B1. Collection device for single assay or oral fluids. Déposé le 29 Mars 1999 et délivré le 31 Mai 2006.
- Brevet US 8,399.261 B2. Lateral flow assay system and methods for its use. Issue du PCT/US2008/0686821 déposé le 27 Juin 2008.
- Demande internationale WO 2017/072078, Subtractive immunoassay method and lateral flow immunochromatography assay strip for performing the method. Déposée le 24 Octobre 2016.
- Demande US2019/0276868A1, Methods for detecting microorganisms using microorganisms detection protein and other applications of cell binding components. Déposée le 11 Mars 2019.

## Revendications

1. Bandelette (1) de chromatographie à flux latéral pour détecter *Staphylococcus aureus* dans un échantillon, caractérisée en qu'elle comprend au moins une partie qui comprend au moins une protéine d'un bactériophage spécifique de *S. aureus,* ladite au moins une protéine étant choisie parmi une protéine de séquence SEQ ID NO :1, une protéine de séquence SEQ ID NO :2, une protéine de séquence SEQ ID NO :3, une protéine de séquence SEQ ID NO :4, une protéine de séquence SEQ ID NO :5, une protéine de séquence SEQ ID NO :6, et une protéine ayant au moins 70% d'identité avec l'une des protéines de séquence SEQ ID NOs :1- 6.

2. Bandelette (1) selon la revendication 1, caractérisée en qu'elle comprend dans l'ordre au moins :
- une partie de contact (2) comprenant une zone de contact (3) avec l'échantillon,
- une partie de détection (4) comprenant une zone de détection (5) de *S. aureus* qui comprend au moins une protéine d'un bactériophage spécifique de *S. aureus,*
- une partie absorbante (6).

3. Bandelette (1) selon la revendication 2, caractérisée en qu'elle comprend en outre entre la partie de contact (2) et la partie de détection (4), une partie de marquage (7) qui comprend une zone de marquage (8).

4. Bandelette (1) selon la revendication 1 ou 3, caractérisée en qu'elle comprend au moins :
- une partie de contact (2) comprenant une zone de contact (3) avec l'échantillon ;
- une partie de marquage (7) comprenant une zone de marquage (8) qui comprend un marqueur,
- une partie de détection (4) comprenant une zone de détection (5) de *S. aureus* qui comprend au moins une protéine d'au moins un bactériophage spécifique de *S. aureus,*
- une partie absorbante (6).

5. Bandelette (1) selon la revendication 1, caractérisée en qu'elle comprend au moins :
- une partie de contact (2) comprenant une zone de contact (3) avec l'échantillon ;
- une partie de marquage (7) comprenant une zone de marquage (8) qui comprend un marqueur conjugué à au moins un ligand spécifique de S. *aureus* choisi parmi au moins une protéine d'au moins un bactériophage spécifique de *S*. *aureus* et au moins une protéine d'un bactériophage spécifique de *S. aureus* couplée à un anticorps dirigé contre *S*. *aureus;*
- une partie de détection (4) comprenant une zone de détection (5) de *S. aureus* qui comprend au moins un ligand spécifique de *S*. *aureus ;*
- une partie absorbante (6).

6. Bandelette (1) selon l'une des revendications 1 à 5, **caractérisée en ce que** ladite au moins une protéine d'au moins un bactériophage spécifique de *S. aureus* est une protéine de liaison au récepteur de phage (RBP).

7. Procédé de détection de *S*. *aureus* dans un échantillon caractérisé en qu'il comprend :
- une étape de marquage des bactéries ou de *S. aureus* dans l'échantillon ;
- une étape de mise en contact de l'échantillon avec la zone de contact (3) d'une bandelette (1) selon l'une des revendications 1 à 6 ;
- une étape de détection du marqueur sur la zone de détection ;
- une étape d'analyse de la présence ou de l'absence de *S*. *aureus* dans l'échantillon.

8. Procédé de détection de S. *aureus* dans un échantillon caractérisé en qu'il comprend :
- une étape de mise en contact de l'échantillon avec la zone de contact (3) d'une bandelette (1) selon l'une des revendications 3 à 6 ;
- une étape de détection du marqueur sur la zone de détection ;
- une étape d'analyse de la présence ou de l'absence de S. *aureus* dans l'échantillon.

9. Kit de détection de S. *aureus* dans un échantillon caractérisé en qu'il comprend :
- une bandelette (1) selon l'une des revendications 1 à 6,
- un contenant destiné à recevoir l'échantillon,
- un contenant comprenant un marqueur.

10. Kit de détection de S. *aureus* dans un échantillon caractérisé en qu'il comprend :
- une bandelette (1) selon l'une des revendications 3 à 6,
- un contenant destiné à recevoir l'échantillon.

11. Utilisation d'au moins une protéine d'au moins un bactériophage spécifique de S. *aureus,* ladite protéine étant une protéine de séquence SEQ ID NO :1, une protéine de séquence SEQ ID NO :2, une protéine de séquence SEQ ID NO :3, une protéine de séquence SEQ ID NO :4, une protéine de séquence SEQ ID NO :5, une protéine de séquence SEQ ID NO :6, ou une protéine ayant au moins 70% d'identité avec l'une des protéines de séquence SEQ ID NOs :1- 6, pour détecter S. *aureus* dans un échantillon, **caractérisée en ce que** ladite au moins une protéine d'un bactériophage spécifique de *S. aureus* est utilisée dans une bandelette (1) de chromatographie à flux latéral.

12. Utilisation selon la revendication 11, **caractérisée en ce que** ladite au moins une protéine d'au moins un bactériophage spécifique de *S. aureus* est une protéine de liaison au récepteur de phage (RBP).

## Patentansprüche

1. Lateralfluss-Chromatographie-Teststreifen (1) zum Nachweis von *Straphylococcus aureus* in einer Probe, **dadurch gekennzeichnet, dass** er mindestens einen Abschnitt umfasst, der mindestens ein Protein eines für *S*. *aureus* spezifischen Bakteriophagen umfasst,
wobei das mindestens eine Protein unter einem Protein der Sequenz SEQ ID NO:1, einem Protein der Sequenz SEQ ID NO:2, einem Protein der Sequenz SEQ ID NO:3, einem Protein der Sequenz SEQ ID NO:4, einem Protein der Sequenz SEQ ID NO:5, einem Protein der Sequenz SEQ ID NO:6 und einem Protein mit mindestens 70% Identität mit einem der Proteine der Sequenz SEQ ID NO:1-6 ausgewählt ist.

2. Teststreifen (1) nach Anspruch 1, **dadurch gekennzeichnet, dass** er in dieser Reihenfolge mindestens umfasst:
- einen Kontaktabschnitt (2), der einen Kontaktbereich (3) mit der Probe umfasst,
- einen Nachweisabschnitt (4), der einen Nachweisbereich (5) für *S*. *aureus* umfasst, der mindestens ein Protein eines für *S*. *aureus* spezifischen Bakteriophagen umfasst,
- einen absorbierenden Abschnitt (6).

3. Teststreifen (1) nach Anspruch 2, **dadurch gekennzeichnet, dass** er ferner zwischen dem Kontaktabschnitt (2) und dem Nachweisabschnitt (4) einen Markierungsabschnitt (7) umfasst, der einen Markierungsbereich (8) umfasst.

4. Teststreifen (1) nach Anspruch 1 oder 3, **dadurch gekennzeichnet, dass** er mindestens umfasst:
- einen Kontaktabschnitt (2), der einen Kontaktbereich (3) mit der Probe umfasst,
- einen Markierungsabschnitt (7), der einen Markierungsbereich (8) umfasst, der einen Marker umfasst,
- einen Nachweisabschnitt (4), der einen Nachweisbereich (5) für *S*. *aureus* umfasst, der mindestens ein Protein mindestens eines für S. *aureus* spezifischen Bakteriophagen umfasst,
- einen absorbierenden Abschnitt (6).

5. Teststreifen (1) nach Anspruch 1, **dadurch gekennzeichnet, dass** er mindestens umfasst:
- einen Kontaktabschnitt (2), der einen Kontaktbereich (3) mit der Probe umfasst;
- einen Markierungsabschnitt (7) der einen Markierungsbereich (8) umfasst, der einen Marker umfasst, der mit mindestens einem für *S*. *aureus* spezifischen Liganden konjugiert ist, der ausgewählt ist aus mindestens einem Protein mindestens eines für S. *aureus* spezifischen Bakteriophagen, mindestens einem Protein eines für *S*. *aureus* spezifischen Bakteriophagen, das an einen gegen *S*. *aureus* gerichteten Antikörper gekoppelt ist;
- einen Nachweisabschnitt (4), der einen Nachweisbereich (5) für *S*. *aureus* umfasst, der mindestens einen für *S*. *aureus* spezifischen Liganden umfasst;
- einen absorbierenden Abschnitt (6).

6. Teststreifen (1) nach einem der Ansprüche 1 bis 5, **dadurch gekennzeichnet, dass** das mindestens eine Protein mindestens eines für *S*. *aureus* spezifischen Bakteriophagen ein Phagenrezeptor-bindendes Protein (PBP) ist.

7. Verfahren zum Nachweis von *S*. aureus-Bakterien in einer Probe, **dadurch gekennzeichnet, dass** es umfasst:
- einen Schritt des Markierens der Bakterien oder von *S*. *aureus* in der Probe;
- einen Schritt, bei dem die Probe mit dem Kontaktbereich (3) eines Teststreifens (1) nach einem der Ansprüche 1 bis 6 in Kontakt gebracht wird;
- einen Schritt des Nachweises des Markers auf dem Nachweisbereich;
- einen Schritt der Analyse des Vorhandenseins oder der Abwesenheit von S. *aureus* in der Probe.

8. Verfahren zum Nachweis von *S*. *aureus* in einer Probe, **dadurch gekennzeichnet, dass** es umfasst:
- einen Schritt, bei dem die Probe mit dem Kontaktbereich (3) eines Teststreifens (1) nach einem der Ansprüche 3 bis 6 in Kontakt gebracht wird;
- einen Schritt des Nachweises des Markers auf dem Nachweisbereich;
- einen Schritt der Analyse des Vorhandenseins oder der Abwesenheit von *S*. *aureus* in der Probe.

9. Kit zum Nachweis von *S*. *aureus* in einer Probe, **dadurch gekennzeichnet, dass** es umfasst:
- einen Teststreifen (1) nach einem der Ansprüche 1 bis 6,
- einen Behälter zur Aufnahme der Probe,
- einen Behälter, der einen Marker umfasst.

10. Kit zum Nachweis von *S*. *aureus* in einer Probe, **dadurch gekennzeichnet, dass** es umfasst:
- einen Teststreifen (1) nach einem der Ansprüche 3 bis 6,
- einen Behälter zur Aufnahme der Probe.

11. Verwendung von mindestens einem Protein mindestens eines für *S*. *aureus* spezifischen Bakteriophagen zum Nachweis von *S*. *aureus* in einer Probe, wobei das mindestens eine Protein ein Protein der Sequenz SEQ ID NO:1, ein Protein der Sequenz SEQ ID NO:2, ein Protein der Sequenz SEQ ID NO:3, ein Protein der Sequenz SEQ ID NO:4, ein Protein der Sequenz SEQ ID NO:5, ein Protein der Sequenz SEQ ID NO:6 oder ein Protein mit mindestens 70% Identität mit einem der Proteine der Sequenz SEQ ID NO:1-6 ist,
**dadurch gekennzeichnet, dass** das mindestens eine Protein eines für *S*. *aureus* spezifischen Bakteriophagen in einem Lateralfluss-Chromatographie-Teststreifen (1) verwendet wird.

12. Verwendung nach Anspruch 11, **dadurch gekennzeichnet, dass** das mindestens eine Protein mindestens eines für *S. aureus* spezifischen Bakteriophagen ein Phagenrezeptor-bindendes Protein (PBP) ist.

## Claims

1. Lateral flow chromatography strip (1) for detecting *Staphylococcus aureus* in a sample **characterised in that** it comprises at least one part that comprises at least one protein of at least one S. *aureus-specific* bacteriophage, said at least one protein being chosen among a protein of sequence SEQ ID NO: 1, a protein of sequence SEQ ID NO:2, a protein of sequence SEQ ID NO:3, a protein of sequence SEQ ID NO:4, a protein of sequence SEQ ID NO:5, a protein of sequence SEQ ID NO:6, and a protein having at least 70% identity with one of the proteins of sequence SEQ ID NOs: 1-6.

2. Strip (1) according to claim 1, **characterised in that** it comprises in order at least:
- a contact part (2) comprising an area (3) of contact with the sample,
- a detection part (4) comprising a detection zone (5) of *S. aureus* that comprises at least one protein of an S. *aureus-specific* bacteriophage,
- an absorbent part (6).

3. Strip (1) according to claim 2, **characterised in that** it furthermore comprises, between the contact part (2) and the detection part (4), a marking part (7) which comprises a marking area (8).

4. Strip (1) according to claim 1 or 3, **characterised in that** it comprises at least:
- a contact part (2) comprising an area (3) of contact with the sample;
- a marking part (7) comprising a marking area (8) that comprises a marker,
- a detection part (4) comprising a zone (5) detecting *S. aureus* that comprises at least one protein of at least one *S*. *aureus-specific* bacteriophage,
- an absorbent part (6).

5. Strip (1) according to claim 1, **characterised in that** it comprises at least:
- a contact part (2) comprising an area (3) of contact with the sample;
- a marking part (7) comprising a marking area (8) that comprises a marker conjugate with at least one S. *aureus-specific* ligand selected from at least one protein of at least one *S. aureus-specific* bacteriophage and at least one protein of an *S. aureus-specific* bacteriophage coupled to an antibody directed against *S. aureus;*
- a detection part (4) comprising a zone (5) detecting *S. aureus* that comprises at least one *S*. *aureus-specific* ligand;
- an absorbent part (6).

6. Strip (1) according to one of claims 1 to 5, **characterised in that** said at least one protein of at least one *S*. *aureus-specific* bacteriophage is a phage receptor binding protein (RBP).

7. Method for detecting *S. aureus* in a sample, **characterised in that** it comprises:
- a step of marking bacteria or *S. aureus* in the sample;
- a step of putting the sample in contact with the contact area (3) of a strip (1) according to one of claims 1 to 6;
- a step of detecting the marker on the detection zone;
- a step of analysing the presence or absence of *S. aureus* in the sample.

8. Method for detecting *S. aureus* in a sample, **characterised in that** it comprises:
- a step of putting the sample in contact with the contact area (3) of a strip (1) according to one of claims 3 to 6;
- a step of detecting the marker on the detection zone;
- a step of analysing the presence or absence of *S. aureus* in the sample.

9. *S. aureus* detection kit in a sample, **characterised in that** it comprises:
- a strip (1) according to one of claims 1 to 6,
- a container intended to receive the sample,
- a container comprising a marker.

10. *S. aureus* detection kit in a sample, **characterised in that** it comprises:
- a strip (1) according to one of claims 3 to 6,
- a container intended to receive the sample.

11. Use of at least one protein of at least one *S*. *aureus-specific* bacteriophage, said protein being a protein of sequence SEQ ID NO:1, a protein of sequence SEQ ID NO:2, a protein of sequence SEQ ID NO:3, a protein of sequence SEQ ID NO:4, a protein of sequence SEQ ID NO:5, a protein of sequence SEQ ID NO:6, or a protein having at least 70% identity with one of the proteins of sequence SEQ ID NOs: 1-6, for detecting *S. aureus* in a sample, **characterised in that** said at least one protein of an *S*. aureus-specific bacteriophage is used in a lateral flow chromatography strip (1).

12. Use according to claim 11, **characterised in that** said at least one protein of at least one *S*. *aureus-specific* bacteriophage is a phage receptor binding protein (RBP).
